# EUROPEAN PATENT APPLICATION

(11) **EP 4 810 515 A1**
(43) Date of publication of application: **23.09.2026**
(21) Application number: 24890616.6
(22) Date of filing: 11.11.2024
(51) Int. Cl.: C07K 16/22, C12N 15/13, A61K 39/395, A61P 35/00, A61P 3/00, G01N 33/68

(54) **GDF15 NEUTRALIZING ANTIBODY AND USE THEREOF**

(30) Priority: 14.11.2023 CN 202311517942
(71) Applicant: Shanghai Haiju Biological Technology Co., Ltd., Shanghai 201203 (CN); Yangtze River Pharmaceutical Group Co., Ltd., Taizhou, Jiangsu 225321 (CN)
(72) Inventor: LIU, Jian, Shanghai 201203 (CN); CHANG, Rulue, Shanghai 201203 (CN); GAO, Shuman, Shanghai 201203 (CN); PANG, Xingchen, Shanghai 201203 (CN); WEI, Jie, Shanghai 201203 (CN); YANG, Haodong, Shanghai 201203 (CN); WANG, Ruwei, Shanghai 201203 (CN); LIAO, Shihua, Shanghai 201203 (CN); MENG, Li, Shanghai 201203 (CN); ZHANG, Ying, Shanghai 201203 (CN); LI, Guanying, Shanghai 201203 (CN); LI, Huali, Shanghai 201203 (CN); MU, Songlin, Shanghai 201203 (CN); WANG, Lichun, Shanghai 201203 (CN); MA, Mark Zhiqing, Shanghai 201203 (CN)
(74) Representative: LKGLOBAL Partnerschaftsgesellschaft mbB
(86) International application number: PCT/CN2024/131269
(87) International publication number: WO 2025/103260

(57) **Abstract**

The present invention provides a GDF15-binding antibody or an antigen-binding fragment thereof. The antibody or the antigen-binding fragment thereof comprises: CDR sequences selected from at least one of the following or amino acid sequences having at least 66% identity with same: heavy chain variable region CDR sequences: SEQ ID NO: 1-34 and SEQ ID NO: 74-76; and light chain variable region CDR sequences: SEQ ID NO: 36-73 and SEQ ID NO: 77-82. According to embodiments of the present invention, the antibody or the antigen-binding fragment thereof can specifically bind to GDF15, can effectively block the binding of GFRAL and GDF15, and can also effectively inhibit Treg differentiation, and therefore can be used for preventing and/or treating cancers and cachexia.

## Description

### TECHNICAL FIELD

The present application belongs to the field of biopharmaceutical technology. Specifically, the present application relates to a GDF15 neutralizing antibody and use thereof. More specifically, the present application relates to an antibody or antigen-binding fragment thereof, a nucleic acid molecule, an expression vector, a recombinant cell, a pharmaceutical composition and use thereof, and a kit and use thereof.

### BACKGROUND

Cachexia, also known as dyscrasia, is a multifactorial syndrome characterized by persistent loss of skeletal muscle (with or without loss of adipose tissue) that cannot be fully reversed by conventional nutritional support, leading to progressive functional impairment. Cachexia can be seen during the progression of many chronic diseases, among which cancer cachexia is the most common. It is characterized by muscle wasting in skeletal and visceral tissues, accompanied by various clinical manifestations such as decreased appetite, anorexia, early satiety, weight loss, muscle atrophy, fatigue, anemia, edema, and hypoalbuminemia. It can be classified into the pre-cachexia stage, cachexia stage, and refractory cachexia stage.

Among malignant tumors, the incidence of cachexia is highest in digestive system tumors, with 71% in pancreatic cancer, 65% in gastroesophageal cancer, 56% in colorectal cancer, 40% in liver cancer and cholangiocarcinoma, and others. The incidence of cachexia also varies at different stages of the same tumor, with a higher incidence in patients with advanced tumors. Among the top ten high-incidence tumors, lung cancer, colorectal cancer, gastric cancer, liver cancer, esophageal cancer, and pancreatic cancer are often accompanied by cachexia.

Currently, for cachexia, only one drug was launched in Japan on April 1, 2021, for the treatment of cachexia in malignant tumors such as non-small cell lung cancer, gastric cancer, pancreatic cancer, or colorectal cancer. However, the results have shown that this marketed drug could increase patient body weight and alleviate symptoms such as anorexia, but could not improve grip strength. No other drugs have been approved for the treatment of cancer cachexia.

Currently, the optimal approach for treating cancer cachexia may involve single or combined strategies, including pharmacological intervention, nutritional intervention, exercise guidance, and psychological intervention. For pharmacological treatment of cancer cachexia, progestogens (Class 1A recommendation) are the first choice to stimulate appetite. However, the use of the above-mentioned drugs also carries many risks, including contraindications, adverse reactions, or lack of significant efficacy. Therefore, there is a need to develop a drug that can effectively ameliorate cancer cachexia.

### SUMMARY

The present application intends to solve at least one of the technical problems existing in the prior art to some extent. To this end, the present application provides a GDF15 neutralizing antibody. This neutralizing antibody can specifically bind to GDF15, effectively block the binding of GFRAL and GDF15, and also effectively inhibit the differentiation of regulatory T cells (Treg cells or Treg), and therefore can be used for preventing and/or treating cancer and cachexia.

The present application is based on the following discoveries of the inventors:
Among all targets for treating cachexia, GDF15 and the receptor thereof, GFRAL, are currently the most focused and fastest-progressing. GDF15, also known as macrophage inhibitory cytokine-1 (MIC-1), is a member of the transforming growth factor β (TGFβ) superfamily. GDF15 is also known as nonsteroidal anti-inflammatory drug-activated gene-1 (NAG-1), prostate derived factor (PDF), placental bone morphogenetic protein (PLAB), placental transforming growth factor beta (PTGFB), and nonsteroidal anti-inflammatory drug-regulated gene-1 (NRG-1). GDF15 exists in cells as a precursor polypeptide, and upon hydrolysis to remove the amino-terminal propeptide, it produces and secretes a mature 25 kDa homodimer into the extracellular space, where it functions.

Currently, studies have found that GFRAL is a high-affinity receptor for GDF15. GFRAL is an orphan receptor and a transmembrane protein with a short cytoplasmic domain. It is relatively enriched in the substantia nigra, hippocampus, and posterior region of the central nervous system. GDF15 binding to GFRAL forms a heterodimeric complex with the transmembrane tyrosine kinase co-receptor RET, thereby activating intracellular signaling pathways consistent with GDNF signaling. This pathway plays a central role in the mechanism of reducing food intake and body weight, making it a hot topic in research on obesity, diabetes, cachexia, and others.

Additionally, besides its role in metabolism related to cachexia, anorexia, and vomiting, the GDF15 molecule has various other functions. In recent years, the most reported function of GDF15 is its role in inhibiting immunity and inflammation.

However, currently, no drug has been seen that possesses both functions of treating cachexia and tumor immunity. For example, Ponsegromab from Pfizer has a good GFRAL blocking function, but its function in inhibiting Treg differentiation to enhance tumor immunity is not high. Visugromab from CaltalYM and the candidate antibodies from the Fourth Military Medical University of the Chinese People's Liberation Army have been reported in the literature to have good tumor immune function, but their GFRAL blocking function is weak.

However, the inventors unexpectedly found through experiments that the GDF15 neutralizing antibody of the present application can achieve a good GFRAL blocking function while also showing high inhibitory function on Treg differentiation in vitro experiments. In particular, the GDF15 neutralizing antibody of the present application has a stronger inhibitory function on Treg differentiation compared to the drug Ponsegromab, and this has been verified at the cellular and in vivo levels, demonstrating the antibody has more excellent tumor immune activity.

In one aspect of the present application, the present application provides an antibody or antigen-binding fragment thereof. According to an embodiment of the present application, the antibody or antigen-binding fragment thereof comprises: at least one CDR sequence selected from the group consisting of the following or an amino acid sequence having at least 66% identity thereto: heavy chain variable region CDR sequences: SEQ ID NOs: 1 to 34 and SEQ ID NOs: 74 to 76; light chain variable region CDR sequences: SEQ ID NOs: 36 to 73 and SEQ ID NOs: 77 to 82. According to an embodiment of the present application, this antibody or antigen-binding fragment thereof can specifically bind to GDF15, effectively block the binding of GFRAL and GDF15, and also effectively inhibit Treg differentiation, and therefore can be used for preventing and/or treating cancer and cachexia.

According to an embodiment of the present application, the antibody or antigen-binding fragment thereof may further comprise at least one of the following technical features:
According to an embodiment of the present application, the antibody comprises: a heavy chain variable region CDR1 selected from the group consisting of the amino acid as set forth in any one of SEQ ID NO: 1, SEQ ID NO: 4, SEQ ID NO: 7, SEQ ID NO: 9, SEQ ID NO: 12, SEQ ID NO: 15, SEQ ID NO: 23, and SEQ ID NO: 32; a heavy chain variable region CDR2 selected from the group consisting of the amino acid as set forth in any one of SEQ ID NO: 2, SEQ ID NO: 5, SEQ ID NO: 10, SEQ ID NO: 13, SEQ ID NO: 16, SEQ ID NO: 17, SEQ ID NO: 19, SEQ ID NO: 21, SEQ ID NO: 24, SEQ ID NO: 26, SEQ ID NO: 28, SEQ ID NO: 33, SEQ ID NO: 74, SEQ ID NO: 75, and SEQ ID NO: 76; a heavy chain variable region CDR3 selected from the group consisting of the amino acid as set forth in any one of SEQ ID NO: 3, SEQ ID NO: 6, SEQ ID NO: 8, SEQ ID NO: 11, SEQ ID NO: 14, SEQ ID NO: 18, SEQ ID NO: 20, SEQ ID NO: 22, SEQ ID NO: 25, SEQ ID NO: 27, SEQ ID NO: 29, SEQ ID NO: 30, SEQ ID NO: 31, and SEQ ID NO: 34; a light chain variable region CDR1 selected from the group consisting of the amino acid as set forth in any one of SEQ ID NO: 36, SEQ ID NO: 39, SEQ ID NO: 43, SEQ ID NO: 46, SEQ ID NO: 49, SEQ ID NO: 52, SEQ ID NO: 55, SEQ ID NO: 58, SEQ ID NO: 63, SEQ ID NO: 66, SEQ ID NO: 68, SEQ ID NO: 70, SEQ ID NO: 73, SEQ ID NO: 77, SEQ ID NO: 78, and SEQ ID NO: 79; a light chain variable region CDR2 selected from the group consisting of the amino acid as set forth in any one of SEQ ID NO: 37, SEQ ID NO: 40, SEQ ID NO: 44, SEQ ID NO: 47, SEQ ID NO: 50, SEQ ID NO: 53, SEQ ID NO: 56, SEQ ID NO: 59, SEQ ID NO: 61, SEQ ID NO: 64, and SEQ ID NO: 71; or a light chain variable region CDR3 selected from the group consisting of the amino acid as set forth in any one of SEQ ID NO: 38, SEQ ID NO: 41, SEQ ID NO: 42, SEQ ID NO: 45, SEQ ID NO: 48, SEQ ID NO: 51, SEQ ID NO: 54, SEQ ID NO: 57, SEQ ID NO: 60, SEQ ID NO: 62, SEQ ID NO: 65, SEQ ID NO: 67, SEQ ID NO: 69, SEQ ID NO: 72, SEQ ID NO: 80, SEQ ID NO: 81, and SEQ ID NO: 82.

According to an embodiment of the present application, the antibody comprises: heavy chain variable region CDR1, CDR2, and CDR3 sequences as set forth in the amino acid sequences of SEQ ID NOs: 1, 2, and 3, respectively; or heavy chain variable region CDR1, CDR2, and CDR3 sequences as set forth in the amino acid sequences of SEQ ID NOs: 4, 5, and 6, respectively; or heavy chain variable region CDR1, CDR2, and CDR3 sequences as set forth in the amino acid sequences of SEQ ID NOs: 7, 5, and 8, respectively; or heavy chain variable region CDR1, CDR2, and CDR3 sequences as set forth in the amino acid sequences of SEQ ID NOs: 9, 10, and 11, respectively; or heavy chain variable region CDR1, CDR2, and CDR3 sequences as set forth in the amino acid sequences of SEQ ID NOs: 12, 13, and 14, respectively; or heavy chain variable region CDR1, CDR2, and CDR3 sequences as set forth in the amino acid sequences of SEQ ID NOs: 15, 16, and 14, respectively; or heavy chain variable region CDR1, CDR2, and CDR3 sequences as set forth in the amino acid sequences of SEQ ID NOs: 1, 17, and 18, respectively; or heavy chain variable region CDR1, CDR2, and CDR3 sequences as set forth in the amino acid sequences of SEQ ID NOs: 15, 19, and 20, respectively; or heavy chain variable region CDR1, CDR2, and CDR3 sequences as set forth in the amino acid sequences of SEQ ID NOs: 4, 21, and 22, respectively; or heavy chain variable region CDR1, CDR2, and CDR3 sequences as set forth in the amino acid sequences of SEQ ID NOs: 23, 24, and 25, respectively; or heavy chain variable region CDR1, CDR2, and CDR3 sequences as set forth in the amino acid sequences of SEQ ID NOs: 12, 26, and 27, respectively; or heavy chain variable region CDR1, CDR2, and CDR3 sequences as set forth in the amino acid sequences of SEQ ID NOs: 7, 28, and 29, respectively; or heavy chain variable region CDR1, CDR2, and CDR3 sequences as set forth in the amino acid sequences of SEQ ID NOs: 7, 5, and 30, respectively; or heavy chain variable region CDR1, CDR2, and CDR3 sequences as set forth in the amino acid sequences of SEQ ID NOs: 7, 5, and 31, respectively; or heavy chain variable region CDR1, CDR2, and CDR3 sequences as set forth in the amino acid sequences of SEQ ID NOs: 32, 33, and 34, respectively; or heavy chain variable region CDR1, CDR2, and CDR3 sequences as set forth in the amino acid sequences of SEQ ID NOs: 9, 74, and 11, respectively; or heavy chain variable region CDR1, CDR2, and CDR3 sequences as set forth in the amino acid sequences of SEQ ID NOs: 9, 75, and 11, respectively; or heavy chain variable region CDR1, CDR2, and CDR3 sequences as set forth in the amino acid sequences of SEQ ID NOs: 9, 76, and 11, respectively.

According to an embodiment of the present application, the antibody comprises: light chain variable region CDR1, CDR2, and CDR3 sequences as set forth in the amino acid sequences of SEQ ID NOs: 36, 37, and 38, respectively; or light chain variable region CDR1, CDR2, and CDR3 sequences as set forth in the amino acid sequences of SEQ ID NOs: 39, 40, and 41, respectively; or light chain variable region CDR1, CDR2, and CDR3 sequences as set forth in the amino acid sequences of SEQ ID NOs: 39, 40, and 42, respectively; or light chain variable region CDR1, CDR2, and CDR3 sequences as set forth in the amino acid sequences of SEQ ID NOs: 43, 44, and 45, respectively; or light chain variable region CDR1, CDR2, and CDR3 sequences as set forth in the amino acid sequences of SEQ ID NOs: 46, 47, and 48, respectively; or light chain variable region CDR1, CDR2, and CDR3 sequences as set forth in the amino acid sequences of SEQ ID NOs: 49, 50, and 51, respectively; or light chain variable region CDR1, CDR2, and CDR3 sequences as set forth in the amino acid sequences of SEQ ID NOs: 52, 53, and 54, respectively; or light chain variable region CDR1, CDR2, and CDR3 sequences as set forth in the amino acid sequences of SEQ ID NOs: 49, 47, and 51, respectively; or light chain variable region CDR1, CDR2, and CDR3 sequences as set forth in the amino acid sequences of SEQ ID NOs: 55, 56, and 57, respectively; or light chain variable region CDR1, CDR2, and CDR3 sequences as set forth in the amino acid sequences of SEQ ID NOs: 58, 59, and 60, respectively; or light chain variable region CDR1, CDR2, and CDR3 sequences as set forth in the amino acid sequences of SEQ ID NOs: 49, 61, and 62, respectively; or light chain variable region CDR1, CDR2, and CDR3 sequences as set forth in the amino acid sequences of SEQ ID NOs: 63, 64, and 65, respectively; or light chain variable region CDR1, CDR2, and CDR3 sequences as set forth in the amino acid sequences of SEQ ID NOs: 66, 40, and 67, respectively; or light chain variable region CDR1, CDR2, and CDR3 sequences as set forth in the amino acid sequences of SEQ ID NOs: 68, 40, and 69, respectively; or light chain variable region CDR1, CDR2, and CDR3 sequences as set forth in the amino acid sequences of SEQ ID NOs: 70, 71, and 72, respectively; or light chain variable region CDR1, CDR2, and CDR3 sequences as set forth in the amino acid sequences of SEQ ID NOs: 77, 53, and 54, respectively; or light chain variable region CDR1, CDR2, and CDR3 sequences as set forth in the amino acid sequences of SEQ ID NOs: 78, 53, and 54, respectively; or light chain variable region CDR1, CDR2, and CDR3 sequences as set forth in the amino acid sequences of SEQ ID NOs: 79, 53, and 54, respectively; or light chain variable region CDR1, CDR2, and CDR3 sequences as set forth in the amino acid sequences of SEQ ID NOs: 39, 40, and 80, respectively; or light chain variable region CDR1, CDR2, and CDR3 sequences as set forth in the amino acid sequences of SEQ ID NOs: 39, 40, and 81, respectively; or light chain variable region CDR1, CDR2, and CDR3 sequences as set forth in the amino acid sequences of SEQ ID NOs: 39, 40, and 82, respectively; or light chain variable region CDR1, CDR2, and CDR3 sequences as set forth in the amino acid sequences of SEQ ID NOs: 73, 53, and 54, respectively.

The above-mentioned CDRs of different neutralizing antibodies screened in the present application were determined and annotated using the Kabat numbering system, wherein the amino acid sequences as set forth in SEQ ID NOs: 1 to 82 are shown in Table 1.

**Table 1**

| Heavy chain name | CDR1 | SEQ ID NO: | CDR2 | SEQ ID NO: | CDR3 | SEQ ID NO: |
|---|---|---|---|---|---|---|
| G2-CH1 VH | TYGIGVG | 1 | HIWWNNNKYFNTALKS | 2 | MGYDIMDY | 3 |
| G2-CH2 VH | ISGMGVS | 4 | HIYWDDDKRYNPSLKS | 5 | AGWDFFDY | 6 |
| G2-CH3 VH | TSGMGVS | 7 | HIYWDDDKRYNPSLKS | 5 | TGWDFFDY | 8 |
| G2-CH5 VH | DYNLN | 9 | DLNPYNGGIIYNQKFKG | 10 | EGRGGAWFDS | 11 |
| G2-CH6 VH | DYNMN | 12 | LINPYNGGTRYNQNFKG | 13 | EVRLGNALDY | 14 |
| G2-CH7 VH | GYNMN | 15 | LINPSNGGSSYSQKFKG | 16 | EVRLGNALDY | 14 |
| G2-CH8 VH | TYGIGVG | 1 | HIWWNDNKYYNTALKS | 17 | LAYDYVDS | 18 |
| G2-CH9 VH | GYNMN | 15 | LINPYNGGTIYNQKFKG | 19 | EVRLGNAMDY | 20 |
| G2-CH10 VH | ISGMGVS | 4 | HIYWDDDKRYKPSLKS | 21 | SAYGMTDY | 22 |
| G2-CH12 VH | NYDIN | 23 | WIFPGDGSTKYNEKFKD | 24 | ILY | 25 |
| G2-CH13 VH | DYNMN | 12 | DINPYNGGIIYNQKFRG | 26 | EVRRGSFFDY | 27 |
| G2-CH14 VH | TSGMGVS | 7 | LIYWDDDKRYNPSLKS | 28 | SPWDWFAY | 29 |
| G2-CH16 VH | TSGMGVS | 7 | HIYWDDDKRYNPSLKS | 5 | RAYGAMDY | 30 |
| G2-CH17 VH | TSGMGVS | 7 | HIYWDDDKRYNPSLKS | 5 | TGWDFFDI | 31 |
| G2-CH23 VH | SYWIE | 32 | EILPGSGITNYNEKFKG | 33 | ETARARGFTY | 34 |
| G2-CH5-VH-P1 | DYNLN | 9 | DLNPYNAGIIYNQKFKG | 74 | EGRGGAWFDS | 11 |
| G2-CH5-VH-P2 | DYNLN | 9 | DLNPYNQGIIYNQKFKG | 75 | EGRGGAWFDS | 11 |
| G2-CH5-VH-P3 | DYNLN | 9 | DLNPYEGGIIYNQKFKG | 76 | EGRGGAWFDS | 11 |

| Light chain name | CDR1 | SEQ ID NO: | CDR2 | SEQ ID NO: | CDR3 | SEQ ID NO: |
|---|---|---|---|---|---|---|
| G2-CH1 VL | KASQDVNTAVA | 36 | SSSYRYT | 37 | QQHYSTPWT | 38 |
| G2-CH2 VL | KASQNVGTNVA | 39 | SASYRYS | 40 | QQYNSYPWT | 41 |
| G2-CH3 VL | KASQNVGTNVA | 39 | SASYRYS | 40 | QQYNSYPYT | 42 |
| G2-CH5 VL | KASEDIYHRLA | 43 | GATSLET | 44 | QQYWSPPFT | 45 |
| G2-CH6 VL | RPSESIYSNLA | 46 | AATNLAD | 47 | QHFWGIPWT | 48 |
| G2-CH7 VL | RASENIYSNLA | 49 | AATNLGD | 50 | QHFWGTPWT | 51 |
| G2-CH8 VL | RSSKSLLHSNGITYLY | 52 | QMSNLVS | 53 | AQNLELPPT | 54 |
| G2-CH9 VL | RASENIYSNLA | 49 | AATNLAD | 47 | QHFWGTPWT | 51 |
| G2-CH10 VL | RASQDISNYLN | 55 | SASNLHS | 56 | QQYNMFPWT | 57 |
| G2-CH12 VL | KASQDINSYLS | 58 | RANKLVD | 59 | LQYDEFPYT | 60 |
| G2-CH13 VL | RASENIYSNLA | 49 | AATDLAD | 61 | QLFWGTPT | 62 |
| G2-CH14 VL | SVSSSISSSNLH | 63 | GTSNLAS | 64 | QQWSSYPLT | 65 |
| G2-CH16 VL | KASQNVGTNIA | 66 | SASYRYS | 40 | QQYNTYPHT | 67 |
| G2-CH17 VL | KASQNVGSNVA | 68 | SASYRYS | 40 | QQYKSYPLT | 69 |
| G2-CH23 VL | ITSIDIDDDIN | 70 | EGNTLRP | 71 | LQSDNLPLT | 72 |
| G2-CH8-VL-P1 | RSSKSLLHSNAITYLY | 77 | QMSNLVS | 53 | AQNLELPPT | 54 |
| G2-CH8-VL-P2 | RSSKSLLHSNQITYLY | 78 | QMSNLVS | 53 | AQNLELPPT | 54 |
| G2-CH8-VL-P3 | RSSKSLLHSEGITYLY | 79 | QMSNLVS | 53 | AQNLELPPT | 54 |
| G2-CH2-VL-P1 | KASQNVGTNVA | 39 | SASYRYS | 40 | QQYNAYPWT | 80 |
| G2-CH2-VL-P2 | KASQNVGTNVA | 39 | SASYRYS | 40 | QQYESYPWT | 81 |
| G2-CH2-VL-P3 | KASQNVGTNVA | 39 | SASYRYS | 40 | QQYNQYPWT | 82 |
| G2-H8-VL1(gsm) | KSSKSLLHSEGITYLY | 73 | QMSNLVS | 53 | AQNLELPPT | 54 |

It can be understood by those skilled in the art that CDRs of the same antibody determined using different numbering systems are not completely identical. It should be noted that the CDRs in the present application (especially in the claims) are determined and annotated using the Kabat numbering system. For the neutralizing antibodies screened in the present application, CDRs determined using other numbering systems are also within the protection scope of the present application, and other numbering systems include, but are not limited to, the IMGT, Chothia, or North numbering system.

Exemplarily, for the neutralizing antibodies screened in the present application, the CDRs determined using numbering systems such as IMGT, Chothia, North, Abm, or Contact are shown in Table 2.

**Table 2**

| IMGT | | | |
|---|---|---|---|
| Heavy chain name | CDR1 | CDR2 | CDR3 |
| G2-CH1 VH | GFSLSTYGIG | IWWNNNK | ARMGYDIMDY |
| G2-CH2 VH | GFSLTISGMG | IYWDDDK | VRAGWDFFDY |
| G2-CH3 VH | GFSLSTSGMG | IYWDDDK | ARTGWDFFDY |
| G2-CH5 VH | GYTLTDYN | LNPYNGGI | AREGRGGAWFDS |
| G2-CH6 VH | AYSFTDYN | INPYNGGT | AREVRLGNALDY |
| G2-CH7 VH | GYSFTGYN | INPSNGGS | AREVRLGNALDY |
| G2-CH8 VH | GFSLSTYGIG | IWWNDNK | ARLAYDYVDS |
| G2-CH9 VH | GYSFTGYN | INPYNGGT | AREVRLGNAMDY |
| G2-CH10 VH | GFSLSISGMG | IYWDDDK | ARSAYGMTDY |
| G2-CH12 VH | GYTFTNYD | IFPGDGST | ARILY |
| G2-CH13 VH | GYTFTDYN | INPYNGGI | AREVRRGSFFDY |
| G2-CH14 VH | GFSLSTSGMG | IYWDDDK | IQSPWDWFAY |
| G2-CH16 VH | GFSLSTSGMG | IYWDDDK | ARRAYGAMDY |
| G2-CH17 VH | GFSLNTSGMG | IYWDDDK | ARTGWDFFDI |
| G2-CH23 VH | GYTFSSYW | ILPGSGIT | ARETARARGFTY |
| G2-CH5-VH-P1 | GYTLTDYN | LNPYNAGI | AREGRGGAWFDS |
| G2-CH5-VH-P2 | GYTLTDYN | LNPYNQGI | AREGRGGAWFDS |
| G2-CH5-VH-P3 | GYTLTDYN | LNPYEGGI | AREGRGGAWFDS |

| Light chain name | CDR1 | CDR2 | CDR3 |
|---|---|---|---|
| G2-CH1 VL | QDVNTA | SSS | QQHYSTPWT |
| G2-CH2 VL | QNVGTN | SAS | QQYNSYPWT |
| G2-CH3 VL | QNVGTN | SAS | QQYNSYPYT |
| G2-CH5 VL | EDIYHR | GAT | QQYWSPPFT |
| G2-CH6 VL | ESIYSN | AAT | QHFWGIPWT |
| G2-CH7 VL | ENIYSN | AAT | QHFWGTPWT |
| G2-CH8 VL | KSLLHSNGITY | QMS | AQNLELPPT |
| G2-CH9 VL | ENIYSN | AAT | QHFWGTPWT |
| G2-CH10 VL | QDISNY | SAS | QQYNMFPWT |
| G2-CH12 VL | QDINSY | RAN | LQYDEFPYT |
| G2-CH13 VL | ENIYSN | AAT | QLFWGTPT |
| G2-CH14 VL | SSISSSN | GTS | QQWSSYPLT |
| G2-CH16 VL | QNVGTN | SAS | QQYNTYPHT |
| G2-CH17 VL | QNVGSN | SAS | QQYKSYPLT |
| G2-CH23 VL | IDIDDD | EGN | LQSDNLPLT |
| G2-CH8-VL-P1 | KSLLHSNAITY | QMS | AQNLELPPT |
| G2-CH8-VL-P2 | KSLLHSNQITY | QMS | AQNLELPPT |
| G2-CH8-VL-P3 | KSLLHSEGITY | QMS | AQNLELPPT |
| G2-CH2-VL-P1 | QNVGTN | SAS | QQYNAYPWT |
| G2-CH2-VL-P2 | QNVGTN | SAS | QQYESYPWT |
| G2-CH2-VL-P3 | QNVGTN | SAS | QQYNQYPWT |
| G2-H8-VL1(gsm) | KSLLHSEGITY | QMS | AQNLELPPT |

| Chothia | | | |
|---|---|---|---|
| Heavy chain name | CDR1 | CDR2 | CDR3 |
| G2-CH1 VH | GFSLSTYGIGVG | WWNNN | MGYDIMDY |
| G2-CH2 VH | GFSLTISGMGVS | YWDDD | AGWDFFDY |
| G2-CH3 VH | GFSLSTSGMGVS | YWDDD | TGWDFFDY |
| G2-CH5 VH | GYTLTDYNLN | NPYNGG | EGRGGAWFDS |
| G2-CH6 VH | AYSFTDYNMN | NPYNGG | EVRLGNALDY |
| G2-CH7 VH | GYSFTGYNMN | NPSNGG | EVRLGNALDY |
| G2-CH8 VH | GFSLSTYGIGVG | WWNDN | LAYDYVDS |
| G2-CH9 VH | GYSFTGYNMN | NPYNGG | EVRLGNAMDY |
| G2-CH10 VH | GFSLSISGMGVS | YWDDD | SAYGMTDY |
| G2-CH12 VH | GYTFTNYDIN | FPGDGS | ILY |
| G2-CH13 VH | GYTFTDYNMN | NPYNGG | EVRRGSFFDY |
| G2-CH14 VH | GFSLSTSGMGVS | YWDDD | SPWDWFAY |
| G2-CH16 VH | GFSLSTSGMGVS | YWDDD | RAYGAMDY |
| G2-CH17 VH | GFSLNTSGMGVS | YWDDD | TGWDFFDI |
| G2-CH23 VH | GYTFSSYWIE | LPGSGI | ETARARGFTY |
| G2-CH5-VH-P1 | GYTLTDY | NPYNAG | EGRGGAWFDS |
| G2-CH5-VH-P2 | GYTLTDY | NPYNQG | EGRGGAWFDS |
| G2-CH5-VH-P3 | GYTLTDY | NPYEGG | EGRGGAWFDS |

| Light chain name | CDR1 | CDR2 | CDR3 |
|---|---|---|---|
| G2-CH1 VL | KASQDVNTAVA | SSSYRYT | QQHYSTPWT |
| G2-CH2 VL | KASQNVGTNVA | SASYRYS | QQYNSYPWT |
| G2-CH3 VL | KASQNVGTNVA | SASYRYS | QQYNSYPYT |
| G2-CH5 VL | KASEDIYHRLA | GATSLET | QQYWSPPFT |
| G2-CH6 VL | RPSESIYSNLA | AATNLAD | QHFWGIPWT |
| G2-CH7 VL | RASENIYSNLA | AATNLGD | QHFWGTPWT |
| G2-CH8 VL | RSSKSLLHSNGITYLY | QMSNLVS | AQNLELPPT |
| G2-CH9 VL | RASENIYSNLA | AATNLAD | QHFWGTPWT |
| G2-CH10 VL | RASQDISNYLN | SASNLHS | QQYNMFPWT |
| G2-CH12 VL | KASQDINSYLS | RANKLVD | LQYDEFPYT |
| G2-CH13 VL | RASENIYSNLA | AATDLAD | QLFWGTPT |
| G2-CH14 VL | SVSSSISSSNLH | GTSNLAS | QQWSSYPLT |
| G2-CH16 VL | KASQNVGTNIA | SASYRYS | QQYNTYPHT |
| G2-CH17 VL | KASQNVGSNVA | SASYRYS | QQYKSYPLT |
| G2-CH23 VL | ITSIDIDDDIN | EGNTLRP | LQSDNLPLT |
| G2-CH8-VL-P1 | RSSKSLLHSNAITYLY | QMSNLVS | AQNLELPPT |
| G2-CH8-VL-P2 | RSSKSLLHSNQITYLY | QMSNLVS | AQNLELPPT |
| G2-CH8-VL-P3 | RSSKSLLHSEGITYLY | QMSNLVS | AQNLELPPT |
| G2-CH2-VL-P1 | KASQNVGTNVA | SASYRYS | QQYNAYPWT |
| G2-CH2-VL-P2 | KASQNVGTNVA | SASYRYS | QQYESYPWT |
| G2-CH2-VL-P3 | KASQNVGTNVA | SASYRYS | QQYNQYPWT |
| G2-H8-VL1(gsm) | KSLLHSEGITY | QMSNLVS | AQNLELPPT |

| North | | | |
|---|---|---|---|
| Heavy chain name | CDR1 | CDR2 | CDR3 |
| G2-CH1 VH | SFSGFSLSTYGIGVG | HIWWNNNKY | ARMGYDIMDY |
| G2-CH2 VH | SFSGFSLTISGMGVS | HIYWDDDKR | VRAGWDFFDY |
| G2-CH3 VH | SFSGFSLSTSGMGVS | HIYWDDDKR | ARTGWDFFDY |
| G2-CH5 VH | KASGYTLTDYNLN | DLNPYNGGII | AREGRGGAWFDS |
| G2-CH6 VH | KASAYSFTDYNMN | LINPYNGGTR | AREVRLGNALDY |
| G2-CH7 VH | KASGYSFTGYNMN | LINPSNGGSS | AREVRLGNALDY |
| G2-CH8 VH | SFSGFSLSTYGIGVG | HIWWNDNKY | ARLAYDYVDS |
| G2-CH9 VH | KASGYSFTGYNMN | LINPYNGGTI | AREVRLGNAMDY |
| G2-CH10 VH | SFSGFSLSISGMGVS | HIYWDDDKR | ARSAYGMTDY |
| G2-CH12 VH | KASGYTFTNYDIN | WIFPGDGSTK | ARILY |
| G2-CH13 VH | KASGYTFTDYNMN | DINPYNGGII | AREVRRGSFFDY |
| G2-CH14 VH | SFSGFSLSTSGMGVS | LIYWDDDKR | IQSPWDWFAY |
| G2-CH16 VH | SFSGFSLSTSGMGVS | HIYWDDDKR | ARRAYGAMDY |
| G2-CH17 VH | SFSGFSLNTSGMGVS | HIYWDDDKR | ARTGWDFFDI |
| G2-CH23 VH | KATGYTFSSYWIE | EILPGSGITN | ARETARARGFTY |
| G2-CH5-VH-P1 | KASGYTLTDYNLN | DLNPYNAGII | AREGRGGAWFDS |
| G2-CH5-VH-P2 | KASGYTLTDYNLN | DLNPYNQGII | AREGRGGAWFDS |
| G2-CH5-VH-P3 | KASGYTLTDYNLN | DLNPYEGGII | AREGRGGAWFDS |

| Light chain name | CDR1 | CDR2 | CDR3 |
|---|---|---|---|
| G2-CH1 VL | KASQDVNTAVA | YSSSYRYT | QQHYSTPWT |
| G2-CH2 VL | KASQNVGTNVA | YSASYRYS | QQYNSYPWT |
| G2-CH3 VL | KASQNVGTNVA | YSASYRYS | QQYNSYPYT |
| G2-CH5 VL | KASEDIYHRLA | SGATSLET | QQYWSPPFT |
| G2-CH6 VL | RPSESIYSNLA | YAATNLAD | QHFWGIPWT |
| G2-CH7 VL | RASENIYSNLA | YAATNLGD | QHFWGTPWT |
| G2-CH8 VL | RSSKSLLHSNGITYLY | YQMSNLVS | AQNLELPPT |
| G2-CH9 VL | RASENIYSNLA | SAATNLAD | QHFWGTPWT |
| G2-CH10 VL | RASQDISNYLN | HSASNLHS | QQYNMFPWT |
| G2-CH12 VL | KASQDINSYLS | YRANKLVD | LQYDEFPYT |
| G2-CH13 VL | RASENIYSNLA | YAATDLAD | QLFWGTPT |
| G2-CH14 VL | SVSSSISSSNLH | YGTSNLAS | QQWSSYPLT |
| G2-CH16 VL | KASQNVGTNIA | YSASYRYS | QQYNTYPHT |
| G2-CH17 VL | KASQNVGSNVA | YSASYRYS | QQYKSYPLT |
| G2-CH23 VL | ITSIDIDDDIN | SEGNTLRP | LQSDNLPLT |
| G2-CH8-VL-P1 | RSSKSLLHSNAITYLY | YQMSNLVS | AQNLELPPT |
| G2-CH8-VL-P2 | RSSKSLLHSNQITYLY | YQMSNLVS | AQNLELPPT |
| G2-CH8-VL-P3 | RSSKSLLHSEGITYLY | YQMSNLVS | AQNLELPPT |
| G2-CH2-VL-P1 | KASQNVGTNVA | YSASYRYS | QQYNAYPWT |
| G2-CH2-VL-P2 | KASQNVGTNVA | YSASYRYS | QQYESYPWT |
| G2-CH2-VL-P3 | KASQNVGTNVA | YSASYRYS | QQYNQYPWT |
| G2-H8-VL1(gsm) | KSSKSLLHSEGITYLY | YQMSNLVS | AQNLELPPT |

According to an embodiment of the present application, the antibody comprises: heavy chain variable region CDR1, CDR2, and CDR3 sequences as set forth in the amino acid sequences of SEQ ID NOs: 1, 2, and 3, respectively, and light chain variable region CDR1, CDR2, and CDR3 sequences as set forth in the amino acid sequences of SEQ ID NOs: 36, 37, and 38, respectively; or heavy chain variable region CDR1, CDR2, and CDR3 sequences as set forth in the amino acid sequences of SEQ ID NOs: 4, 5, and 6, respectively, and light chain variable region CDR1, CDR2, and CDR3 sequences as set forth in the amino acid sequences of SEQ ID NOs: 39, 40, and 41, respectively; or heavy chain variable region CDR1, CDR2, and CDR3 sequences as set forth in the amino acid sequences of SEQ ID NOs: 7, 5, and 8, respectively, and light chain variable region CDR1, CDR2, and CDR3 sequences as set forth in the amino acid sequences of SEQ ID NOs: 39, 40, and 42, respectively; or heavy chain variable region CDR1, CDR2, and CDR3 sequences as set forth in the amino acid sequences of SEQ ID NOs: 9, 10, and 11, respectively, and light chain variable region CDR1, CDR2, and CDR3 sequences as set forth in the amino acid sequences of SEQ ID NOs: 43, 44, and 45, respectively; or heavy chain variable region CDR1, CDR2, and CDR3 sequences as set forth in the amino acid sequences of SEQ ID NOs: 12, 13, and 14, respectively, and light chain variable region CDR1, CDR2, and CDR3 sequences as set forth in the amino acid sequences of SEQ ID NOs: 46, 47, and 48, respectively; or heavy chain variable region CDR1, CDR2, and CDR3 sequences as set forth in the amino acid sequences of SEQ ID NOs: 15, 16, and 14, respectively, and light chain variable region CDR1, CDR2, and CDR3 sequences as set forth in the amino acid sequences of SEQ ID NOs: 49, 50, and 51, respectively; or heavy chain variable region CDR1, CDR2, and CDR3 sequences as set forth in the amino acid sequences of SEQ ID NOs: 1, 17, and 18, respectively, and light chain variable region CDR1, CDR2, and CDR3 sequences as set forth in the amino acid sequences of SEQ ID NOs: 52, 53, and 54, respectively; or heavy chain variable region CDR1, CDR2, and CDR3 sequences as set forth in the amino acid sequences of SEQ ID NOs: 15, 19, and 20, respectively, and light chain variable region CDR1, CDR2, and CDR3 sequences as set forth in the amino acid sequences of SEQ ID NOs: 49, 47, and 51, respectively; or heavy chain variable region CDR1, CDR2, and CDR3 sequences as set forth in the amino acid sequences of SEQ ID NOs: 4, 21, and 22, respectively, and light chain variable region CDR1, CDR2, and CDR3 sequences as set forth in the amino acid sequences of SEQ ID NOs: 55, 56, and 57, respectively; or heavy chain variable region CDR1, CDR2, and CDR3 sequences as set forth in the amino acid sequences of SEQ ID NOs: 23, 24, and 25, respectively, and light chain variable region CDR1, CDR2, and CDR3 sequences as set forth in the amino acid sequences of SEQ ID NOs: 58, 59, and 60, respectively; or heavy chain variable region CDR1, CDR2, and CDR3 sequences as set forth in the amino acid sequences of SEQ ID NOs: 12, 26, and 27, respectively, and light chain variable region CDR1, CDR2, and CDR3 sequences as set forth in the amino acid sequences of SEQ ID NOs: 49, 61, and 62, respectively; or heavy chain variable region CDR1, CDR2, and CDR3 sequences as set forth in the amino acid sequences of SEQ ID NOs: 7, 28, and 29, respectively, and light chain variable region CDR1, CDR2, and CDR3 sequences as set forth in the amino acid sequences of SEQ ID NOs: 63, 64, and 65, respectively; or heavy chain variable region CDR1, CDR2, and CDR3 sequences as set forth in the amino acid sequences of SEQ ID NOs: 7, 5, and 30, respectively, and light chain variable region CDR1, CDR2, and CDR3 sequences as set forth in the amino acid sequences of SEQ ID NOs: 66, 40, and 67, respectively; or heavy chain variable region CDR1, CDR2, and CDR3 sequences as set forth in the amino acid sequences of SEQ ID NOs: 7, 5, and 31, respectively, and light chain variable region CDR1, CDR2, and CDR3 sequences as set forth in the amino acid sequences of SEQ ID NOs: 68, 40, and 69, respectively; or heavy chain variable region CDR1, CDR2, and CDR3 sequences as set forth in the amino acid sequences of SEQ ID NOs: 32, 33, and 34, respectively, and light chain variable region CDR1, CDR2, and CDR3 sequences as set forth in the amino acid sequences of SEQ ID NOs: 70, 71, and 72, respectively; or heavy chain variable region CDR1, CDR2, and CDR3 sequences as set forth in the amino acid sequences of SEQ ID NOs: 9, 74, and 11, respectively, and light chain variable region CDR1, CDR2, and CDR3 sequences as set forth in the amino acid sequences of SEQ ID NOs: 43, 44, and 45, respectively; or heavy chain variable region CDR1, CDR2, and CDR3 sequences as set forth in the amino acid sequences of SEQ ID NOs: 9, 75, and 11, respectively, and light chain variable region CDR1, CDR2, and CDR3 sequences as set forth in the amino acid sequences of SEQ ID NOs: 43, 44, and 45, respectively; or heavy chain variable region CDR1, CDR2, and CDR3 sequences as set forth in the amino acid sequences of SEQ ID NOs: 9, 76, and 11, respectively, and light chain variable region CDR1, CDR2, and CDR3 sequences as set forth in the amino acid sequences of SEQ ID NOs: 43, 44, and 45, respectively; or heavy chain variable region CDR1, CDR2, and CDR3 sequences as set forth in the amino acid sequences of SEQ ID NOs: 1, 17, and 18, respectively, and light chain variable region CDR1, CDR2, and CDR3 sequences as set forth in the amino acid sequences of SEQ ID NOs: 77, 53, and 54, respectively; or heavy chain variable region CDR1, CDR2, and CDR3 sequences as set forth in the amino acid sequences of SEQ ID NOs: 1, 17, and 18, respectively, and light chain variable region CDR1, CDR2, and CDR3 sequences as set forth in the amino acid sequences of SEQ ID NOs: 78, 53, and 54, respectively; or heavy chain variable region CDR1, CDR2, and CDR3 sequences as set forth in the amino acid sequences of SEQ ID NOs: 1, 17, and 18, respectively, and light chain variable region CDR1, CDR2, and CDR3 sequences as set forth in the amino acid sequences of SEQ ID NOs: 79, 53, and 54, respectively; or heavy chain variable region CDR1, CDR2, and CDR3 sequences as set forth in the amino acid sequences of SEQ ID NOs: 4, 5, and 6, respectively, and light chain variable region CDR1, CDR2, and CDR3 sequences as set forth in the amino acid sequences of SEQ ID NOs: 39, 40, and 80, respectively; or heavy chain variable region CDR1, CDR2, and CDR3 sequences as set forth in the amino acid sequences of SEQ ID NOs: 4, 5, and 6, respectively, and light chain variable region CDR1, CDR2, and CDR3 sequences as set forth in the amino acid sequences of SEQ ID NOs: 39, 40, and 81, respectively; or heavy chain variable region CDR1, CDR2, and CDR3 sequences as set forth in the amino acid sequences of SEQ ID NOs: 4, 5, and 6, respectively, and light chain variable region CDR1, CDR2, and CDR3 sequences as set forth in the amino acid sequences of SEQ ID NOs: 39, 40, and 82, respectively; or heavy chain variable region CDR1, CDR2, and CDR3 sequences as set forth in the amino acid sequences of SEQ ID NOs: 1, 17, and 18, respectively, and light chain variable region CDR1, CDR2, and CDR3 sequences as set forth in the amino acid sequences of SEQ ID NOs: 73, 53, and 54, respectively.

According to an embodiment of the present application, the antibody or antigen-binding fragment thereof specifically recognizes GDF15.

According to an embodiment of the present application, the antibody further comprises: at least one of a heavy chain framework region sequence and a light chain framework region sequence; wherein at least a portion of at least one of the heavy chain framework region sequence and the light chain framework region sequence is derived from at least one of a murine antibody, a human antibody, a primate antibody, or a mutant thereof.

According to an embodiment of the present application, at least a portion of the heavy chain framework region sequence and the light chain framework region sequence is derived from a human antibody or a mutant thereof.

According to an embodiment of the present application, the antibody has a heavy chain variable region of the amino acid sequence as set forth in any one of SEQ ID NOs: 83 to 100, SEQ ID NOs: 124 to 127, SEQ ID NOs: 132 to 135, SEQ ID NOs: 140 to 141, and SEQ ID NOs: 144 to 145.
G2-CH1 VH:
   QVTLKESGPGILQPSQTLSLTCSFSGFSLSTYGIGVGWIRQPSGKGLEWLAHIWWNN NKYFNTALKSRLTISKDTSNNQVFLKIASVDTADSATYYCARMGYDIMDYWGQGTSVT VSS (SEQ ID NO: 83, wherein the underlined portions are, in order, the heavy chain variable region CDR1, CDR2, and CDR3 as determined by the Kabat numbering system);
G2-CH2 VH: 
QVTLKESGPGILQPSQTLSLTCSFSGFSLTISGMGVSWIRQPSGKGLEWLAHIYWDD
DKRYNPSLKSRLTISKDTSSNQVFLEITSVDTADTATYYCVRAGWDFFDYWGQGTTLTV
SS (SEQ ID NO: 84);
G2-CH3 VH: 
QVTLKESGPGILQPSQTLSLTCSFSGFSLSTSGMGVSWIRQPSGKGLEWLAHIYWDD
DKRYNPSLKSRLTISKDTSSNRVFLKITSVDTADTATYYCARTGWDFFDYWGQGTTLTV
SS (SEQ ID NO: 85);
G2-CH5 VH: 
EVLLQQSGPELVKPGASVKIACKASGYTLTDYNLNWVKQSHGKSLEWIGDLNPYN
GGIIYNQKFKGKATLTVDKSSSTAYMELRSLTSEDIAVYYCAREGRGGAWFDSWGQGT
LVTVSA (SEQ ID NO: 86);
G2-CH6 VH: 
EVQLRQSGPELVKPGTSMKISCKASAYSFTDYNMNWVKQSHGKNLEWIGLINPYN
GGTRYNQNFKGKATITVDRSSTTAYMELLSLTSEDSAIYYCAREVRLGNALDYWGQGT
SVTVSS (SEQ ID NO: 87);
G2-CH7 VH: 
EVQLQQSGPELVKPGASLKISCKASGYSFTGYNMNWVKQSHGKNLEWIGLINPSN
GGSSYSQKFKGKATLTVDKSSSTAYMELLSLTSKDSAVYYCAREVRLGNALDYWGQG
TSVTVSS (SEQ ID NO: 88);
G2-CH8 VH: 
QVTLKESGPGILQPSQTLSLTCSFSGFSLSTYGIGVGWIRQPSGKGLEWLAHIWWND
NKYYNTALKSRLTISKDTSNNQVFLKIASVDTADTATYYCARLAYDYVDSWGQGTTLT
VSS (SEQ ID NO: 89);
G2-CH9 VH: 
EVQLQQSGPELVKPGASMKISCKASGYSFTGYNMNWVKQSQGKNLEWIGLINPYN
GGTIYNQKFKGKATLTVDKSSTTAYMELLSLTSEDSAVYYCAREVRLGNAMDYWGQG
TSVTVSS (SEQ ID NO: 90);
G2-CH10 VH: 
QGTLKESGPGILQPSQTLSLTCSFSGFSLSISGMGVSWIRQPSGKGLEWLAHIYWDD
DKRYKPSLKSRLTISKDTSRNQVFLKITSVDTADTATYYCARSAYGMTDYWGQGTTLT
VSS (SEQ ID NO: 91);
G2-CH12 VH: 
QVQLQQSGAELVKPGASVKLSCKASGYTFTNYDINWVRQRPEQGLEWIGWIFPGD
GSTKYNEKFKDKATLTTDTSSSTAYMQLIRLTSEDSAVYFCARILYWGQGTTLTVSS
(SEQ ID NO: 92);
G2-CH13 VH: 
EVLLQQSGPELVKPGASVKITCKASGYTFTDYNMNWVKQSHGKSLEWIGDINPYN
GGIIYNQKFRGKATLTIDKSSSTAYMELRSLTSEDTAVYYCAREVRRGSFFDYWGQGTT
LTVSS (SEQ ID NO: 93);
G2-CH14 VH: 
QVTLKESGPGILQPSQTLSLTCSFSGFSLSTSGMGVSWIRQPSGKGLEWLALIYWDD
DKRYNPSLKSRLTISKDASSNQVFLKITSVDTADTATYYCIQSPWDWFAYWGQGTLVTV
 SA (SEQ ID NO: 94);
G2-CH16 VH: 
QVTLKESGPGILQPSQTLSLTCSFSGFSLSTSGMGVSWIRQPSGKGLEWLAHIYWDD
DKRYNPSLKSRLTISKDTSRNQVFLKITSVDTADTATYYCARRAYGAMDYWGHGTSVT
VSS (SEQ ID NO: 95);
G2-CH17 VH: 
QVTLKESGPGILQPSQTLSLTCSFSGFSLNTSGMGVSWIRQPSGKGLEWLAHIYWDD
DKRYNPSLKSRLTISKDTSRNQVFLKITGVDTADTAAYYCARTGWDFFDIWGQGTTLTV
SS (SEQ ID NO: 96);
G2-CH23 VH: 
QVQLQQSGAELMKPGASVKISCKATGYTFSSYWIEWVKQRPGHGLEWIGEILPGSG
ITNYNEKFKGKATFTADTSSNTAYMQLSSLTSEDSAVYYCARETARARGFTYWGQGTL
VTVSA (SEQ ID NO: 97);
G2-CH5-VH-P1: 
EVLLQQSGPELVKPGASVKIACKASGYTLTDYNLNWVKQSHGKSLEWIGDLNPYN
AGIIYNQKFKGKATLTVDKSSSTAYMELRSLTSEDIAVYYCAREGRGGAWFDSWGQGT
LVTVSA (SEQ ID NO: 98);
G2-CH5-VH-P2: 
EVLLQQSGPELVKPGASVKIACKASGYTLTDYNLNWVKQSHGKSLEWIGDLNPYN
QGIIYNQKFKGKATLTVDKSSSTAYMELRSLTSEDIAVYYCAREGRGGAWFDSWGQGT
LVTVSA (SEQ ID NO: 99);
G2-CH5-VH-P3: 
EVLLQQSGPELVKPGASVKIACKASGYTLTDYNLNWVKQSHGKSLEWIGDLNPYE
GGIIYNQKFKGKATLTVDKSSSTAYMELRSLTSEDIAVYYCAREGRGGAWFDSWGQGT
LVTVSA (SEQ ID NO: 100);
G2-H1-VH_2(4-39): 
QVQLQESGPGLVKPSETLSLTCTFSGFSLSTYGIGVGWIRQPPGKGLEWIAHIWWNN
NKYFNTALKSRLTISKDTSKNQVSLKLSSVTAADTAVYYCARMGYDIMDYWGQGTLV
TVSS (SEQ ID NO: 124);
G2-H1-VH_3(4-39): 
QVTLQESGPGLVKPSETLSLTCSFSGFSLSTYGIGVGWIRQPPGKGLEWLAHIWWN
NNKYFNTALKSRLTISKDTSKNQVSLKLSSVTAADTATYYCARMGYDIMDYWGQGTSV
TVSS (SEQ ID NO: 125);
G2-H1-VH_2(3-23): 
EVQLLESGGGLVQPGGSLRLSCAFSGFSLSTYGIGVGWIRQAPGKGLEWVAHIWW
NNNKYFNTALKSRLTISKDTSKNTVYLQMNSLRAEDTAVYYCAKMGYDIMDYWGQGT
TVTVSS (SEQ ID NO: 126);
G2-H1-VH_3(3-23): 
QVTLLESGGGLVQPGGSLRLSCSFSGFSLSTYGIGVGWIRQAPGKGLEWLAHIWWN
NNKYFNTALKSRLTISKDTSKNTVYLQMNSVRAEDTAVYYCARMGYDIMDYWGQGTT
VTVSS (SEQ ID NO: 127);
G2-H5-VH_1: 
EVQLVQSGAEVKKPGASVKVSCKASGYTFTDYNLNWVRQATGQGLEWMGDLNP
YNQGIIYNQKFKGRVTMTVDTSISTAYMELSSLRSEDTAVYYCAREGRGGAWFDSWGQ
GTLVTVSS (SEQ ID NO: 132);
G2-H5-VH_2: 
EVQLVQSGAEVKKPGASVKVSCKASGYTFTDYNLNWVRQATGQGLEWMGDLNP
YNQGIIYNQKFKGRVTLTVDKSISTAYMELSSLRSEDTAVYYCAREGRGGAWFDSWGQ
GTLVTVSS (SEQ ID NO: 133);
G2-H5-VH_3: 
EVQLVQSGAEVKKPGASVKVSCKASGYTFTDYNLNWVRQATGQGLEWIGDLNPY
NQGIIYNQKFKGRATLTVDKSISTAYMELSSLRSEDTAVYYCAREGRGGAWFDSWGQG
TLVTVSS (SEQ ID NO: 134);
G2-H5-VH_4: 
EVQLVQSGAEVKKPGASVKVSCKASGYTLTDYNLNWVRQATGQGLEWIGDLNPY
NQGIIYNQKFKGRATLTVNKSISTAYMELSSLRSEDTAVYYCAREGRGGAWFDSWGQG
TLVTVSS (SEQ ID NO: 135);
G2-H8-VH_1: 
QVTLKESGPALVKPTQTLTLTCTFSGFSLSTYGIGVGWIRQPPGKALEWLAHIWWN
DNKYYNTALKSRLTISKDTSKNQVVLTMTNMDPVDTATYYCARLAYDYVDSWGQGTT
 LTVSS (SEQ ID NO: 140);
G2-H8-VH_2: 
QVTLKESGPALVKPTQTLTLTCTFSGFSLSTYGIGVGWIRQPPGKGLEWLAHIWWN
DNKYYNTALKSRLTISKDTSKNQVVLTMTNMDPVDTATYYCARLAYDYVDSWGQGTT
LTVSS (SEQ ID NO: 141);
G2-H8-VH1(gsm): 
QITLKESGPTLVKPTQTLTLTCTFSGFSLSTYGIGVGWIRQPPGKALEWLAHIWWND
NKYYNTALKSRLTITKDTSKNQVVLTMTNMDPVDTATYYCARLAYDYVDSWGQGTLV
TVSS (SEQ ID NO: 144);
G2-H8-VH2(gsm):

QITLKESGPALVKPTQTLTLTCTFSGFSLSTYGIGVGWIRQPPGKALEWLAHIWWND
NKYYNTALKSRLTITKDTSKNQVFLTMTNMDPVDTATYYCARLAYDYVDSWGQGTLV
TVSS (SEQ ID NO: 145).

According to an embodiment of the present application, the antibody has a light chain variable region of the amino acid sequence as set forth in any one of SEQ ID NOs: 102 to 122, SEQ ID NOs: 128 to 131, SEQ ID NOs: 136 to 139, SEQ ID NOs: 142 to 143, and SEQ ID NOs: 146 to 147.
G2-CH1 VL: 
DIVMTQSHKFMSTSVGDRVSITCKASQDVNTAVAWYQQKPGQSPKFLIYSSSYRYT
GVPDRFTGSGSGTDFTFTISSVQAEDLAVYYCQQHYSTPWTFGGGTKLEIK (SEQ ID NO:
102);
G2-CH2 VL: 
DIVMTQSQKFMSTSVGDRVSVTCKASQNVGTNVAWYQQKPGQSPKGLIYSASYRY
SGVPDRFTGSGSGTDFTLTISNVQSEDLAEYFCQQYNSYPWTFGGGTKLEIK (SEQ ID
NO: 103);
G2-CH3 VL: 
DIVMTQSQKFMSTSVGDRVSVTCKASQNVGTNVAWYQQKPGQSPKGLIYSASYRY
SGVPDRFTGSGSGTDFTLTINNVQSEDLAEYFCQQYNSYPYTFGGGTKLEIK (SEQ ID
NO: 104);
G2-CH5 VL: 
DIQMTQSSSSFSVSLGDRVTITCKASEDIYHRLAWYQQKPGNAPRLLISGATSLETGI
PSRFSGSGSGKDYALSITSLQTEDVGTYYCQQYWSPPFTFGSGTKLEIK (SEQ ID NO:
105);
G2-CH6 VL: 
DIQMTQSPASLSVSVGETVTITCRPSESIYSNLAWYQQKQGKSPQLLVYAATNLAD
GVPSRFSGSGSGTQYSLKINSLQSEDFGTYYCQHFWGIPWTFGGGTKLEIK (SEQ ID NO:
106);
G2-CH7 VL: 
DIQMTQSPASLSVSVGETVTITCRASENIYSNLAWYQQKQGKSPQLLVYAATNLGD
GAPSRFSGSGSGTQYSLKINSLQSEDFGSYYCQHFWGTPWTFGGGTKLEIK (SEQ ID NO:
107);
G2-CH8 VL: 
DIVMTQAAFSNPVTLGTSASISCRSSKSLLHSNGITYLYWYLQKPGQSPQLLIYQMS
NLVSGVPDRFSSSGSGTDFTLRISRVEAEDVGVYYCAQNLELPPTFGAGTKLELK (SEQ
ID NO: 108);
G2-CH9 VL: 
DIQMTQSPASLSVSVGETVTITCRASENIYSNLAWYQQKQGKSPQLLVSAATNLAD
GVPSRFSGSGSGTQYSLKIISLQSVDFGSYYCQHFWGTPWTFGGGTKLEIK (SEQ ID NO:
109);
G2-CH10 VL: 
DIQMTQTTSSLSASLGDRVTISCRASQDISNYLNWYQQKPDGTVKLLIHSASNLHSG
VPSRFSGSGSGTDYSLTISNLEQEDIATYFCQQYNMFPWTFGGGTKLEIK (SEQ ID NO:
110);
G2-CH12 VL: 
DNKMTQSPSSMYASLGERVTITCKASQDINSYLSWFQQKPGKSPKTLIYRANKLVD
GVPSRFSGSGSGQDYSLTISSLEYEDMGIYHCLQYDEFPYTFGGGTKLEIK (SEQ ID NO:
111);
G2-CH13 VL: 
DIQMTQSPASLSVSVGETVTITCRASENIYSNLAWYQQKQGKSPQLLVYAATDLAD
GVPSRFSGSGSGTQYSLKINSLQSEDFGNYHCQLFWGTPTFGGGTKLEIK (SEQ ID NO:
 112);
G2-CH14 VL: 
EIVLTQSPALMAASPGEKVTITCSVSSSISSSNLHWYQQKSETSPKPWIYGTSNLASG
VPVRFSGSGSGTSYSLTISSMEAEDAATYYCQQWSSYPLTFGAGTKLELK (SEQ ID NO:
113);
G2-CH16 VL: 
DIVMTQSQKFMSTSVGDRVSVTCKASQNVGTNIAWFQQKPGQSPEALIYSASYRYS
GVPDRFTGSGSGTDFTLTISNVQSEDLAEYFCQQYNTYPHTFGGGTKLEIR (SEQ ID NO:
114);
G2-CH17 VL: 
DIVMTQSQKFMSTSVGDRVSVTCKASQNVGSNVAWYQQKPGQSPKALIYSASYRY
SGVPDRFTGSGSGTDFTLTITNVQSEDLAEYFCQQYKSYPLTFGAGTKLELK (SEQ ID
NO: 115);
G2-CH23 VL: 
ETTVTQSPASLSMAIGEKVTIRCITSIDIDDDINWYQQKPGEPPNLLISEGNTLRPGVP
SRFSSSGYGTDFVFTIENMLSEDVADYYCLQSDNLPLTFGGGTKLEIK (SEQ ID NO: 116);
G2-CH8-VL-P1: 
DIVMTQAAFSNPVTLGTSASISCRSSKSLLHSNAITYLYWYLQKPGQSPQLLIYQMS
NLVSGVPDRFSSSGSGTDFTLRISRVEAEDVGVYYCAQNLELPPTFGAGTKLELK (SEQ
ID NO: 117);
G2-CH8-VL-P2: 
DIVMTQAAFSNPVTLGTSASISCRSSKSLLHSNQITYLYWYLQKPGQSPQLLIYQMS
NLVSGVPDRFSSSGSGTDFTLRISRVEAEDVGVYYCAQNLELPPTFGAGTKLELK (SEQ
ID NO: 118);
G2-CH8-VL-P3: 
DIVMTQAAFSNPVTLGTSASISCRSSKSLLHSEGITYLYWYLQKPGQSPQLLIYQMS
NLVSGVPDRFSSSGSGTDFTLRISRVEAEDVGVYYCAQNLELPPTFGAGTKLELK (SEQ
ID NO: 119);
G2-CH2-VL-P1: DIVMTQSQKFMSTSVGDRVSVTCKASQNVGTNVAWYQQKPGQSPKGLIYSASYRY 
SGVPDRFTGSGSGTDFTLTISNVQSEDLAEYFCQQYNAYPWTFGGGTKLEIK (SEQ ID
NO: 120);
G2-CH2-VL-P2: 
DIVMTQSQKFMSTSVGDRVSVTCKASQNVGTNVAWYQQKPGQSPKGLIYSASYRY
SGVPDRFTGSGSGTDFTLTISNVQSEDLAEYFCQQYESYPWTFGGGTKLEIK (SEQ ID
NO: 121);
G2-CH2-VL-P3: 
DIVMTQSQKFMSTSVGDRVSVTCKASQNVGTNVAWYQQKPGQSPKGLIYSASYRY
SGVPDRFTGSGSGTDFTLTISNVQSEDLAEYFCQQYNQYPWTFGGGTKLEIK (SEQ ID
NO: 122);
G2-H1-VL_2 (1-39): 
DIQMTQSPSSLSTSVGDRVTITCKASQDVNTAVAWYQQKPGKAPKFLIYSSSYRYT
GVPDRFSGSGSGTDFTFTISSLQPEDFAVYYCQQHYSTPWTFGQGTKVEIK (SEQ ID NO:
128);
G2-H1-VL_3 (1-39): 
DIVMTQSPSSLSTSVGDRVTITCKASQDVNTAVAWYQQKPGKSPKFLIYSSSYRYT
GVPDRFSGSGSGTDFTFTISSVQPEDFAVYYCQQHYSTPWTFGGGTKVEIK (SEQ ID NO:
129);
G2-H1-VL_2(3-15): 
DIVMTQSPATLSTSPGERATLSCKASQDVNTAVAWYQQKPGQAPRFLIYSSSYRYT
GVPARFSGSGSGTEFTFTISSLQSEDFAVYYCQQHYSTPWTFGGGTKVEIK (SEQ ID NO:
130);
G2-H1-VL_3(3-15): 
DIVMTQSPATLSTSPGERVTISCKASQDVNTAVAWYQQKPGQSPRFLIYSSSYRYTG
VPDRFSGSGSGTDFTFTISSVQSEDFAVYYCQQHYSTPWTFGGGTKVEIK (SEQ ID NO:
131);
G2-H5-VL_1: 
DIQMTQSPSSLSASVGDRVTITCKASEDIYHRLAWYQQKPGKAPKLLIYGATSLETG
VPSRFSGSGSGTDFTFTISSLQPEDIATYYCQQYWSPPFTFGGGTKVEIK (SEQ ID NO:
136);
G2-H5-VL_2: 
DIQMTQSPSSLSASVGDRVTITCKASEDIYHRLAWYQQKPGKAPKLLIYGATSLETG
VPSRFSGSGSGTDYTFTISSLQPEDIATYYCQQYWSPPFTFGGGTKVEIK (SEQ ID NO:
137);
G2-H5-VL_3: 
DIQMTQSPSSLSASVGDRVTITCKASEDIYHRLAWYQQKPGKAPKLLISGATSLETG
VPSRFSGSGSGTDYTFTISSLQPEDIATYYCQQYWSPPFTFGGGTKVEIK (SEQ ID NO:
138);
G2-H5-VL_4: 
DIQMTQSPSSLSASVGDRVTITCKASEDIYHRLAWYQQKPGKAPKLLISGATSLETG
IPSRFSGSGSGTDYTFTISSLQPEDIATYYCQQYWSPPFTFGGGTKVEIK (SEQ ID NO:
139);
G2-H8-VL_1: 
DIVMTQTPLSLSVTPGQPASISCRSSKSLLHSEGITYLYWYLQKPGQSPQLLIYQMSN
LVSGVPDRFSGSGSGTDFTLKISRVEAEDVGVYYCAQNLELPPTFGQGTKLEIK (SEQ ID
NO: 142);
G2-H8-VL_2: 
DIVMTQTPLSLSVTPGQPASISCRSSKSLLHSEGITYLYWYLQKPGQSPQLLIYQMSN
LVSGVPDRFSSSGSGTDFTLKISRVEAEDVGVYYCAQNLELPPTFGQGTKLEIK (SEQ ID
NO: 143);
G2-H8-VL1(gsm): 
DIVMTQTPLSLSVTPGQPASISCKSSKSLLHSEGITYLYWYLQKPGQSPQLLIYQMSN
LVSGVPDRFSGSGSGTDFTLKISRVEAEDVGVYYCAQNLELPPTFGQGTKLEIK (SEQ ID
NO: 146);
G2-H8-VL2(gsm):

DIVMTQSPLSLSVTPGEPASISCRSSKSLLHSEGITYLYWYLQKPGQSPQLLIYQMSN
LVSGVPDRFSGSGSGTDFTLKISRVEAEDVGVYYCAQNLELPPTFGQGTKLEIK (SEQ ID
NO: 147).

According to an embodiment of the present application, the antibody has a heavy chain variable region of the amino acid sequence as set forth in SEQ ID NO: 83 and a light chain variable region of the amino acid sequence as set forth in SEQ ID NO: 102.

According to an embodiment of the present application, the antibody has a heavy chain variable region of the amino acid sequence as set forth in SEQ ID NO: 84 and a light chain variable region of the amino acid sequence as set forth in SEQ ID NO: 103.

According to an embodiment of the present application, the antibody has a heavy chain variable region of the amino acid sequence as set forth in SEQ ID NO: 85 and a light chain variable region of the amino acid sequence as set forth in SEQ ID NO: 104.

According to an embodiment of the present application, the antibody has a heavy chain variable region of the amino acid sequence as set forth in SEQ ID NO: 86 and a light chain variable region of the amino acid sequence as set forth in SEQ ID NO: 105.

According to an embodiment of the present application, the antibody has a heavy chain variable region of the amino acid sequence as set forth in SEQ ID NO: 87 and a light chain variable region of the amino acid sequence as set forth in SEQ ID NO: 106.

According to an embodiment of the present application, the antibody has a heavy chain variable region of the amino acid sequence as set forth in SEQ ID NO: 88 and a light chain variable region of the amino acid sequence as set forth in SEQ ID NO: 107.

According to an embodiment of the present application, the antibody has a heavy chain variable region of the amino acid sequence as set forth in SEQ ID NO: 89 and a light chain variable region of the amino acid sequence as set forth in SEQ ID NO: 108.

According to an embodiment of the present application, the antibody has a heavy chain variable region of the amino acid sequence as set forth in SEQ ID NO: 90 and a light chain variable region of the amino acid sequence as set forth in SEQ ID NO: 109.

According to an embodiment of the present application, the antibody has a heavy chain variable region of the amino acid sequence as set forth in SEQ ID NO: 91 and a light chain variable region of the amino acid sequence as set forth in SEQ ID NO: 110.

According to an embodiment of the present application, the antibody has a heavy chain variable region of the amino acid sequence as set forth in SEQ ID NO: 92 and a light chain variable region of the amino acid sequence as set forth in SEQ ID NO: 111.

According to an embodiment of the present application, the antibody has a heavy chain variable region of the amino acid sequence as set forth in SEQ ID NO: 93 and a light chain variable region of the amino acid sequence as set forth in SEQ ID NO: 112.

According to an embodiment of the present application, the antibody has a heavy chain variable region of the amino acid sequence as set forth in SEQ ID NO: 94 and a light chain variable region of the amino acid sequence as set forth in SEQ ID NO: 113.

According to an embodiment of the present application, the antibody has a heavy chain variable region of the amino acid sequence as set forth in SEQ ID NO: 95 and a light chain variable region of the amino acid sequence as set forth in SEQ ID NO: 114.

According to an embodiment of the present application, the antibody has a heavy chain variable region of the amino acid sequence as set forth in SEQ ID NO: 96 and a light chain variable region of the amino acid sequence as set forth in SEQ ID NO: 115.

According to an embodiment of the present application, the antibody has a heavy chain variable region of the amino acid sequence as set forth in SEQ ID NO: 97 and a light chain variable region of the amino acid sequence as set forth in SEQ ID NO: 116.

According to an embodiment of the present application, the antibody has a heavy chain variable region of the amino acid sequence as set forth in SEQ ID NO: 98 and a light chain variable region of the amino acid sequence as set forth in SEQ ID NO: 105.

According to an embodiment of the present application, the antibody has a heavy chain variable region of the amino acid sequence as set forth in SEQ ID NO: 99 and a light chain variable region of the amino acid sequence as set forth in SEQ ID NO: 105.

According to an embodiment of the present application, the antibody has a heavy chain variable region of the amino acid sequence as set forth in SEQ ID NO: 100 and a light chain variable region of the amino acid sequence as set forth in SEQ ID NO: 105.

According to an embodiment of the present application, the antibody has a heavy chain variable region of the amino acid sequence as set forth in SEQ ID NO: 89 and a light chain variable region of the amino acid sequence as set forth in SEQ ID NO: 117.

According to an embodiment of the present application, the antibody has a heavy chain variable region of the amino acid sequence as set forth in SEQ ID NO: 89 and a light chain variable region of the amino acid sequence as set forth in SEQ ID NO: 118.

According to an embodiment of the present application, the antibody has a heavy chain variable region of the amino acid sequence as set forth in SEQ ID NO: 89 and a light chain variable region of the amino acid sequence as set forth in SEQ ID NO: 119.

According to an embodiment of the present application, the antibody has a heavy chain variable region of the amino acid sequence as set forth in SEQ ID NO: 84 and a light chain variable region of the amino acid sequence as set forth in SEQ ID NO: 120.

According to an embodiment of the present application, the antibody has a heavy chain variable region of the amino acid sequence as set forth in SEQ ID NO: 84 and a light chain variable region of the amino acid sequence as set forth in SEQ ID NO: 121.

According to an embodiment of the present application, the antibody has a heavy chain variable region of the amino acid sequence as set forth in SEQ ID NO: 84 and a light chain variable region of the amino acid sequence as set forth in SEQ ID NO: 122.

According to an embodiment of the present application, the antibody has a heavy chain variable region of the amino acid sequence as set forth in SEQ ID NO: 124 and a light chain variable region of the amino acid sequence as set forth in SEQ ID NO: 128.

According to an embodiment of the present application, the antibody has a heavy chain variable region of the amino acid sequence as set forth in SEQ ID NO: 124 and a light chain variable region of the amino acid sequence as set forth in SEQ ID NO: 129.

Optionally, the antibody has a heavy chain variable region of the amino acid sequence as set forth in SEQ ID NO: 124 and a light chain variable region of the amino acid sequence as set forth in SEQ ID NO: 130.

According to an embodiment of the present application, the antibody has a heavy chain variable region of the amino acid sequence as set forth in SEQ ID NO: 124 and a light chain variable region of the amino acid sequence as set forth in SEQ ID NO: 131.

According to an embodiment of the present application, the antibody has a heavy chain variable region of the amino acid sequence as set forth in SEQ ID NO: 125 and a light chain variable region of the amino acid sequence as set forth in SEQ ID NO: 128.

According to an embodiment of the present application, the antibody has a heavy chain variable region of the amino acid sequence as set forth in SEQ ID NO: 125 and a light chain variable region of the amino acid sequence as set forth in SEQ ID NO: 129.

According to an embodiment of the present application, the antibody has a heavy chain variable region of the amino acid sequence as set forth in SEQ ID NO: 125 and a light chain variable region of the amino acid sequence as set forth in SEQ ID NO: 130.

According to an embodiment of the present application, the antibody has a heavy chain variable region of the amino acid sequence as set forth in SEQ ID NO: 125 and a light chain variable region of the amino acid sequence as set forth in SEQ ID NO: 131.

According to an embodiment of the present application, the antibody has a heavy chain variable region of the amino acid sequence as set forth in SEQ ID NO: 126 and a light chain variable region of the amino acid sequence as set forth in SEQ ID NO: 128.

According to an embodiment of the present application, the antibody has a heavy chain variable region of the amino acid sequence as set forth in SEQ ID NO: 126 and a light chain variable region of the amino acid sequence as set forth in SEQ ID NO: 129.

According to an embodiment of the present application, the antibody has a heavy chain variable region of the amino acid sequence as set forth in SEQ ID NO: 126 and a light chain variable region of the amino acid sequence as set forth in SEQ ID NO: 130.

According to an embodiment of the present application, the antibody has a heavy chain variable region of the amino acid sequence as set forth in SEQ ID NO: 126 and a light chain variable region of the amino acid sequence as set forth in SEQ ID NO: 131.

According to an embodiment of the present application, the antibody has a heavy chain variable region of the amino acid sequence as set forth in SEQ ID NO: 127 and a light chain variable region of the amino acid sequence as set forth in SEQ ID NO: 128.

According to an embodiment of the present application, the antibody has a heavy chain variable region of the amino acid sequence as set forth in SEQ ID NO: 127 and a light chain variable region of the amino acid sequence as set forth in SEQ ID NO: 129.

According to an embodiment of the present application, the antibody has a heavy chain variable region of the amino acid sequence as set forth in SEQ ID NO: 127 and a light chain variable region of the amino acid sequence as set forth in SEQ ID NO: 130.

According to an embodiment of the present application, the antibody has a heavy chain variable region of the amino acid sequence as set forth in SEQ ID NO: 127 and a light chain variable region of the amino acid sequence as set forth in SEQ ID NO: 131.

According to an embodiment of the present application, the antibody has a heavy chain variable region of the amino acid sequence as set forth in SEQ ID NO: 132 and a light chain variable region of the amino acid sequence as set forth in SEQ ID NO: 136.

According to an embodiment of the present application, the antibody has a heavy chain variable region of the amino acid sequence as set forth in SEQ ID NO: 133 and a light chain variable region of the amino acid sequence as set forth in SEQ ID NO: 136.

According to an embodiment of the present application, the antibody has a heavy chain variable region of the amino acid sequence as set forth in SEQ ID NO: 134 and a light chain variable region of the amino acid sequence as set forth in SEQ ID NO: 136.

According to an embodiment of the present application, the antibody has a heavy chain variable region of the amino acid sequence as set forth in SEQ ID NO: 135 and a light chain variable region of the amino acid sequence as set forth in SEQ ID NO: 136.

According to an embodiment of the present application, the antibody has a heavy chain variable region of the amino acid sequence as set forth in SEQ ID NO: 132 and a light chain variable region of the amino acid sequence as set forth in SEQ ID NO: 137.

According to an embodiment of the present application, the antibody has a heavy chain variable region of the amino acid sequence as set forth in SEQ ID NO: 133 and a light chain variable region of the amino acid sequence as set forth in SEQ ID NO: 137.

According to an embodiment of the present application, the antibody has a heavy chain variable region of the amino acid sequence as set forth in SEQ ID NO: 134 and a light chain variable region of the amino acid sequence as set forth in SEQ ID NO: 137.

According to an embodiment of the present application, the antibody has a heavy chain variable region of the amino acid sequence as set forth in SEQ ID NO: 135 and a light chain variable region of the amino acid sequence as set forth in SEQ ID NO: 137.

According to an embodiment of the present application, the antibody has a heavy chain variable region of the amino acid sequence as set forth in SEQ ID NO: 132 and a light chain variable region of the amino acid sequence as set forth in SEQ ID NO: 138.

According to an embodiment of the present application, the antibody has a heavy chain variable region of the amino acid sequence as set forth in SEQ ID NO: 133 and a light chain variable region of the amino acid sequence as set forth in SEQ ID NO: 138.

According to an embodiment of the present application, the antibody has a heavy chain variable region of the amino acid sequence as set forth in SEQ ID NO: 134 and a light chain variable region of the amino acid sequence as set forth in SEQ ID NO: 138.

According to an embodiment of the present application, the antibody has a heavy chain variable region of the amino acid sequence as set forth in SEQ ID NO: 135 and a light chain variable region of the amino acid sequence as set forth in SEQ ID NO: 138.

According to an embodiment of the present application, the antibody has a heavy chain variable region of the amino acid sequence as set forth in SEQ ID NO: 132 and a light chain variable region of the amino acid sequence as set forth in SEQ ID NO: 139.

According to an embodiment of the present application, the antibody has a heavy chain variable region of the amino acid sequence as set forth in SEQ ID NO: 133 and a light chain variable region of the amino acid sequence as set forth in SEQ ID NO: 139.

According to an embodiment of the present application, the antibody has a heavy chain variable region of the amino acid sequence as set forth in SEQ ID NO: 134 and a light chain variable region of the amino acid sequence as set forth in SEQ ID NO: 139.

According to an embodiment of the present application, the antibody has a heavy chain variable region of the amino acid sequence as set forth in SEQ ID NO: 135 and a light chain variable region of the amino acid sequence as set forth in SEQ ID NO: 139.

According to an embodiment of the present application, the antibody has a heavy chain variable region of the amino acid sequence as set forth in SEQ ID NO: 140 and a light chain variable region of the amino acid sequence as set forth in SEQ ID NO: 142.

According to an embodiment of the present application, the antibody has a heavy chain variable region of the amino acid sequence as set forth in SEQ ID NO: 141 and a light chain variable region of the amino acid sequence as set forth in SEQ ID NO: 142.

According to an embodiment of the present application, the antibody has a heavy chain variable region of the amino acid sequence as set forth in SEQ ID NO: 140 and a light chain variable region of the amino acid sequence as set forth in SEQ ID NO: 143.

According to an embodiment of the present application, the antibody has a heavy chain variable region of the amino acid sequence as set forth in SEQ ID NO: 141 and a light chain variable region of the amino acid sequence as set forth in SEQ ID NO: 143.

According to an embodiment of the present application, the antibody has a heavy chain variable region of the amino acid sequence as set forth in SEQ ID NO: 144 and a light chain variable region of the amino acid sequence as set forth in SEQ ID NO: 146.

According to an embodiment of the present application, the antibody has a heavy chain variable region of the amino acid sequence as set forth in SEQ ID NO: 144 and a light chain variable region of the amino acid sequence as set forth in SEQ ID NO: 147.

According to an embodiment of the present application, the antibody has a heavy chain variable region of the amino acid sequence as set forth in SEQ ID NO: 145 and a light chain variable region of the amino acid sequence as set forth in SEQ ID NO: 146.

According to an embodiment of the present application, the antibody has a heavy chain variable region of the amino acid sequence as set forth in SEQ ID NO: 145 and a light chain variable region of the amino acid sequence as set forth in SEQ ID NO: 147.

According to an embodiment of the present application, the antibody comprises three heavy chain variable region CDRs from a heavy chain variable region of the amino acid sequence as set forth in SEQ ID NO: 144, and three light chain variable region CDRs from a light chain variable region of the amino acid sequence as set forth in SEQ ID NO: 146;
optionally, the antibody comprises three heavy chain variable region CDRs from a heavy chain variable region of the amino acid sequence as set forth in SEQ ID NO: 83, and three light chain variable region CDRs from a light chain variable region of the amino acid sequence as set forth in SEQ ID NO: 102;
optionally, the antibody comprises three heavy chain variable region CDRs from a heavy chain variable region of the amino acid sequence as set forth in SEQ ID NO: 84, and three light chain variable region CDRs from a light chain variable region of the amino acid sequence as set forth in SEQ ID NO: 103;
optionally, the antibody comprises three heavy chain variable region CDRs from a heavy chain variable region of the amino acid sequence as set forth in SEQ ID NO: 85, and three light chain variable region CDRs from a light chain variable region of the amino acid sequence as set forth in SEQ ID NO: 104;
optionally, the antibody comprises three heavy chain variable region CDRs from a heavy chain variable region of the amino acid sequence as set forth in SEQ ID NO: 86, and three light chain variable region CDRs from a light chain variable region of the amino acid sequence as set forth in SEQ ID NO: 105;
optionally, the antibody comprises three heavy chain variable region CDRs from a heavy chain variable region of the amino acid sequence as set forth in SEQ ID NO: 87, and three light chain variable region CDRs from a light chain variable region of the amino acid sequence as set forth in SEQ ID NO: 106;
optionally, the antibody comprises three heavy chain variable region CDRs from a heavy chain variable region of the amino acid sequence as set forth in SEQ ID NO: 88, and three light chain variable region CDRs from a light chain variable region of the amino acid sequence as set forth in SEQ ID NO: 107;
optionally, the antibody comprises three heavy chain variable region CDRs from a heavy chain variable region of the amino acid sequence as set forth in SEQ ID NO: 89, and three light chain variable region CDRs from a light chain variable region of the amino acid sequence as set forth in SEQ ID NO: 108;
optionally, the antibody comprises three heavy chain variable region CDRs from a heavy chain variable region of the amino acid sequence as set forth in SEQ ID NO: 90, and three light chain variable region CDRs from a light chain variable region of the amino acid sequence as set forth in SEQ ID NO: 109;
optionally, the antibody comprises three heavy chain variable region CDRs from a heavy chain variable region of the amino acid sequence as set forth in SEQ ID NO: 91, and three light chain variable region CDRs from a light chain variable region of the amino acid sequence as set forth in SEQ ID NO: 110;
optionally, the antibody comprises three heavy chain variable region CDRs from a heavy chain variable region of the amino acid sequence as set forth in SEQ ID NO: 92, and three light chain variable region CDRs from a light chain variable region of the amino acid sequence as set forth in SEQ ID NO: 111;
optionally, the antibody comprises three heavy chain variable region CDRs from a heavy chain variable region of the amino acid sequence as set forth in SEQ ID NO: 93, and three light chain variable region CDRs from a light chain variable region of the amino acid sequence as set forth in SEQ ID NO: 112;
optionally, the antibody comprises three heavy chain variable region CDRs from a heavy chain variable region of the amino acid sequence as set forth in SEQ ID NO: 94, and three light chain variable region CDRs from a light chain variable region of the amino acid sequence as set forth in SEQ ID NO: 113;
optionally, the antibody comprises three heavy chain variable region CDRs from a heavy chain variable region of the amino acid sequence as set forth in SEQ ID NO: 95, and three light chain variable region CDRs from a light chain variable region of the amino acid sequence as set forth in SEQ ID NO: 114;
optionally, the antibody comprises three heavy chain variable region CDRs from a heavy chain variable region of the amino acid sequence as set forth in SEQ ID NO: 96, and three light chain variable region CDRs from a light chain variable region of the amino acid sequence as set forth in SEQ ID NO: 115;
optionally, the antibody comprises three heavy chain variable region CDRs from a heavy chain variable region of the amino acid sequence as set forth in SEQ ID NO: 97, and three light chain variable region CDRs from a light chain variable region of the amino acid sequence as set forth in SEQ ID NO: 116;
optionally, the antibody comprises three heavy chain variable region CDRs from a heavy chain variable region of the amino acid sequence as set forth in SEQ ID NO: 98, and three light chain variable region CDRs from a light chain variable region of the amino acid sequence as set forth in SEQ ID NO: 105;
optionally, the antibody comprises three heavy chain variable region CDRs from a heavy chain variable region of the amino acid sequence as set forth in SEQ ID NO: 99, and three light chain variable region CDRs from a light chain variable region of the amino acid sequence as set forth in SEQ ID NO: 105;
optionally, the antibody comprises three heavy chain variable region CDRs from a heavy chain variable region of the amino acid sequence as set forth in SEQ ID NO: 100, and three light chain variable region CDRs from a light chain variable region of the amino acid sequence as set forth in SEQ ID NO: 105;
optionally, the antibody comprises three heavy chain variable region CDRs from a heavy chain variable region of the amino acid sequence as set forth in SEQ ID NO: 89, and three light chain variable region CDRs from a light chain variable region of the amino acid sequence as set forth in SEQ ID NO: 117;
optionally, the antibody comprises three heavy chain variable region CDRs from a heavy chain variable region of the amino acid sequence as set forth in SEQ ID NO: 89, and three light chain variable region CDRs from a light chain variable region of the amino acid sequence as set forth in SEQ ID NO: 118;
optionally, the antibody comprises three heavy chain variable region CDRs from a heavy chain variable region of the amino acid sequence as set forth in SEQ ID NO: 89, and three light chain variable region CDRs from a light chain variable region of the amino acid sequence as set forth in SEQ ID NO: 119;
optionally, the antibody comprises three heavy chain variable region CDRs from a heavy chain variable region of the amino acid sequence as set forth in SEQ ID NO: 84, and three light chain variable region CDRs from a light chain variable region of the amino acid sequence as set forth in SEQ ID NO: 120;
optionally, the antibody comprises three heavy chain variable region CDRs from a heavy chain variable region of the amino acid sequence as set forth in SEQ ID NO: 84, and three light chain variable region CDRs from a light chain variable region of the amino acid sequence as set forth in SEQ ID NO: 121;
optionally, the antibody comprises three heavy chain variable region CDRs from a heavy chain variable region of the amino acid sequence as set forth in SEQ ID NO: 84, and three light chain variable region CDRs from a light chain variable region of the amino acid sequence as set forth in SEQ ID NO: 122;
optionally, the antibody comprises three heavy chain variable region CDRs from a heavy chain variable region of the amino acid sequence as set forth in SEQ ID NO: 124, and three light chain variable region CDRs from a light chain variable region of the amino acid sequence as set forth in SEQ ID NO: 128;
optionally, the antibody comprises three heavy chain variable region CDRs from a heavy chain variable region of the amino acid sequence as set forth in SEQ ID NO: 124, and three light chain variable region CDRs from a light chain variable region of the amino acid sequence as set forth in SEQ ID NO: 129;
optionally, the antibody comprises three heavy chain variable region CDRs from a heavy chain variable region of the amino acid sequence as set forth in SEQ ID NO: 124, and three light chain variable region CDRs from a light chain variable region of the amino acid sequence as set forth in SEQ ID NO: 130;
optionally, the antibody comprises three heavy chain variable region CDRs from a heavy chain variable region of the amino acid sequence as set forth in SEQ ID NO: 124, and three light chain variable region CDRs from a light chain variable region of the amino acid sequence as set forth in SEQ ID NO: 131;
optionally, the antibody comprises three heavy chain variable region CDRs from a heavy chain variable region of the amino acid sequence as set forth in SEQ ID NO: 125, and three light chain variable region CDRs from a light chain variable region of the amino acid sequence as set forth in SEQ ID NO: 128;
optionally, the antibody comprises three heavy chain variable region CDRs from a heavy chain variable region of the amino acid sequence as set forth in SEQ ID NO: 125, and three light chain variable region CDRs from a light chain variable region of the amino acid sequence as set forth in SEQ ID NO: 129;
optionally, the antibody comprises three heavy chain variable region CDRs from a heavy chain variable region of the amino acid sequence as set forth in SEQ ID NO: 125, and three light chain variable region CDRs from a light chain variable region of the amino acid sequence as set forth in SEQ ID NO: 130;
optionally, the antibody comprises three heavy chain variable region CDRs from a heavy chain variable region of the amino acid sequence as set forth in SEQ ID NO: 125, and three light chain variable region CDRs from a light chain variable region of the amino acid sequence as set forth in SEQ ID NO: 131;
optionally, the antibody comprises three heavy chain variable region CDRs from a heavy chain variable region of the amino acid sequence as set forth in SEQ ID NO: 126, and three light chain variable region CDRs from a light chain variable region of the amino acid sequence as set forth in SEQ ID NO: 128;
optionally, the antibody comprises three heavy chain variable region CDRs from a heavy chain variable region of the amino acid sequence as set forth in SEQ ID NO: 126, and three light chain variable region CDRs from a light chain variable region of the amino acid sequence as set forth in SEQ ID NO: 129;
optionally, the antibody comprises three heavy chain variable region CDRs from a heavy chain variable region of the amino acid sequence as set forth in SEQ ID NO: 126, and three light chain variable region CDRs from a light chain variable region of the amino acid sequence as set forth in SEQ ID NO: 130;
optionally, the antibody comprises three heavy chain variable region CDRs from a heavy chain variable region of the amino acid sequence as set forth in SEQ ID NO: 126, and three light chain variable region CDRs from a light chain variable region of the amino acid sequence as set forth in SEQ ID NO: 131;
optionally, the antibody comprises three heavy chain variable region CDRs from a heavy chain variable region of the amino acid sequence as set forth in SEQ ID NO: 127, and three light chain variable region CDRs from a light chain variable region of the amino acid sequence as set forth in SEQ ID NO: 128;
optionally, the antibody comprises three heavy chain variable region CDRs from a heavy chain variable region of the amino acid sequence as set forth in SEQ ID NO: 127, and three light chain variable region CDRs from a light chain variable region of the amino acid sequence as set forth in SEQ ID NO: 129;
optionally, the antibody comprises three heavy chain variable region CDRs from a heavy chain variable region of the amino acid sequence as set forth in SEQ ID NO: 127, and three light chain variable region CDRs from a light chain variable region of the amino acid sequence as set forth in SEQ ID NO: 130;
optionally, the antibody comprises three heavy chain variable region CDRs from a heavy chain variable region of the amino acid sequence as set forth in SEQ ID NO: 127, and three light chain variable region CDRs from a light chain variable region of the amino acid sequence as set forth in SEQ ID NO: 131;
optionally, the antibody comprises three heavy chain variable region CDRs from a heavy chain variable region of the amino acid sequence as set forth in SEQ ID NO: 132, and three light chain variable region CDRs from a light chain variable region of the amino acid sequence as set forth in SEQ ID NO: 136;
optionally, the antibody comprises three heavy chain variable region CDRs from a heavy chain variable region of the amino acid sequence as set forth in SEQ ID NO: 133, and three light chain variable region CDRs from a light chain variable region of the amino acid sequence as set forth in SEQ ID NO: 136;
optionally, the antibody comprises three heavy chain variable region CDRs from a heavy chain variable region of the amino acid sequence as set forth in SEQ ID NO: 134, and three light chain variable region CDRs from a light chain variable region of the amino acid sequence as set forth in SEQ ID NO: 136;
optionally, the antibody comprises three heavy chain variable region CDRs from a heavy chain variable region of the amino acid sequence as set forth in SEQ ID NO: 135, and three light chain variable region CDRs from a light chain variable region of the amino acid sequence as set forth in SEQ ID NO: 136;
optionally, the antibody comprises three heavy chain variable region CDRs from a heavy chain variable region of the amino acid sequence as set forth in SEQ ID NO: 132, and three light chain variable region CDRs from a light chain variable region of the amino acid sequence as set forth in SEQ ID NO: 137;
optionally, the antibody comprises three heavy chain variable region CDRs from a heavy chain variable region of the amino acid sequence as set forth in SEQ ID NO: 133, and three light chain variable region CDRs from a light chain variable region of the amino acid sequence as set forth in SEQ ID NO: 137;
optionally, the antibody comprises three heavy chain variable region CDRs from a heavy chain variable region of the amino acid sequence as set forth in SEQ ID NO: 134, and three light chain variable region CDRs from a light chain variable region of the amino acid sequence as set forth in SEQ ID NO: 137;
optionally, the antibody comprises three heavy chain variable region CDRs from a heavy chain variable region of the amino acid sequence as set forth in SEQ ID NO: 135, and three light chain variable region CDRs from a light chain variable region of the amino acid sequence as set forth in SEQ ID NO: 137;
optionally, the antibody comprises three heavy chain variable region CDRs from a heavy chain variable region of the amino acid sequence as set forth in SEQ ID NO: 132, and three light chain variable region CDRs from a light chain variable region of the amino acid sequence as set forth in SEQ ID NO: 138;
optionally, the antibody comprises three heavy chain variable region CDRs from a heavy chain variable region of the amino acid sequence as set forth in SEQ ID NO: 133, and three light chain variable region CDRs from a light chain variable region of the amino acid sequence as set forth in SEQ ID NO: 138;
optionally, the antibody comprises three heavy chain variable region CDRs from a heavy chain variable region of the amino acid sequence as set forth in SEQ ID NO: 134, and three light chain variable region CDRs from a light chain variable region of the amino acid sequence as set forth in SEQ ID NO: 138;
optionally, the antibody comprises three heavy chain variable region CDRs from a heavy chain variable region of the amino acid sequence as set forth in SEQ ID NO: 135, and three light chain variable region CDRs from a light chain variable region of the amino acid sequence as set forth in SEQ ID NO: 138;
optionally, the antibody comprises three heavy chain variable region CDRs from a heavy chain variable region of the amino acid sequence as set forth in SEQ ID NO: 132, and three light chain variable region CDRs from a light chain variable region of the amino acid sequence as set forth in SEQ ID NO: 139;
optionally, the antibody comprises three heavy chain variable region CDRs from a heavy chain variable region of the amino acid sequence as set forth in SEQ ID NO: 133, and three light chain variable region CDRs from a light chain variable region of the amino acid sequence as set forth in SEQ ID NO: 139;
optionally, the antibody comprises three heavy chain variable region CDRs from a heavy chain variable region of the amino acid sequence as set forth in SEQ ID NO: 134, and three light chain variable region CDRs from a light chain variable region of the amino acid sequence as set forth in SEQ ID NO: 139;
optionally, the antibody comprises three heavy chain variable region CDRs from a heavy chain variable region of the amino acid sequence as set forth in SEQ ID NO: 135, and three light chain variable region CDRs from a light chain variable region of the amino acid sequence as set forth in SEQ ID NO: 139;
optionally, the antibody comprises three heavy chain variable region CDRs from a heavy chain variable region of the amino acid sequence as set forth in SEQ ID NO: 140, and three light chain variable region CDRs from a light chain variable region of the amino acid sequence as set forth in SEQ ID NO: 142;
optionally, the antibody comprises three heavy chain variable region CDRs from a heavy chain variable region of the amino acid sequence as set forth in SEQ ID NO: 141, and three light chain variable region CDRs from a light chain variable region of the amino acid sequence as set forth in SEQ ID NO: 142;
optionally, the antibody comprises three heavy chain variable region CDRs from a heavy chain variable region of the amino acid sequence as set forth in SEQ ID NO: 140, and three light chain variable region CDRs from a light chain variable region of the amino acid sequence as set forth in SEQ ID NO: 143;
optionally, the antibody comprises three heavy chain variable region CDRs from a heavy chain variable region of the amino acid sequence as set forth in SEQ ID NO: 141, and three light chain variable region CDRs from a light chain variable region of the amino acid sequence as set forth in SEQ ID NO: 143;
optionally, the antibody comprises three heavy chain variable region CDRs from a heavy chain variable region of the amino acid sequence as set forth in SEQ ID NO: 144, and three light chain variable region CDRs from a light chain variable region of the amino acid sequence as set forth in SEQ ID NO: 147;
optionally, the antibody comprises three heavy chain variable region CDRs from a heavy chain variable region of the amino acid sequence as set forth in SEQ ID NO: 145, and three light chain variable region CDRs from a light chain variable region of the amino acid sequence as set forth in SEQ ID NO: 146;
optionally, the antibody comprises three heavy chain variable region CDRs from a heavy chain variable region of the amino acid sequence as set forth in SEQ ID NO: 145, and three light chain variable region CDRs from a light chain variable region of the amino acid sequence as set forth in SEQ ID NO: 147;
the above CDR sequences further comprise an amino acid sequence having at least 66% identity thereto, and the above CDRs are determined according to the Kabat, IMGT, Chothia, or North numbering system.

According to an embodiment of the present application, the antibody contains at least one of a heavy chain constant region and a light chain constant region, and at least a portion of at least one of the heavy chain constant region and the light chain constant region is derived from at least one of a murine antibody, a human antibody, a primate antibody, or a mutant thereof.

According to an embodiment of the present application, both the light chain constant region and the heavy chain constant region are derived from a human IgG antibody or a mutant thereof.

According to an embodiment of the present application, the heavy chain constant region is a human IgG mutant.

According to an embodiment of the present application, the heavy chain constant region has at least one of the following site mutations compared to a heavy chain constant region of a human wild-type IgG1 antibody: L234A, L235A, G237A, and K447, wherein for K447, this site is preferably deleted.

Thus, the L234A, L235A, and G237A mutations can further eliminate the Fc effect of the antibody, and the K447 mutation can avoid antibody heterogeneity. Alternatively, further options include, but are not limited to, site substitutions such as K214/R214, E356, M358/D356, L358, and others. The K447 mutation is preferably a deletion of this site.

Exemplarily, according to an embodiment of the present application, the heavy chain constant region has the amino acid sequence as set forth in SEQ ID NO: 148, i.e., hIgG1 (LALAGA, -K)

ASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPE
AAGAPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSFFLYSK
LTVDKSRWQQGNVFSCSVMHEALHNHYTQKSLSLSPG (SEQ ID NO: 148).

Exemplarily, according to an embodiment of the present application, the heavy chain constant region has the amino acid sequence as set forth in SEQ ID NO: 228, i.e., hIgG1' (LALAGA, -K)

ASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKRVEPKSCDKTHTCPPCPAPE
AAGAPSVFLFPPKPKDTLMISRTPEVTCVVVDVSHEDPEVKFNWYVDGVEVHNAKTKP

REEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPG (SEQ ID NO: 228).

According to an embodiment of the present application, the light chain constant region has the amino acid sequence as set forth in SEQ ID NO: 149.


 (SEQ ID
NO: 149).

According to an embodiment of the present application, the antibody has a heavy chain as set forth in the amino acid sequence of any one of SEQ ID NOs: 150 to 164, 166 to 182, and 229, or a light chain as set forth in the amino acid sequence of any one of SEQ ID NOs: 183 to 197 and 199 to 217.
G2-CH1 H: 
QVTLKESGPGILQPSQTLSLTCSFSGFSLSTYGIGVGWIRQPSGKGLEWLAHIWWNN
NKYFNTALKSRLTISKDTSNNQVFLKIASVDTADSATYYCARMGYDIMDYWGQGTSVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEA
AGAPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPG (SEQ ID NO: 150);
G2-CH2 H: 
QVTLKESGPGILQPSQTLSLTCSFSGFSLTISGMGVSWIRQPSGKGLEWLAHIYWDD
DKRYNPSLKSRLTISKDTSSNQVFLEITSVDTADTATYYCVRAGWDFFDYWGQGTTLTV
SSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPEAA
GAPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYSKLTV
DKSRWQQGNVFSCSVMHEALHNHYTQKSLSLSPG (SEQ ID NO: 151);
G2-CH3 H: 
QVTLKESGPGILQPSQTLSLTCSFSGFSLSTSGMGVSWIRQPSGKGLEWLAHIYWDD
DKRYNPSLKSRLTISKDTSSNRVFLKITSVDTADTATYYCARTGWDFFDYWGQGTTLTV
SSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPEAA
GAPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYSKLTV
DKSRWQQGNVFSCSVMHEALHNHYTQKSLSLSPG (SEQ ID NO: 152);
G2-CH5 H: 
EVLLQQSGPELVKPGASVKIACKASGYTLTDYNLNWVKQSHGKSLEWIGDLNPYN
GGIIYNQKFKGKATLTVDKSSSTAYMELRSLTSEDIAVYYCAREGRGGAWFDSWGQGT
LVTVSAASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPA
PEAAGAPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPG (SEQ ID NO: 153);
G2-CH6 H: 
EVQLRQSGPELVKPGTSMKISCKASAYSFTDYNMNWVKQSHGKNLEWIGLINPYN
GGTRYNQNFKGKATITVDRSSTTAYMELLSLTSEDSAIYYCAREVRLGNALDYWGQGT
SVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPA
PEAAGAPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPG (SEQ ID NO: 154);
G2-CH7 H: 
EVQLQQSGPELVKPGASLKISCKASGYSFTGYNMNWVKQSHGKNLEWIGLINPSN
GGSSYSQKFKGKATLTVDKSSSTAYMELLSLTSKDSAVYYCAREVRLGNALDYWGQG
 
TSVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPCP
APEAAGAPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPG (SEQ ID NO: 155);
G2-CH8 H: 
QVTLKESGPGILQPSQTLSLTCSFSGFSLSTYGIGVGWIRQPSGKGLEWLAHIWWND
NKYYNTALKSRLTISKDTSNNQVFLKIASVDTADTATYYCARLAYDYVDSWGQGTTLT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEA
AGAPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPG (SEQ ID NO: 156);
G2-CH9 H: 
EVQLQQSGPELVKPGASMKISCKASGYSFTGYNMNWVKQSQGKNLEWIGLINPYN
GGTIYNQKFKGKATLTVDKSSTTAYMELLSLTSEDSAVYYCAREVRLGNAMDYWGQG
TSVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPCP
APEAAGAPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPG (SEQ ID NO: 157);
G2-CH10 H: 
QGTLKESGPGILQPSQTLSLTCSFSGFSLSISGMGVSWIRQPSGKGLEWLAHIYWDD
DKRYKPSLKSRLTISKDTSRNQVFLKITSVDTADTATYYCARSAYGMTDYWGQGTTLT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEA
AGAPSVFLFPPKPKDTLMISRTPEVTCVVVDVSHEDPEVKFNWYVDGVEVHNAKTKPR
 
EEQYNSTYRVVSVLTVLHQDWLNGKEYKCKVSNKALPAPIEKTISKAKGQPREPQVYT
LPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPG (SEQ ID NO: 158);
G2-CH12 H: 
QVQLQQSGAELVKPGASVKLSCKASGYTFTNYDINWVRQRPEQGLEWIGWIFPGD
GSTKYNEKFKDKATLTTDTSSSTAYMQLIRLTSEDSAVYFCARILYWGQGTTLTVSSAST
KGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPEAAGAPS
VFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRE
EMTKNQVSLTCLVKGFYPSDIAVEWESNGQPENNYKTTPPVLDSDGSFFLYSKLTVDKS
RWQQGNVFSCSVMHEALHNHYTQKSLSLSPG (SEQ ID NO: 159);
G2-CH13 H: 
EVLLQQSGPELVKPGASVKITCKASGYTFTDYNMNWVKQSHGKSLEWIGDINPYN
GGIIYNQKFRGKATLTIDKSSSTAYMELRSLTSEDTAVYYCAREVRRGSFFDYWGQGTT
LTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCPAP
EAAGAPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPG (SEQ ID NO: 160);
G2-CH14 H: 
QVTLKESGPGILQPSQTLSLTCSFSGFSLSTSGMGVSWIRQPSGKGLEWLALIYWDD
DKRYNPSLKSRLTISKDASSNQVFLKITSVDTADTATYYCIQSPWDWFAYWGQGTLVTV
SAASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEA
AGAPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPG (SEQ ID NO: 161);
G2-CH16 H: 
QVTLKESGPGILQPSQTLSLTCSFSGFSLSTSGMGVSWIRQPSGKGLEWLAHIYWDD
DKRYNPSLKSRLTISKDTSRNQVFLKITSVDTADTATYYCARRAYGAMDYWGHGTSVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEA
AGAPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPG (SEQ ID NO: 162);
G2-CH17 H: 
QVTLKESGPGILQPSQTLSLTCSFSGFSLNTSGMGVSWIRQPSGKGLEWLAHIYWDD
DKRYNPSLKSRLTISKDTSRNQVFLKITGVDTADTAAYYCARTGWDFFDIWGQGTTLTV
SSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPEAA
GAPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYSKLTV
DKSRWQQGNVFSCSVMHEALHNHYTQKSLSLSPG (SEQ ID NO: 163);
G2-CH23 H: 
QVQLQQSGAELMKPGASVKISCKATGYTFSSYWIEWVKQRPGHGLEWIGEILPGSG
ITNYNEKFKGKATFTADTSSNTAYMQLSSLTSEDSAVYYCARETARARGFTYWGQGTL
VTVSAASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPA
PEAAGAPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPG (SEQ ID NO: 164);
G2-CH5-P1 H: 
EVLLQQSGPELVKPGASVKIACKASGYTLTDYNLNWVKQSHGKSLEWIGDLNPYN
AGIIYNQKFKGKATLTVDKSSSTAYMELRSLTSEDIAVYYCAREGRGGAWFDSWGQGT
 
LVTVSAASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPA
PEAAGAPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPG (SEQ ID NO: 166);
G2-CH5-P2 H: 
EVLLQQSGPELVKPGASVKIACKASGYTLTDYNLNWVKQSHGKSLEWIGDLNPYN
QGIIYNQKFKGKATLTVDKSSSTAYMELRSLTSEDIAVYYCAREGRGGAWFDSWGQGT
LVTVSAASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPA
PEAAGAPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPG (SEQ ID NO: 167);
G2-CH5-P3 H: 
EVLLQQSGPELVKPGASVKIACKASGYTLTDYNLNWVKQSHGKSLEWIGDLNPYE
GGIIYNQKFKGKATLTVDKSSSTAYMELRSLTSEDIAVYYCAREGRGGAWFDSWGQGT
LVTVSAASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPA
PEAAGAPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPG (SEQ ID NO: 168);
G2-CH8 H (same as G2-CH8-P1 H, G2-CH8-P2 H and G2-CH8-P3 H): 
QVTLKESGPGILQPSQTLSLTCSFSGFSLSTYGIGVGWIRQPSGKGLEWLAHIWWND
NKYYNTALKSRLTISKDTSNNQVFLKIASVDTADTATYYCARLAYDYVDSWGQGTTLT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEA
AGAPSVFLFPPKPKDTLMISRTPEVTCVVVDVSHEDPEVKFNWYVDGVEVHNAKTKPR
 
EEQYNSTYRVVSVLTVLHQDWLNGKEYKCKVSNKALPAPIEKTISKAKGQPREPQVYT
LPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPG (SEQ ID NO: 169);
G2-CH2 H (same as G2-CH2-P1 H, G2-CH2-P2 H and G2-CH2-P3 H): 
QVTLKESGPGILQPSQTLSLTCSFSGFSLTISGMGVSWIRQPSGKGLEWLAHIYWDD
DKRYNPSLKSRLTISKDTSSNQVFLEITSVDTADTATYYCVRAGWDFFDYWGQGTTLTV
SSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPEAA
GAPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYSKLTV
DKSRWQQGNVFSCSVMHEALHNHYTQKSLSLSPG (SEQ ID NO: 170);
G2-H1-H_2(4-39): 
QVQLQESGPGLVKPSETLSLTCTFSGFSLSTYGIGVGWIRQPPGKGLEWIAHIWWNN
NKYFNTALKSRLTISKDTSKNQVSLKLSSVTAADTAVYYCARMGYDIMDYWGQGTLV
TVSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPE
AAGAPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSFFLYSK
LTVDKSRWQQGNVFSCSVMHEALHNHYTQKSLSLSPG (SEQ ID NO: 171);
G2-H1-H_3(4-39): 
QVTLQESGPGLVKPSETLSLTCSFSGFSLSTYGIGVGWIRQPPGKGLEWLAHIWWN
NNKYFNTALKSRLTISKDTSKNQVSLKLSSVTAADTATYYCARMGYDIMDYWGQGTSV
TVSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPE
AAGAPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSFFLYSK
LTVDKSRWQQGNVFSCSVMHEALHNHYTQKSLSLSPG (SEQ ID NO: 172);
G2-H1-H_2(3-23): 
EVQLLESGGGLVQPGGSLRLSCAFSGFSLSTYGIGVGWIRQAPGKGLEWVAHIWW
NNNKYFNTALKSRLTISKDTSKNTVYLQMNSLRAEDTAVYYCAKMGYDIMDYWGQGT
TVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPA
PEAAGAPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPG (SEQ ID NO: 173);
G2-H1-H_3(3-23): 
QVTLLESGGGLVQPGGSLRLSCSFSGFSLSTYGIGVGWIRQAPGKGLEWLAHIWWN
NNKYFNTALKSRLTISKDTSKNTVYLQMNSVRAEDTAVYYCARMGYDIMDYWGQGTT
VTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCPAP
EAAGAPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPG (SEQ ID NO: 174);
G2-H5-H_1: 
EVQLVQSGAEVKKPGASVKVSCKASGYTFTDYNLNWVRQATGQGLEWMGDLNP
YNQGIIYNQKFKGRVTMTVDTSISTAYMELSSLRSEDTAVYYCAREGRGGAWFDSWGQ
GTLVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSCDKTHTCPPC
PAPEAAGAPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLDSDGSFF
LYSKLTVDKSRWQQGNVFSCSVMHEALHNHYTQKSLSLSPG (SEQ ID NO: 175);
G2-H5-H_2: 
EVQLVQSGAEVKKPGASVKVSCKASGYTFTDYNLNWVRQATGQGLEWMGDLNP
YNQGIIYNQKFKGRVTLTVDKSISTAYMELSSLRSEDTAVYYCAREGRGGAWFDSWGQ
 
GTLVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSCDKTHTCPPC
PAPEAAGAPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLDSDGSFF
LYSKLTVDKSRWQQGNVFSCSVMHEALHNHYTQKSLSLSPG (SEQ ID NO: 176);
G2-H5-H_3: 
EVQLVQSGAEVKKPGASVKVSCKASGYTFTDYNLNWVRQATGQGLEWIGDLNPY
NQGIIYNQKFKGRATLTVDKSISTAYMELSSLRSEDTAVYYCAREGRGGAWFDSWGQG
TLVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPCP
APEAAGAPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPG (SEQ ID NO: 177);
G2-H5-H_4: 
EVQLVQSGAEVKKPGASVKVSCKASGYTLTDYNLNWVRQATGQGLEWIGDLNPY
NQGIIYNQKFKGRATLTVNKSISTAYMELSSLRSEDTAVYYCAREGRGGAWFDSWGQG
TLVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPCP
APEAAGAPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPG (SEQ ID NO: 178);
G2-H8-H_1: 
QVTLKESGPALVKPTQTLTLTCTFSGFSLSTYGIGVGWIRQPPGKALEWLAHIWWN
DNKYYNTALKSRLTISKDTSKNQVVLTMTNMDPVDTATYYCARLAYDYVDSWGQGTT
LTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCPAP
EAAGAPSVFLFPPKPKDTLMISRTPEVTCVVVDVSHEDPEVKFNWYVDGVEVHNAKTK
 
PREEEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPG (SEQ ID NO: 179);
G2-H8-H_2: 
QVTLKESGPALVKPTQTLTLTCTFSGFSLSTYGIGVGWIRQPPGKGLEWLAHIWWN
DNKYYNTALKSRLTISKDTSKNQVVLTMTNMDPVDTATYYCARLAYDYVDSWGQGTT
LTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCPAP
EAAGAPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPG (SEQ ID NO: 180);
G2-H8-H1(gsm)': 
QITLKESGPTLVKPTQTLTLTCTFSGFSLSTYGIGVGWIRQPPGKALEWLAHIWWND
NKYYNTALKSRLTITKDTSKNQVVLTMTNMDPVDTATYYCARLAYDYVDSWGQGTLV
TVSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKRVEPKSCDKTHTCPPCPAPE
AAGAPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPG (SEQ ID NO: 229);
G2-H8-H1(gsm): 
QITLKESGPTLVKPTQTLTLTCTFSGFSLSTYGIGVGWIRQPPGKALEWLAHIWWND
NKYYNTALKSRLTITKDTSKNQVVLTMTNMDPVDTATYYCARLAYDYVDSWGQGTLV
TVSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPE
AAGAPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSFFLYSK
LTVDKSRWQQGNVFSCSVMHEALHNHYTQKSLSLSPG (SEQ ID NO: 181);
G2-H8-H2(gsm): 
QITLKESGPALVKPTQTLTLTCTFSGFSLSTYGIGVGWIRQPPGKALEWLAHIWWND
NKYYNTALKSRLTITKDTSKNQVFLTMTNMDPVDTATYYCARLAYDYVDSWGQGTLV
TVSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPE
AAGAPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSFFLYSK
LTVDKSRWQQGNVFSCSVMHEALHNHYTQKSLSLSPG (SEQ ID NO: 182);
G2-CH1 L: 
DIVMTQSHKFMSTSVGDRVSITCKASQDVNTAVAWYQQKPGQSPKFLIYSSSYRYT
GVPDRFTGSGSGTDFTFTISSVQAEDLAVYYCQQHYSTPWTFGGGTKLEIKRTVAAPSVF
IFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC (SEQ ID NO: 183);
G2-CH2 L: 
DIVMTQSQKFMSTSVGDRVSVTCKASQNVGTNVAWYQQKPGQSPKGLIYSASYRY
SGVPDRFTGSGSGTDFTLTISNVQSEDLAEYFCQQYNSYPWTFGGGTKLEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC (SEQ ID NO: 184);
G2-CH3 L: 
DIVMTQSQKFMSTSVGDRVSVTCKASQNVGTNVAWYQQKPGQSPKGLIYSASYRY
SGVPDRFTGSGSGTDFTLTINNVQSEDLAEYFCQQYNSYPYTFGGGTKLEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC (SEQ ID NO: 185);
G2-CH5 L: 
DIQMTQSSSSFSVSLGDRVTITCKASEDIYHRLAWYQQKPGNAPRLLISGATSLETGI
PSRFSGSGSGKDYALSITSLQTEDVGTYYCQQYWSPPFTFGSGTKLEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO: 186);
G2-CH6 L: 
DIQMTQSPASLSVSVGETVTITCRPSESIYSNLAWYQQKQGKSPQLLVYAATNLAD
GVPSRFSGSGSGTQYSLKINSLQSEDFGTYYCQHFWGIPWTFGGGTKLEIKRTVAAPSVF
IFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC (SEQ ID NO: 187);
G2-CH7 L: 
DIQMTQSPASLSVSVGETVTITCRASENIYSNLAWYQQKQGKSPQLLVYAATNLGD
GAPSRFSGSGSGTQYSLKINSLQSEDFGSYYCQHFWGTPWTFGGGTKLEIKRTVAAPSVF
IFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC (SEQ ID NO: 188);
G2-CH8 L: 
DIVMTQAAFSNPVTLGTSASISCRSSKSLLHSNGITYLYWYLQKPGQSPQLLIYQMS
NLVSGVPDRFSSSGSGTDFTLRISRVEAEDVGVYYCAQNLELPPTFGAGTKLELKRTVA
APSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC (SEQ ID NO: 189);
G2-CH9 L: 
DIQMTQSPASLSVSVGETVTITCRASENIYSNLAWYQQKQGKSPQLLVSAATNLAD
GVPSRFSGSGSGTQYSLKIISLQSVDFGSYYCQHFWGTPWTFGGGTKLEIKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC (SEQ ID NO: 190);
G2-CH10 L: 
DIQMTQTTSSLSASLGDRVTISCRASQDISNYLNWYQQKPDGTVKLLIHSASNLHSG
VPSRFSGSGSGTDYSLTISNLEQEDIATYFCQQYNMFPWTFGGGTKLEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 191);
G2-CH12 L: 
DNKMTQSPSSMYASLGERVTITCKASQDINSYLSWFQQKPGKSPKTLIYRANKLVD
GVPSRFSGSGSGQDYSLTISSLEYEDMGIYHCLQYDEFPYTFGGGTKLEIKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC (SEQ ID NO: 192);
G2-CH13 L: 
DIQMTQSPASLSVSVGETVTITCRASENIYSNLAWYQQKQGKSPQLLVYAATDLAD
GVPSRFSGSGSGTQYSLKINSLQSEDFGNYHCQLFWGTPTFGGGTKLEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 193);
G2-CH14 L: 
EIVLTQSPALMAASPGEKVTITCSVSSSISSSNLHWYQQKSETSPKPWIYGTSNLASG
VPVRFSGSGSGTSYSLTISSMEAEDAATYYCQQWSSYPLTFGAGTKLELKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC (SEQ ID NO: 194);
G2-CH16 L: 
DIVMTQSQKFMSTSVGDRVSVTCKASQNVGTNIAWFQQKPGQSPEALIYSASYRYS
GVPDRFTGSGSGTDFTLTISNVQSEDLAEYFCQQYNTYPHTFGGGTKLEIRRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC (SEQ ID NO: 195);
G2-CH17 L: 
DIVMTQSQKFMSTSVGDRVSVTCKASQNVGSNVAWYQQKPGQSPKALIYSASYRY
SGVPDRFTGSGSGTDFTLTITNVQSEDLAEYFCQQYKSYPLTFGAGTKLELKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC (SEQ ID NO: 196);
G2-CH23 L: 
ETTVTQSPASLSMAIGEKVTIRCITSIDIDDDINWYQQKPGEPPNLLISEGNTLRPGVP
SRFSSSGYGTDFVFTIENMLSEDVADYYCLQSDNLPLTFGGGTKLEIKRTVAAPSVFIFPP
SDEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO: 197);
G2-CH5 L (same as G2-CH5-P1 L, G2-CH5-P2 L and G2-CH5-P3 L): 
DIQMTQSSSSFSVSLGDRVTITCKASEDIYHRLAWYQQKPGNAPRLLISGATSLETGI
PSRFSGSGSGKDYALSITSLQTEDVGTYYCQQYWSPPFTFGSGTKLEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO: 199);
G2-CH8-P1 L: 
DIVMTQAAFSNPVTLGTSASISCRSSKSLLHSNAITYLYWYLQKPGQSPQLLIYQMS
NLVSGVPDRFSSSGSGTDFTLRISRVEAEDVGVYYCAQNLELPPTFGAGTKLELKRTVA
APSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC (SEQ ID NO: 200);
G2-CH8-P2 L: 
DIVMTQAAFSNPVTLGTSASISCRSSKSLLHSNQITYLYWYLQKPGQSPQLLIYQMS
NLVSGVPDRFSSSGSGTDFTLRISRVEAEDVGVYYCAQNLELPPTFGAGTKLELKRTVA
APSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC (SEQ ID NO: 201);
G2-CH8-P3 L: 
DIVMTQAAFSNPVTLGTSASISCRSSKSLLHSEGITYLYWYLQKPGQSPQLLIYQMS
NLVSGVPDRFSSSGSGTDFTLRISRVEAEDVGVYYCAQNLELPPTFGAGTKLELKRTVA
APSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC (SEQ ID NO: 202);
G2-CH2-P1 L: 
DIVMTQSQKFMSTSVGDRVSVTCKASQNVGTNVAWYQQKPGQSPKGLIYSASYRY
SGVPDRFTGSGSGTDFTLTISNVQSEDLAEYFCQQYNAYPWTFGGGTKLEIKRTVAAPS
VFIFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC (SEQ ID NO: 203);
G2-CH2-P2 L: 
DIVMTQSQKFMSTSVGDRVSVTCKASQNVGTNVAWYQQKPGQSPKGLIYSASYRY
SGVPDRFTGSGSGTDFTLTISNVQSEDLAEYFCQQYESYPWTFGGGTKLEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC (SEQ ID NO: 204);
G2-CH2-P3 L: 
DIVMTQSQKFMSTSVGDRVSVTCKASQNVGTNVAWYQQKPGQSPKGLIYSASYRY
SGVPDRFTGSGSGTDFTLTISNVQSEDLAEYFCQQYNQYPWTFGGGTKLEIKRTVAAPS
VFIFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC (SEQ ID NO: 205);
G2-H1-L_2 (1-39): 
DIQMTQSPSSLSTSVGDRVTITCKASQDVNTAVAWYQQKPGKAPKFLIYSSSYRYT
GVPDRFSGSGSGTDFTFTISSLQPEDFAVYYCQQHYSTPWTFGQGTKVEIKRTVAAPSVF
IFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC (SEQ ID NO: 206);
G2-H1-L_3 (1-39): 
DIVMTQSPSSLSTSVGDRVTITCKASQDVNTAVAWYQQKPGKSPKFLIYSSSYRYT
GVPDRFSGSGSGTDFTFTISSVQPEDFAVYYCQQHYSTPWTFGGGTKVEIKRTVAAPSVF
IFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC (SEQ ID NO: 207);
G2-H1-L_2 (3-15): 
DIVMTQSPATLSTSPGERATLSCKASQDVNTAVAWYQQKPGQAPRFLIYSSSYRYT
GVPARFSGSGSGTEFTFTISSLQSEDFAVYYCQQHYSTPWTFGGGTKVEIKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC (SEQ ID NO: 208);
G2-H1-L_3 (3-15): 
DIVMTQSPATLSTSPGERVTISCKASQDVNTAVAWYQQKPGQSPRFLIYSSSYRYTG
VPDRFSGSGSGTDFTFTISSVQSEDFAVYYCQQHYSTPWTFGGGTKVEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 209);
G2-H5-L_1: 
DIQMTQSPSSLSASVGDRVTITCKASEDIYHRLAWYQQKPGKAPKLLIYGATSLETG
VPSRFSGSGSGTDFTFTISSLQPEDIATYYCQQYWSPPFTFGGGTKVEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO: 210);
G2-H5-L_2: 
DIQMTQSPSSLSASVGDRVTITCKASEDIYHRLAWYQQKPGKAPKLLIYGATSLETG
VPSRFSGSGSGTDYTFTISSLQPEDIATYYCQQYWSPPFTFGGGTKVEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO: 211);
G2-H5-L_3: 
DIQMTQSPSSLSASVGDRVTITCKASEDIYHRLAWYQQKPGKAPKLLISGATSLETG
VPSRFSGSGSGTDYTFTISSLQPEDIATYYCQQYWSPPFTFGGGTKVEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO: 212);
G2-H5-L_4: 
DIQMTQSPSSLSASVGDRVTITCKASEDIYHRLAWYQQKPGKAPKLLISGATSLETG
IPSRFSGSGSGTDYTFTISSLQPEDIATYYCQQYWSPPFTFGGGTKVEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO: 213);
G2-H8-L_1: 
DIVMTQTPLSLSVTPGQPASISCRSSKSLLHSEGITYLYWYLQKPGQSPQLLIYQMSN
LVSGVPDRFSGSGSGTDFTLKISRVEAEDVGVYYCAQNLELPPTFGQGTKLEIKRTVAAP
SVFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC (SEQ ID NO: 214);
G2-H8-L_2: 
DIVMTQTPLSLSVTPGQPASISCRSSKSLLHSEGITYLYWYLQKPGQSPQLLIYQMSN
LVSGVPDRFSSSGSGTDFTLKISRVEAEDVGVYYCAQNLELPPTFGQGTKLEIKRTVAAP
SVFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC (SEQ ID NO: 215);
G2-H8-L_1(gsm): 
DIVMTQTPLSLSVTPGQPASISCKSSKSLLHSEGITYLYWYLQKPGQSPQLLIYQMSN
LVSGVPDRFSGSGSGTDFTLKISRVEAEDVGVYYCAQNLELPPTFGQGTKLEIKRTVAAP
SVFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC (SEQ ID NO: 216);
G2-H8-L_2(gsm):

DIVMTQSPLSLSVTPGEPASISCRSSKSLLHSEGITYLYWYLQKPGQSPQLLIYQMSN
LVSGVPDRFSGSGSGTDFTLKISRVEAEDVGVYYCAQNLELPPTFGQGTKLEIKRTVAAP
SVFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC (SEQ ID NO: 217).

According to an embodiment of the present application, the antibody comprises a heavy chain as set forth in the amino acid sequence of SEQ ID NO: 150 and a light chain as set forth in the amino acid sequence of SEQ ID NO: 183.

According to an embodiment of the present application, the antibody comprises a heavy chain as set forth in the amino acid sequence of SEQ ID NO: 151 and a light chain as set forth in the amino acid sequence of SEQ ID NO: 184.

According to an embodiment of the present application, the antibody comprises a heavy chain as set forth in the amino acid sequence of SEQ ID NO: 152 and a light chain as set forth in the amino acid sequence of SEQ ID NO: 185.

According to an embodiment of the present application, the antibody comprises a heavy chain as set forth in the amino acid sequence of SEQ ID NO: 153 and a light chain as set forth in the amino acid sequence of SEQ ID NO: 186.

According to an embodiment of the present application, the antibody comprises a heavy chain as set forth in the amino acid sequence of SEQ ID NO: 154 and a light chain as set forth in the amino acid sequence of SEQ ID NO: 187.

According to an embodiment of the present application, the antibody comprises a heavy chain as set forth in the amino acid sequence of SEQ ID NO: 155 and a light chain as set forth in the amino acid sequence of SEQ ID NO: 188.

According to an embodiment of the present application, the antibody comprises a heavy chain as set forth in the amino acid sequence of SEQ ID NO: 156 and a light chain as set forth in the amino acid sequence of SEQ ID NO: 189.

According to an embodiment of the present application, the antibody comprises a heavy chain as set forth in the amino acid sequence of SEQ ID NO: 157 and a light chain as set forth in the amino acid sequence of SEQ ID NO: 190.

According to an embodiment of the present application, the antibody comprises a heavy chain as set forth in the amino acid sequence of SEQ ID NO: 158 and a light chain as set forth in the amino acid sequence of SEQ ID NO: 191.

According to an embodiment of the present application, the antibody comprises a heavy chain as set forth in the amino acid sequence of SEQ ID NO: 159 and a light chain as set forth in the amino acid sequence of SEQ ID NO: 192.

According to an embodiment of the present application, the antibody comprises a heavy chain as set forth in the amino acid sequence of SEQ ID NO: 160 and a light chain as set forth in the amino acid sequence of SEQ ID NO: 193.

According to an embodiment of the present application, the antibody comprises a heavy chain as set forth in the amino acid sequence of SEQ ID NO: 161 and a light chain as set forth in the amino acid sequence of SEQ ID NO: 194.

According to an embodiment of the present application, the antibody comprises a heavy chain as set forth in the amino acid sequence of SEQ ID NO: 162 and a light chain as set forth in the amino acid sequence of SEQ ID NO: 195.

According to an embodiment of the present application, the antibody comprises a heavy chain as set forth in the amino acid sequence of SEQ ID NO: 163 and a light chain as set forth in the amino acid sequence of SEQ ID NO: 196.

According to an embodiment of the present application, the antibody comprises a heavy chain as set forth in the amino acid sequence of SEQ ID NO: 166 and a light chain as set forth in the amino acid sequence of SEQ ID NO: 199.

According to an embodiment of the present application, the antibody comprises a heavy chain as set forth in the amino acid sequence of SEQ ID NO: 167 and a light chain as set forth in the amino acid sequence of SEQ ID NO: 199.

According to an embodiment of the present application, the antibody comprises a heavy chain as set forth in the amino acid sequence of SEQ ID NO: 168 and a light chain as set forth in the amino acid sequence of SEQ ID NO: 199.

According to an embodiment of the present application, the antibody comprises a heavy chain as set forth in the amino acid sequence of SEQ ID NO: 169 and a light chain as set forth in the amino acid sequence of SEQ ID NO: 200.

According to an embodiment of the present application, the antibody comprises a heavy chain as set forth in the amino acid sequence of SEQ ID NO: 169 and a light chain as set forth in the amino acid sequence of SEQ ID NO: 201.

According to an embodiment of the present application, the antibody comprises a heavy chain as set forth in the amino acid sequence of SEQ ID NO: 169 and a light chain as set forth in the amino acid sequence of SEQ ID NO: 202.

According to an embodiment of the present application, the antibody comprises a heavy chain as set forth in the amino acid sequence of SEQ ID NO: 170 and a light chain as set forth in the amino acid sequence of SEQ ID NO: 203.

According to an embodiment of the present application, the antibody comprises a heavy chain as set forth in the amino acid sequence of SEQ ID NO: 170 and a light chain as set forth in the amino acid sequence of SEQ ID NO: 204.

According to an embodiment of the present application, the antibody comprises a heavy chain as set forth in the amino acid sequence of SEQ ID NO: 170 and a light chain as set forth in the amino acid sequence of SEQ ID NO: 205.

According to an embodiment of the present application, the antibody comprises a heavy chain as set forth in the amino acid sequence of SEQ ID NO: 171 and a light chain as set forth in the amino acid sequence of SEQ ID NO: 206.

According to an embodiment of the present application, the antibody comprises a heavy chain as set forth in the amino acid sequence of SEQ ID NO: 171 and a light chain as set forth in the amino acid sequence of SEQ ID NO: 207.

According to an embodiment of the present application, the antibody comprises a heavy chain as set forth in the amino acid sequence of SEQ ID NO: 171 and a light chain as set forth in the amino acid sequence of SEQ ID NO: 208.

According to an embodiment of the present application, the antibody comprises a heavy chain as set forth in the amino acid sequence of SEQ ID NO: 171 and a light chain as set forth in the amino acid sequence of SEQ ID NO: 209.

According to an embodiment of the present application, the antibody comprises a heavy chain as set forth in the amino acid sequence of SEQ ID NO: 172 and a light chain as set forth in the amino acid sequence of SEQ ID NO: 206.

According to an embodiment of the present application, the antibody comprises a heavy chain as set forth in the amino acid sequence of SEQ ID NO: 172 and a light chain as set forth in the amino acid sequence of SEQ ID NO: 207.

According to an embodiment of the present application, the antibody comprises a heavy chain as set forth in the amino acid sequence of SEQ ID NO: 172 and a light chain as set forth in the amino acid sequence of SEQ ID NO: 208.

According to an embodiment of the present application, the antibody comprises a heavy chain as set forth in the amino acid sequence of SEQ ID NO: 172 and a light chain as set forth in the amino acid sequence of SEQ ID NO: 209.

According to an embodiment of the present application, the antibody comprises a heavy chain as set forth in the amino acid sequence of SEQ ID NO: 173 and a light chain as set forth in the amino acid sequence of SEQ ID NO: 206.

According to an embodiment of the present application, the antibody comprises a heavy chain as set forth in the amino acid sequence of SEQ ID NO: 173 and a light chain as set forth in the amino acid sequence of SEQ ID NO: 207.

According to an embodiment of the present application, the antibody comprises a heavy chain as set forth in the amino acid sequence of SEQ ID NO: 173 and a light chain as set forth in the amino acid sequence of SEQ ID NO: 208.

According to an embodiment of the present application, the antibody comprises a heavy chain as set forth in the amino acid sequence of SEQ ID NO: 173 and a light chain as set forth in the amino acid sequence of SEQ ID NO: 209.

According to an embodiment of the present application, the antibody comprises a heavy chain as set forth in the amino acid sequence of SEQ ID NO: 174 and a light chain as set forth in the amino acid sequence of SEQ ID NO: 206.

According to an embodiment of the present application, the antibody comprises a heavy chain as set forth in the amino acid sequence of SEQ ID NO: 174 and a light chain as set forth in the amino acid sequence of SEQ ID NO: 207.

According to an embodiment of the present application, the antibody comprises a heavy chain as set forth in the amino acid sequence of SEQ ID NO: 174 and a light chain as set forth in the amino acid sequence of SEQ ID NO: 208.

According to an embodiment of the present application, the antibody comprises a heavy chain as set forth in the amino acid sequence of SEQ ID NO: 174 and a light chain as set forth in the amino acid sequence of SEQ ID NO: 209.

According to an embodiment of the present application, the antibody comprises a heavy chain as set forth in the amino acid sequence of SEQ ID NO: 175 and a light chain as set forth in the amino acid sequence of SEQ ID NO: 210.

According to an embodiment of the present application, the antibody comprises a heavy chain as set forth in the amino acid sequence of SEQ ID NO: 175 and a light chain as set forth in the amino acid sequence of SEQ ID NO: 211.

According to an embodiment of the present application, the antibody comprises a heavy chain as set forth in the amino acid sequence of SEQ ID NO: 175 and a light chain as set forth in the amino acid sequence of SEQ ID NO: 212.

According to an embodiment of the present application, the antibody comprises a heavy chain as set forth in the amino acid sequence of SEQ ID NO: 175 and a light chain as set forth in the amino acid sequence of SEQ ID NO: 213.

According to an embodiment of the present application, the antibody comprises a heavy chain as set forth in the amino acid sequence of SEQ ID NO: 176 and a light chain as set forth in the amino acid sequence of SEQ ID NO: 210.

According to an embodiment of the present application, the antibody comprises a heavy chain as set forth in the amino acid sequence of SEQ ID NO: 176 and a light chain as set forth in the amino acid sequence of SEQ ID NO: 211.

According to an embodiment of the present application, the antibody comprises a heavy chain as set forth in the amino acid sequence of SEQ ID NO: 176 and a light chain as set forth in the amino acid sequence of SEQ ID NO: 212.

According to an embodiment of the present application, the antibody comprises a heavy chain as set forth in the amino acid sequence of SEQ ID NO: 176 and a light chain as set forth in the amino acid sequence of SEQ ID NO: 213.

According to an embodiment of the present application, the antibody comprises a heavy chain as set forth in the amino acid sequence of SEQ ID NO: 177 and a light chain as set forth in the amino acid sequence of SEQ ID NO: 210.

According to an embodiment of the present application, the antibody comprises a heavy chain as set forth in the amino acid sequence of SEQ ID NO: 177 and a light chain as set forth in the amino acid sequence of SEQ ID NO: 211.

According to an embodiment of the present application, the antibody comprises a heavy chain as set forth in the amino acid sequence of SEQ ID NO: 177 and a light chain as set forth in the amino acid sequence of SEQ ID NO: 212.

According to an embodiment of the present application, the antibody comprises a heavy chain as set forth in the amino acid sequence of SEQ ID NO: 177 and a light chain as set forth in the amino acid sequence of SEQ ID NO: 213.

According to an embodiment of the present application, the antibody comprises a heavy chain as set forth in the amino acid sequence of SEQ ID NO: 178 and a light chain as set forth in the amino acid sequence of SEQ ID NO: 210.

According to an embodiment of the present application, the antibody comprises a heavy chain as set forth in the amino acid sequence of SEQ ID NO: 178 and a light chain as set forth in the amino acid sequence of SEQ ID NO: 211.

According to an embodiment of the present application, the antibody comprises a heavy chain as set forth in the amino acid sequence of SEQ ID NO: 178 and a light chain as set forth in the amino acid sequence of SEQ ID NO: 212.

According to an embodiment of the present application, the antibody comprises a heavy chain as set forth in the amino acid sequence of SEQ ID NO: 178 and a light chain as set forth in the amino acid sequence of SEQ ID NO: 213.

According to an embodiment of the present application, the antibody comprises a heavy chain as set forth in the amino acid sequence of SEQ ID NO: 179 and a light chain as set forth in the amino acid sequence of SEQ ID NO: 214.

According to an embodiment of the present application, the antibody comprises a heavy chain as set forth in the amino acid sequence of SEQ ID NO: 179 and a light chain as set forth in the amino acid sequence of SEQ ID NO: 215.

According to an embodiment of the present application, the antibody comprises a heavy chain as set forth in the amino acid sequence of SEQ ID NO: 180 and a light chain as set forth in the amino acid sequence of SEQ ID NO: 214.

According to an embodiment of the present application, the antibody comprises a heavy chain as set forth in the amino acid sequence of SEQ ID NO: 180 and a light chain as set forth in the amino acid sequence of SEQ ID NO: 215.

According to an embodiment of the present application, the antibody comprises a heavy chain as set forth in the amino acid sequence of SEQ ID NO: 181 and a light chain as set forth in the amino acid sequence of SEQ ID NO: 216.

According to an embodiment of the present application, the antibody comprises a heavy chain as set forth in the amino acid sequence of SEQ ID NO: 229 and a light chain as set forth in the amino acid sequence of SEQ ID NO: 216.

According to an embodiment of the present application, the antibody comprises a heavy chain as set forth in the amino acid sequence of SEQ ID NO: 181 and a light chain as set forth in the amino acid sequence of SEQ ID NO: 217.

According to an embodiment of the present application, the antibody comprises a heavy chain as set forth in the amino acid sequence of SEQ ID NO: 182 and a light chain as set forth in the amino acid sequence of SEQ ID NO: 216.

According to an embodiment of the present application, the antibody comprises a heavy chain as set forth in the amino acid sequence of SEQ ID NO: 182 and a light chain as set forth in the amino acid sequence of SEQ ID NO: 217.

According to an embodiment of the present application, the antibody comprises at least one selected from the group consisting of a monoclonal antibody, a polyclonal antibody, a multimeric antibody, and a CDR grafted antibody.

Herein, the term "monoclonal antibody" refers to an antibody that can recognize only one specific antigen epitope. Common monoclonal antibodies comprise two light chains with lighter molecular weights and two heavy chains with heavier molecular weights. The heavy chains (H chains) and light chains (L chains) are linked by disulfide bonds to form a tetrapeptide chain molecule. The amino acid sequence at the amino-terminal end (N-terminus) of the heavy or light chain is highly variable, termed the variable region (V region). The amino acid sequence at the carboxyl-terminal end (C-terminus) of the heavy or light chain is relatively stable with little variation, termed the constant region (C region). The V regions of the L and H chains are termed VL and VH, respectively. Furthermore, the monoclonal antibody also includes, but is not limited to, at least one of a single-chain antibody, a Fab antibody, an Fv antibody, a single-domain antibody, and a minimal recognition unit.

Herein, the terms "CDR grafted antibody" and "reshaped antibody" both refer to grafting the CDRs of a monoclonal antibody from one species into the variable region of an antibody from another species. For example, the CDRs of a murine monoclonal antibody can be grafted into a human antibody variable region to replace the human antibody CDRs, thereby conferring the antigen-binding specificity of the murine monoclonal antibody onto the human antibody while reducing its heterology. It should be noted that both polyclonal antibodies and monoclonal antibodies in the present application can be CDR grafted antibodies.

According to an embodiment of the present application, the antigen-binding fragment comprises at least one of a Fab fragment, a (Fab)₂ fragment, an scFv-Fc fusion protein, an scFv-Fv fusion protein, an Fv fragment, and a minimal recognition unit.

In another aspect of the present application, the present application provides a nucleic acid molecule. According to an embodiment of the present application, the nucleic acid molecule encodes the aforementioned antibody or antigen-binding fragment thereof. The nucleic acid molecule according to an embodiment of the present application encodes the aforementioned antibody or the antigen-binding fragment.

According to an embodiment of the present application, the nucleic acid molecule is DNA.

It should be noted that, for the nucleic acid molecules mentioned herein, a person skilled in the art should understand that the nucleic acid molecules actually include either strand of complementary double strands, or both strands. For convenience, although only one strand is given in most cases in the specification and claims, in fact, the other complementary strand is also disclosed. Additionally, the nucleic acid sequences in the present application include a DNA form or an RNA form, and disclosing one form implies the other is also disclosed.

In yet another aspect of the present application, the present application provides an expression vector. According to an embodiment of the present application, the expression vector carries the aforementioned nucleic acid molecule. When ligating the aforementioned nucleic acid molecule into a vector, the nucleic acid molecule can be directly or indirectly connected to control elements on the vector, as long as these control elements can control the translation and expression of the nucleic acid molecule. Certainly, these control elements can be derived directly from the vector itself, or can be exogenous, i.e., not derived from the vector itself. Certainly, it is sufficient that the nucleic acid molecule is operably linked to the control element. As used herein, "operably linked" refers to linking an exogenous gene to a vector such that control elements within the vector, such as transcription control sequences and translation control sequences, can perform their intended function of regulating the transcription and translation of the exogenous gene. Commonly used vectors can be, for example, plasmids, phages, and others. After the expression vector according to some specific embodiments of the present application is introduced into a suitable recipient cell, it can effectively achieve the expression of the aforementioned antibody or antigen-binding fragment under the mediation of a regulatory system, thereby achieving large-scale obtainment of the antibody or antigen-binding fragment in vitro.

According to an embodiment of the present application, the expression vector is a eukaryotic expression vector or a prokaryotic expression vector. Preferably, the expression vector is a plasmid expression vector.

In yet another aspect of the present application, the present application provides a recombinant cell. According to an embodiment of the present application, the recombinant cell: carries the aforementioned nucleic acid molecule; or expresses the aforementioned antibody or antigen-binding fragment thereof. Using this recombinant cell under suitable conditions, the aforementioned antibody or antigen-binding fragment can be effectively expressed within the cell.

It should be noted that "suitable conditions" as described in the specification of the present application refer to conditions suitable for the expression of the antibody or antigen-binding fragment of the present application. A person skilled in the art can easily understand that conditions suitable for the expression of the antibody or antigen-binding fragment include, but are not limited to, suitable transformation or transfection methods, suitable transformation or transfection conditions, healthy host cell status, suitable host cell density, suitable cell culture environment, and suitable cell culture time. The "suitable conditions" are not particularly limited, and a person skilled in the art can optimize the most suitable conditions for the expression of the antibody or antigen-binding fragment according to the specific environment of the laboratory.

According to an embodiment of the present application, the recombinant cell is obtained by introducing the aforementioned expression vector into a host cell.

According to an embodiment of the present application, the recombinant cell is a eukaryotic cell.

According to an embodiment of the present application, the recombinant cell is a mammalian cell.

In yet another aspect of the present application, the present application provides a pharmaceutical composition. According to an embodiment of the present application, the pharmaceutical composition comprises: the aforementioned antibody or antigen-binding fragment thereof; the aforementioned nucleic acid molecule; the aforementioned expression vector; or the aforementioned recombinant cell. The pharmaceutical composition of the present application can specifically bind to GDF15, effectively block the binding of GFRAL and GDF15, and also effectively inhibit Treg differentiation, and therefore can be used for preventing and/or treating cancer and cachexia.

According to an embodiment of the present application, the pharmaceutical composition further comprises a pharmaceutically acceptable excipient.

In yet another aspect of the present application, the present application provides a combination medicament or pharmaceutical kit. According to an embodiment of the present application, the combination medicament or pharmaceutical kit comprises: the aforementioned antibody or antigen-binding fragment thereof or the aforementioned pharmaceutical composition as a first active ingredient; and an anti-PDL1 agent as a second active ingredient. The combination medicament or pharmaceutical kit of the present application can effectively block the binding of GFRAL and GDF15, and also effectively inhibit Treg differentiation, and therefore can be used for preventing and/or treating cancer and cachexia.

Herein, the term "anti-PDL1 agent" refers to an agent that can be used to inhibit PDL1 expression or activity, for example, by inhibiting the activity of PDL1 to block the PD1/PDL1 pathway. The "anti-PDL1 agent" includes, but are not limited to, an anti-PDL1 antibody.

Exemplarily, the anti-PDL1 agent is Atezolizumab (Tecentriq).

In yet another aspect of the present application, the present application provides use of the aforementioned antibody or antigen-binding fragment thereof, the aforementioned nucleic acid molecule, the aforementioned expression vector, the aforementioned recombinant cell, the aforementioned pharmaceutical composition, or the aforementioned combination medicament or pharmaceutical kit in the manufacture of a medicament, wherein the medicament is for preventing and/or treating cancer and cachexia.

In yet another aspect of the present application, the present application provides a method for preventing and/or treating cancer and cachexia. According to an embodiment of the present application, the method comprises: administering to a subject a pharmaceutically acceptable amount of the aforementioned antibody or antigen-binding fragment, the aforementioned pharmaceutical composition, or the aforementioned combination medicament or pharmaceutical kit. The method of the present application can effectively prevent and/or treat cancer and cachexia.

The effective amount of the antibody or antigen-binding fragment or pharmaceutical composition of the present application can vary depending on the mode of administration and the severity of the disease to be treated. The selection of a preferred effective amount can be determined by a person of ordinary skill in the art based on various factors (e.g., through clinical trials). Such factors include, but are not limited to: the pharmacokinetic parameters of the active ingredient such as bioavailability, metabolism, half-life, and others; the severity of the disease to be treated in the patient; the body weight of the patient; the immune status of the patient; the route of administration; and others. For example, depending on the urgency of the treatment situation, several divided doses may be administered daily, or the dose may be proportionally reduced.

The antibody or antigen-binding fragment or pharmaceutical composition of the present application can be incorporated into medicaments suitable for parenteral administration (e.g., intravenous, subcutaneous, intraperitoneal, intramuscular administration, etc.). These medicaments can be prepared in various forms. For example, liquid, semi-solid, and solid dosage forms, etc., including but not limited to liquid solutions (e.g., injectable solutions and infusion solutions) or lyophilized powders. A typical pharmaceutical composition is in the form of an injectable solution or infusion solution. The aforementioned antibody or antigen-binding fragment or the aforementioned pharmaceutical composition can be administered by intravenous infusion or injection or intramuscular or subcutaneous injection.

In yet another aspect of the present application, the present application provides a kit. According to an embodiment of the present application, the kit comprises: the aforementioned antibody or antigen-binding fragment thereof; the aforementioned nucleic acid molecule; the aforementioned expression vector; or the aforementioned recombinant cell. The antibody or antigen-binding fragment in the kit provided by the present application can effectively bind to GDF15. Furthermore, under suitable conditions, the nucleic acid molecule, expression vector, or recombinant cell can all express the antibody or antigen-binding fragment. Furthermore, a kit comprising the above substances can effectively bind to GDF15 and can be used for effective detection of GDF15. The kit can be used for scientific research, such as qualitative or quantitative detection of GDF15 in a biological sample, and can also be used to judge the state of an individual, such as determining whether the GDF15 level of the individual is higher or lower than a normal level after obtaining the GDF15 level of the individual. The biological sample can be cells, tissues, blood, and others.

In yet another aspect of the present application, the present application provides a use of the aforementioned antibody or antigen-binding fragment thereof, the aforementioned nucleic acid molecule, the aforementioned expression vector, or the aforementioned recombinant cell in the manufacture of a kit, wherein the kit is for detecting GDF15.

In yet another aspect of the present application, the present application provides a method for detecting GDF15. According to an embodiment of the present application, the method comprises: using the aforementioned antibody or antigen-binding fragment thereof, or the aforementioned kit to detect a sample to be tested. As can be seen from the above, the aforementioned antibody or antigen-binding fragment can effectively bind to GDF15. Thus, the method of the present application can effectively detect GDF15, such as qualitatively or quantitatively detecting GDF15 in a biological sample, and can also be used to judge the state of an individual, such as determining whether the GDF15 level of the individual is higher or lower than a normal level after obtaining the GDF15 level of the individual. The biological sample can be cells, tissues, blood, and others.

According to an embodiment of the present application, the antibody or antigen-binding fragment thereof forms an immune complex with GDF15 in the sample to be tested; the presence of the immune complex is detected, and whether the sample to be tested contains GDF15 is determined based on the presence of the immune complex.

According to an embodiment of the present application, the presence of the immune complex indicates the presence of GDF15 in the sample to be tested.

According to an embodiment of the present application, the immune complex further comprises a second antibody, wherein the second antibody binds to the antibody.

According to an embodiment of the present application, the immune complex further comprises a second antibody, wherein the second antibody binds to GDF15.

The method for detecting GDF15 can be any method known to those skilled in the art. For example, after GDF15 contacts the solid-phase support, GDF15 is detected using an additional another antibody against GDF15 (which is typically labeled with a signal substance, or detected using a secondary antibody labeled with a signal substance) (double-antibody sandwich).

Alternatively, a conventional antibody is used as the antibody coated on the solid phase and incubated with the GDF15 to be tested, and detection is performed using the immunoglobulin as a free detection antibody (which is typically labeled with a signal substance or detected using a secondary antibody labeled with a signal substance).

Alternatively, the solid-phase support is used in conjunction with the immunoglobulin to detect GDF15, in which case they are paired antibodies.

The aforementioned signal substance can be any one of a fluorescent substance, a quantum dot, a digoxigenin-labeled probe, biotin, a radioactive isotope, a radiographic contrast agent, a paramagnetic ion fluorescent microsphere, an electron-dense substance, a chemiluminescent label, an ultrasound contrast agent, a photosensitizer, colloidal gold, or an enzyme. In some embodiments, the signal substance is colloidal gold, fluorescein, fluorescent microspheres, acridinium ester, horseradish peroxidase, alkaline phosphatase, or β-galactosidase.

Additional aspects and advantages of the present application will be partially presented in the following description, and in part will be obvious from the following description, or will be learned by practice of the present application.

### DESCRIPTION OF THE DRAWINGS

The above and/or additional aspects and advantages of the present application will become apparent and easily understood from the description of the embodiments in combination with the following drawings, wherein:
Figure 1 shows the detection results of the binding activity of each anti-GDF15 neutralizing antibody in Test Example 1 of the present application to human GDF15 protein;
Figure 2 shows the detection results of the binding activity of each anti-GDF15 neutralizing antibody in Test Example 1 of the present application to murine GDF15 protein;
Figure 3 shows the detection results of the binding activity of each anti-GDF15 neutralizing antibody in Test Example 1 of the present application to cynomolgus monkey GDF15 protein;
Figure 4 shows the neutralization activity detection results of each anti-GDF15 neutralizing antibody in Test Example 1 of the present application in a reporter gene cell strain (mGFRAL);
Figure 5 shows the activity detection results of inhibiting Treg differentiation by each anti-GDF15 neutralizing antibody in Test Example 1 of the present application;
Figure 6 shows the reporter gene assay activity detection results of the anti-GDF15 neutralizing antibody after PTM modification in Test Example 2 of the present application;
Figure 7 shows the detection results of inhibiting Treg differentiation by the anti-GDF15 neutralizing antibody after PTM modification in Test Example 2 of the present application (Day 2);
Figure 8 shows the detection results of inhibiting Treg differentiation by the anti-GDF15 neutralizing antibody after PTM modification in Test Example 2 of the present application (Days 5 and 7);
Figure 9 shows a summary bar graph of the results of inhibiting Treg differentiation by the anti-GDF15 neutralizing antibody after PTM modification in Test Example 2 of the present application;
Figure 10 shows the detection results of the affinity kinetics of the humanized anti-GDF15 neutralizing antibody to GDF15 in Test Example 3 of the present application;
Figure 11 shows the detection results of the affinity kinetics of the humanized anti-GDF15 neutralizing antibody to GDF15 in Test Example 3 of the present application;
Figure 12 shows the detection results of the affinity kinetics of the humanized anti-GDF15 neutralizing antibody to GDF15 in Test Example 3 of the present application;
Figure 13 shows the detection results of the affinity kinetics of the humanized anti-GDF15 neutralizing antibody to GDF15 in Test Example 3 of the present application;
Figure 14 shows the detection results of the affinity kinetics of the humanized anti-GDF15 neutralizing antibody to GDF15 in Test Example 3 of the present application;
Figure 15 shows the detection results of the affinity kinetics of the humanized anti-GDF15 neutralizing antibody to GDF15 in Test Example 3 of the present application;
Figure 16 shows the detection results of the affinity kinetics of the humanized anti-GDF15 neutralizing antibody to GDF15 in Test Example 3 of the present application;
Figure 17 shows the detection results of the affinity kinetics of the humanized anti-GDF15 neutralizing antibody to GDF15 in Test Example 3 of the present application;
Figure 18 shows the detection results of the affinity kinetics of the humanized anti-GDF15 neutralizing antibody to GDF15 in Test Example 3 of the present application;
Figure 19 shows the reporter gene assay activity detection results of the humanized anti-GDF15 neutralizing antibody in Test Example 3 of the present application;
Figure 20 shows the reporter gene assay activity detection results of the humanized anti-GDF15 neutralizing antibody in Test Example 3 of the present application;
Figure 21 shows the activity results of the humanized anti-GDF15 neutralizing antibody and comparative antibodies detected by reporter gene assay in Test Example 3 of the present application;
Figure 22 shows a bar graph of the results of inhibition of Treg differentiation by the humanized anti-GDF15 neutralizing antibody in Test Example 3 of the present application;
Figure 23 shows the results of the effect of the anti-GDF15 antibody in reversing cachexia in the HT1080 cachexia model in Test Example 3 of the present application, wherein Figure 23A shows body weight change, and Figure 23B shows body weight change rate;
Figure 24 shows the forelimb grip strength results (Figure 24A) and four-limb grip strength results (Figure 24B) of the effect of the anti-GDF15 antibody in reversing cachexia in the HT1080 cachexia model in Test Example 3 of the present application;
Figure 25 shows the results of the effect of the anti-GDF15 antibody in reversing cachexia in the HT1080 cachexia model in Test Example 3 of the present application, wherein Figure 25A shows fat mass change, and Figure 25B shows muscle mass change;
Figure 26 shows the results of the effect of the anti-GDF15 antibody in reversing cachexia in the HT1080 cachexia model in Test Example 3 of the present application, wherein Figure 26A shows serum free GDF15 level in animals before administration, and Figure 26B shows serum free GDF15 level in animals after administration;
Figure 27 shows the results of the effect of the anti-GDF15 antibody in reversing cachexia in the LNCaP cachexia model in Test Example 3 of the present application, wherein Figure 27A shows body weight change, and Figure 27B shows body weight change rate;
Figure 28 shows the results of the effect of the anti-GDF15 antibody in reversing cachexia in the LNCaP cachexia model in Test Example 3 of the present application, wherein Figure 28A shows fat mass change, Figure 28B shows muscle mass change, Figure 28C shows fat mass change rate, and Figure 28D shows muscle mass change rate;
Figure 29 shows the body weight change results of the effect of the anti-GDF15 antibody in reversing cachexia in the MKN45 cachexia model in Test Example 3 of the present application;
Figure 30 shows the results of the effect of the anti-GDF15 antibody in reversing cachexia in the MKN45 cachexia model in Test Example 3 of the present application, wherein Figure 30A shows fat mass change, Figure 30B shows muscle mass change, Figure 30C shows fat mass change rate, and Figure 30D shows muscle mass change rate;
Figure 31 shows the results of the effect of the anti-GDF15 antibody in reversing cachexia in the MKN45 cachexia model in Test Example 3 of the present application;
Figure 31A shows serum free GDF15 levels in animals at the end of the experiment, and Figure 31B shows serum total GDF15 levels in animals at the end of the experiment;
Figure 32 shows the survival curves of groups G1 to G9 in Test Example 3 of the present application; and
Figure 33 shows the tumor volume and TGI results of groups G1 to G7 in Test Example 3 of the present application.

### DETAILED DESCRIPTION

The embodiments of the present application are described in detail below. The embodiments described below are exemplary and are only for explaining the present application, and should not be construed as limiting the present application.

It should be noted that the terms "first" and "second" are used for descriptive purposes only, and should not be understood as indicating or implying relative importance or implicitly indicating the number of indicated technical features. Thus, features defined as "first" and "second" may explicitly or implicitly include one or more of said features. Further, in the description of the present application, unless otherwise specified, "a plurality" means two or more.

Herein, the terms "comprising", "including", or "containing" are open-ended expressions, meaning encompassing the content specified by the present application, but not excluding other aspects.

Herein, the terms "optionally" or "optional" generally mean that the subsequently described event or circumstance may but need not occur, and that the description includes instances where the event or circumstance occurs and instances where it does not.

Herein, the amino acid numbering for the IgG1 portion is according to the EU numbering system. For example, position 334 refers to position 334 according to the EU numbering system; L234A means that the leucine at position 234 according to the EU numbering system is substituted by alanine; L235A means that the leucine at position 235 according to the EU numbering system is substituted by alanine; G237A means that the glycine at position 237 according to the EU numbering system is substituted by alanine; "K447" means that the lysine at position 447 according to the EU numbering system is deleted.

Herein, the term "antibody" generally refers to an antibody that can recognize one or more antigenic epitopes, including but not limited to monoclonal antibodies, polyclonal antibodies, multimeric antibodies, and CDR grafted antibodies.

Herein, the term "Fab antibody" generally refers to an antibody containing only a Fab molecule, which is composed of the VH and CH1 of a heavy chain and a complete light chain, with the light chain and heavy chain linked by a disulfide bond.

Herein, the term "Fv antibody" generally refers to an antibody composed only of a light chain variable region (VL) and a heavy chain variable region (VH) linked by non-covalent bonds, and is the smallest functional fragment of an antibody that retains the complete antigen-binding site.

Herein, the terms "single-domain antibody", "nanobody", and "VHH antibody" are used interchangeably and were originally described as the antigen-binding immunoglobulin (variable) domain of "heavy chain antibodies" (i.e., "antibodies devoid of light chains") (Hamers-Casterman C, Atarhouch T, Muyldermans S, Robinson G, Hamers C, Songa EB, Bendahman N, Hamers R.: "Naturally occurring antibodies devoid of light chains"; Nature 363, 446-448 (1993)), containing only a heavy chain variable region (VH) and conventional CH2 and CH3 regions, which specifically bind to the antigen through the heavy chain variable region.

Herein, the term "single-chain antibody" generally refers to an antibody composed of a heavy chain variable region (VH) and a light chain variable region (VL) linked by a linker peptide.

Herein, the terms "minimal recognition unit" and "MRU" both refer to an antibody composed of only one CDR, whose molecular weight is very small, accounting for only about 1% of the complete antibody.

Herein, the terms "identity", "homology" or "similarity" are all used to describe the percentage of identical amino acids or nucleotides between two amino acid sequences or nucleic acid sequences determined by conventional methods when described relative to a reference sequence. See, for example, Ausubel et al., eds. (1995), Current Protocols in Molecular Biology, Chapter 19 (Greene Publishing and Wiley-Interscience, New York); and the ALIGN program (Dayhoff (1978), Atlas of Protein Sequence and Structure 5: Suppl. 3 (National Biomedical Research Foundation, Washington, D.C.)). There are many algorithms for aligning sequences and determining sequence identity, including the homology alignment algorithm of Needleman et al. (1970) J. Mol. Biol. 48: 443; the local homology algorithm of Smith et al. (1981) Adv. Appl. Math. 2: 482; the similarity search method of Pearson et al. (1988) Proc. Natl. Acad. Sci. USA 85: 2444; the Smith-Waterman algorithm (Meth. Mol. Biol. 70: 173-187 (1997)); and the BLASTP, BLASTN, and BLASTX algorithms (see Altschul et al. (1990) J. Mol. Biol. 215: 403-410). Computer programs utilizing these algorithms are also available and include, but are not limited to: ALIGN or Megalign (DNASTAR) software, or WU-BLAST-2 (Altschul et al., Meth. Enzym., 266: 460-480 (1996)); or GAP, BESTFIT, BLAST (Altschul et al., supra), FASTA, and TFASTA, available in the Genetics Computing Group (GCG) package, Version 8, Madison, Wisconsin, USA; and CLUSTAL in the PC/Gene program provided by Intelligenetics, Mountain View, California.

Herein, the term "at least 66% identity" refers to at least 66% identity with each reference sequence, which can be 66%, 67%, 70%, 75%, 80%, 81%, 82%, 83%, 84%, 85%, 86%, 87%, 88%, 89%, 90%, 91%, 92%, 93%, 94%, 95%, 96%, 97%, 98%, 99%, 99.5%, and 99.9% identity.

Herein, the term "expression vector" generally refers to a nucleic acid molecule capable of self-replication in a suitable host into which it is inserted, which transfers an inserted nucleic acid molecule into and/or between host cells. The expression vector may include vectors primarily intended for inserting DNA or RNA into cells, vectors primarily intended for replicating DNA or RNA, and expression vectors primarily intended for transcription and/or translation of DNA or RNA. The expression vector also includes vectors having multiple of the above functions. The expression vector can be a polynucleotide capable of being transcribed and translated into a polypeptide when introduced into a suitable host cell. Typically, by culturing a suitable host cell comprising the expression vector, the expression vector can produce the desired expression product.

Herein, the term "recombinant cell" generally refers to a cell whose genetic material has been modified or recombined using genetic engineering techniques or cell fusion techniques to obtain a cell with unique stably inherited traits. The term "host cell" refers to a prokaryotic or eukaryotic cell into which a recombinant expression vector can be introduced. The terms "transformed" or "transfected" as used herein refer to the introduction of a nucleic acid (e.g., a vector) into a cell by various techniques known in the art. Suitable host cells can be transformed or transfected with the DNA sequences of the present application and can be used for the expression and/or secretion of the target protein. Examples of suitable host cells that can be used in the present application include immortalized hybridoma cells, NS/0 myeloma cells, 293 cells, Chinese Hamster Ovary (CHO) cells, HeLa cells, Cap cells (human amniotic fluid-derived cells), and CoS cells.

Herein, the term "pharmaceutical composition" generally refers to a unit dosage form and can be prepared by any of the methods well known in the pharmaceutical arts. All methods include the step of bringing the active ingredient into association with a carrier that constitutes one or more accessory ingredients. Typically, the compositions are prepared by uniformly and intimately bringing the active antibody or antigen-binding fragment thereof into association with a liquid carrier, a finely divided solid carrier, or both.

Herein, the term "pharmaceutically acceptable excipient" can include any solvent, solid excipient, diluent, or other liquid excipient, etc., suitable for the particular intended dosage form. Except to the extent that any conventional excipient is incompatible with the antibody or antigen-binding fragment thereof of the present application, such as by producing any undesirable biological effect or interacting in a deleterious manner with any other component of the pharmaceutically acceptable composition, its use is also contemplated within the scope of the present application.

Herein, the term "administration" refers to introducing a predetermined amount of a substance into a patient by a suitable approach. The antibody or antigen-binding fragment or pharmaceutical composition of the present application can be administered by any common route as long as it can reach the intended tissue. Various modes of administration are contemplated, including intraperitoneal, intravenous, intramuscular, subcutaneous injection, etc., but the present application is not limited to these exemplified modes of administration.

Herein, the term "treatment" refers to obtaining desired pharmacological and/or physiological effects. The effects may be prophylactic in terms of completely or partially preventing a disease or its symptoms, and/or therapeutic in terms of partially or completely curing a disease and/or adverse effects caused by the disease. "Treatment", as used herein, covers treatment of a disease in a mammal, particularly a human, including: (a) preventing the disease or condition from occurring in an individual who may be predisposed to the disease but has not yet been diagnosed as having it; (b) inhibiting the disease, e.g., arresting its development; or (c) relieving the disease, e.g., alleviating symptoms associated with the disease. "Treatment" as used herein covers any administration of a drug or antibody or antigen-binding fragment thereof to an individual for the purpose of treating, curing, alleviating, improving, reducing, or inhibiting a disease in the individual, including but not limited to administering a medicament comprising the antibody or antigen-binding fragment thereof described herein to an individual in need thereof.

Herein, "anti-GDF15 neutralizing monoclonal antibody", "anti-GDF15 neutralizing antibody", "GDF15 neutralizing antibody" and "GDF15 antibody" are synonymous.

The present application provides an antibody, a nucleic acid molecule, an expression vector, a recombinant cell, a pharmaceutical composition and use thereof, and a kit and use thereof, which will be described in detail below.

### Antibody

Herein, the term "antibody" is an immunoglobulin molecule capable of binding to a specific antigen. It comprises two light chains with lighter molecular weights and two heavy chains with heavier molecular weights. The heavy chains (H chains) and light chains (L chains) are linked by disulfide bonds to form a tetrapeptide chain molecule. The amino acid sequence at the amino-terminal end (N-terminus) of the peptide chain is highly variable, termed the variable region (V region). The carboxyl-terminal end (C-terminus) is relatively stable with little variation, termed the constant region (C region). The V regions of the L and H chains are referred to as VL and VH, respectively.

Certain regions within the variable region have a higher degree of variability in amino acid composition and arrangement order, called hypervariable regions (HVRs). The hypervariable regions are the sites where an antigen and antibody bind, and are therefore also called complementarity-determining regions (CDRs). There are three CDRs on both the heavy chain variable region and the light chain variable region.

The present application used human GDF15 or murine GDF15 with an hFc or his tag as antigens to immunize female BALB/c, A/J, and C57bl/6 mice. Through hybridoma fusion technology, 15 strains of murine antibodies capable of specifically binding to human GDF15 protein were screened. These antibody fragments can block the binding of GDF15 and GFRAL, and furthermore, these antibodies have the function of inhibiting Treg differentiation, demonstrating more excellent tumor immune activity. Therefore, these antibodies can effectively prevent and treat cancer and cachexia.

In some embodiments, the present application provides an antibody or antigen-binding fragment thereof, wherein the antibody or antigen-binding fragment thereof comprises: a CDR sequence selected from the group consisting of at least one of the following, or an amino acid sequence having at least 66% identity thereto: heavy chain variable region CDR sequences: SEQ ID NOs: 1 to 34 and SEQ ID NOs: 74 to 76; light chain variable region CDR sequences: SEQ ID NOs: 36 to 73 and SEQ ID NOs: 77 to 82. In other embodiments, the antibody or antigen-binding fragment provided by the present application has conservative amino acid substitutions compared to the above heavy and light chains. "Antigen-binding fragment" refers to an antibody fragment that retains the ability to specifically bind to an antigen. Examples of antigen-binding fragments include, but are not limited to, at least one of an Fv fragment, a disulfide-stabilized Fv fragment (dsFv), a Fab fragment, an (Fab)₂, an scFv-Fc fusion protein, an scFv-Fv fusion protein, an Fv-Fc fusion protein, a multispecific antibody formed by antigen-binding fragments, a single-domain antibody, a bivalent domain antibody, a VHH nanobody, or a minimal recognition unit. "Conservative amino acid substitution" refers to the amino acid being substituted by another residue that is biologically similar, chemically similar, or structurally similar. "Biologically similar" means that the substitution does not destroy the biological activity of the GDF15 antibody or the GDF15 antigen. "Structurally similar" means that the amino acids have side chains of similar length, such as alanine, glycine, or serine, or have side chains of similar size. "Chemical similar" means that the amino acids have the same charge or are both hydrophilic or hydrophobic. For example, hydrophobic residues isoleucine, valine, leucine, or methionine are substituted for one another. Or a polar amino acid such as arginine is substituted for lysine, glutamic acid for aspartic acid, glutamine for asparagine, serine for threonine, and others.

In some embodiments, the present application provides an antibody or antigen-binding fragment thereof, wherein the antibody has a heavy chain variable region of the amino acid sequence as set forth in any one of SEQ ID NOs: 83 to 100, SEQ ID NOs: 124 to 127, SEQ ID NOs: 132 to 135, SEQ ID NOs: 140 to 141, and SEQ ID NOs: 144 to 145; or the antibody has a light chain variable region of the amino acid sequence as set forth in any one of SEQ ID NOs: 102 to 122, SEQ ID NOs: 128 to 131, SEQ ID NOs: 136 to 139, SEQ ID NOs: 142 to 143, and SEQ ID NOs: 146 to 147. The inventors obtained the CDR regions of the heavy chain variable region sequences (as set forth in SEQ ID NOs: 1 to 34 and SEQ ID NOs: 74 to 76) and the CDR regions of the light chain variable region sequences (as set forth in SEQ ID NOs: 36 to 73 and SEQ ID NOs: 77 to 82) of the above antibodies through antibody sequence alignment databases (Kabat) or related software. In other embodiments, the heavy chain variable region sequence of the antibody or antigen-binding fragment has conservative amino acid substitutions compared to the amino acid sequences as set forth in any one of SEQ ID NOs: 83 to 100, SEQ ID NOs: 124 to 127, SEQ ID NOs: 132 to 135, SEQ ID NOs: 140 to 141, and SEQ ID NOs: 144 to 145. In some embodiments, the light chain variable region sequence of the antibody or antigen-binding fragment has conservative amino acid substitutions compared to the amino acid sequences as set forth in any one of SEQ ID NOs: 102 to 122, SEQ ID NOs: 128 to 131, SEQ ID NOs: 136 to 139, SEQ ID NOs: 142 to 143, and SEQ ID NOs: 146 to 147. Certainly, these conservative amino acid substitutions will not change the biological function of the antibody or antigen-binding fragment. In some specific embodiments, such conservative amino acid substitutions can occur on amino acids outside the CDR regions in the heavy chain variable region and light chain variable region.

In some preferred embodiments, the present application provides an antibody, wherein the antibody has a heavy chain as set forth in the amino acid sequence of any one of SEQ ID NOs: 150 to 164, 166 to 182, 229 and a light chain as set forth in the amino acid sequence of any one of SEQ ID NOs: 183 to 197, 199 to 217.

### Nucleic Acid Molecule, Expression Vector, Recombinant Cell

In the process of preparing or obtaining these antibodies or antigen-binding fragments thereof, nucleic acid molecules expressing these antibodies or antigen-binding fragments thereof can be used, ligated with different vectors, and then expressed in different cells to obtain the corresponding antibodies or antigen-binding fragments thereof.

To this end, the present application also provides an isolated nucleic acid molecule, wherein the nucleic acid molecule encodes the antibody or antigen-binding fragment thereof described above.

In some preferred embodiments, the nucleic acid molecule is optimized for species to facilitate expression in prokaryotic cells and mammalian cells. For a person skilled in the art, designing and obtaining nucleotide sequences, through a known amino acid sequence of an antibody and by selecting appropriate preferred codons according to the requirements of the expression vector, is a routine genetic engineering technique.

The present application also provides an expression vector, wherein the expression vector comprises the above isolated nucleic acid molecule. When ligating the above isolated polynucleotide into a vector, the polynucleotide can be directly or indirectly connected to control elements on the vector, as long as these control elements can control the translation and expression of the polynucleotide. Certainly, these control elements can be derived directly from the vector itself, or can be exogenous, i.e., not derived from the vector itself. Certainly, it is sufficient that the polynucleotide is operably linked to the control element.

As used herein, "operably linked" refers to linking an exogenous gene to a vector such that control elements within the vector, such as transcription control sequences and translation control sequences, can perform their intended function of regulating the transcription and translation of the exogenous gene. Certainly, the polynucleotides encoding the antibody heavy and light chains can be inserted independently into different vectors, but are commonly inserted into a same vector. Commonly used vectors can be, for example, plasmids, phages, and others.

The present application also provides a recombinant cell, wherein the recombinant cell comprises the expression vector. The expression vector can be introduced into mammalian cells to construct recombinant cells, and then these recombinant cells are used to express the antibody or antigen-binding fragment provided by the present application. By culturing the recombinant cells, the corresponding antibody can be obtained. Suitable mammalian cells can be, for example, CHO, 293, 293T, BHK cells, and others.

Pharmaceutical Composition, Combination Medicament, Pharmaceutical Use and Use in Kit Manufacture

The present application also provides a pharmaceutical composition, wherein the pharmaceutical composition comprises the aforementioned antibody or antigen-binding fragment thereof, and may also comprise the aforementioned nucleic acid molecule, expression vector, or recombinant cell.

The GDF15 antibodies provided herein can be incorporated into pharmaceutical compositions suitable for administration to a subject. Typically, these pharmaceutical compositions comprise the GDF15 antibodies provided herein.

In some embodiments, these pharmaceutical compositions further comprise a pharmaceutically acceptable carrier, suitable for the particular intended dosage form. Except to the extent that any conventional excipient is incompatible with the antibody or antigen-binding fragment thereof of the present application, such as by producing any undesirable biological effect or interacting in a deleterious manner with any other component of the pharmaceutically acceptable composition, its use is also contemplated within the scope of the present application. For example, the antibody of the present application can be incorporated into pharmaceutical compositions suitable for parenteral administration (e.g., intravenous, subcutaneous, intraperitoneal, and intramuscular). These pharmaceutical compositions can be prepared in various forms. For example, liquid, semi-solid, and solid dosage forms, etc., including but not limited to liquid solutions (e.g., injectable solutions and infusion solutions), dispersions or suspensions, tablets, pills, powders, liposomes, and suppositories. A typical pharmaceutical composition is in the form of an injectable solution or infusion solution. The antibody can be administered by intravenous infusion or injection or intramuscular or subcutaneous injection.

The present application also provides a combination medicament or pharmaceutical kit. According to an embodiment of the present application, the combination medicament or pharmaceutical kit comprises: the aforementioned antibody or antigen-binding fragment thereof or the aforementioned pharmaceutical composition as a first active ingredient; and an anti-PDL1 agent as a second active ingredient.

Certainly, the GDF15 antibody herein can also be made into a kit or part of other diagnostic reagents as needed. According to an embodiment of the present application, the present application also provides a kit, wherein the kit comprises the aforementioned GDF15 antibody. The kit provided by the present application can be used, for example, for immunoblotting, immunoprecipitation, and other kits for detection involving the specific binding properties of the GDF15 antigen and the antibody. These kits may comprise any one or more of the following: antagonists, GDF15 antibodies or drug reference materials; protein purification columns; immunoglobulin affinity purification buffers; cell assay diluents; instructions or literature, and others. GDF15 antibodies can be used in different types of diagnostic tests, for example, to detect the presence of various diseases or drugs, toxins, or other proteins in vitro or in vivo. For example, GDF15 can be tested by testing the serum or blood of a subject.

The GDF15 antibody and/or the drug containing the GDF15 antibody of the present application can prevent and/or treat cachexia or cancer, wherein the cachexia is caused by tumors or other chronic diseases or injuries. These cancers (or tumors) can be any uncontrolled cell growth. Specifically, they can be lung cancer, colorectal cancer, gastric cancer, liver cancer, esophageal cancer, pancreatic cancer, non-small cell lung cancer, papillary thyroid cancer, glioblastoma multiforme, head and neck cancer, kidney cancer, bladder cancer, breast cancer, ovarian cancer, cholangiocarcinoma or sarcoma, acute myeloid leukemia, large cell neuroendocrine carcinoma, neuroblastoma, prostate cancer, neuroblastoma, melanoma, head and neck squamous cell carcinoma or gastric cancer, and others; other chronic diseases include but are not limited to renal failure, AIDS, malabsorption, and severe sepsis, and others; injuries include but are not limited to severe trauma, post-surgery, massive blood loss, and others.

When using the GDF15 antibody provided by the present application to treat the aforementioned diseases, it is sufficient that the GDF15 antibody provided by the present application can be administered to a subject. To this end, the present application provides a method for treating the above diseases, comprising administering to a subject in need thereof the antibody or antigen-binding fragment thereof, pharmaceutical composition, or combination medicament or pharmaceutical kit provided by the present application.

### Cell Line Models

The cachexia tumor cell line models mentioned herein may include various tumor cell lines, including but not limited to HT1080 (human fibrosarcoma), LNCAP (human prostate cancer) cell line, MKN45 (human gastric cancer) cell line, K562 (human myeloid leukemia) cell line, A549, HeLa, MCF-7, HCT-116, Panc-1, T24, BT16, A498, and others.

The model can be established in various ways, with the aim of simulating cachexia symptoms caused by tumors or other chronic diseases or injuries. By way of example only, a human tumor cell line can be inoculated into an immunodeficient animal model to induce cachexia symptoms, such as decreased animal body weight, reduced food intake, and others. The administration mode can be subcutaneous (SC) and/or intraperitoneal (IP), and the recommended administration frequency is 5-30 mg/kg, optionally including 10 mg/kg/Q3D, 20 mg/kg/Q6D, 10 mg/kg/Q7D, 20 mg/kg/Q3D, and others.

It should be noted that "mg/kg/QnD" means the dosage administered once every n days. Exemplarily, 10 mg/kg/Q3D means administration once every three days at a dosage of 10 mg/kg.

Observation indicators include animal body weight change, fat and muscle changes, and serum GDF15 concentration; the animals include but are not limited to monkeys, mice, rats, with monkeys including, but are not limited to, cynomolgus monkeys, rhesus monkeys, and others.

Overall, compared with the blank control, the antibody molecules related to the experiments in the present application can significantly reverse the trend of animal body weight loss, for example, can significantly reverse fat and/or muscle loss; the antibody molecules related to the experiments in the present application have relatively similar activity compared with the control antibody. By detecting the levels of free GDF15 and total GDF15 bound to antibody, the antibody molecules related to the experiments in the present application and the control antibody can both occupy the target well, with a target occupancy rate of over 90%.

Furthermore, the present application also conducted tumor inhibition experiments, detecting tumor size and observing tumor growth.

### In Vivo Efficacy Verification

The antibody molecules involved in the present application were used for in vivo efficacy verification, using, but not limited to, HT1080 (human fibrosarcoma), LNCAP (human prostate cancer) cell line, MKN45 (human gastric cancer) cell line, K562 (human myeloid leukemia) cell line, A549, HeLa, MCF-7, HCT-116, Panc-1, T24, BT16, A498, etc., inoculated into animals, including, but not limited to, mice, rats, cynomolgus monkeys, rhesus monkeys, etc., to establish animal models, test anti-tumor activity, and verify by detecting indicators such as tumor volume and survival rate, thereby determining the effect on tumors.

### Drug Developability Verification

The antibody molecules involved in the present application were prepared into formulations and incubated at a certain temperature under different formulation conditions, including but not limited to 20 to 50°C and 30 to 45°C, for example, 40°C, for an incubation time of one to five weeks, for example, four weeks, in order to perform stability testing for thermal stability and freeze-thaw stability (e.g., five repeated freeze-thaw cycles at -80°C/4°C), in three buffers: PBS, 529, and His. Detection methods such as Tm, Tagg, SEC, NR-CE-SDS, icIEF, and mass spectrometry analysis were performed. The test results demonstrate that the antibody molecules involved in the present application have good drug developability.

In the rat PK experiment, the antibody drug was injected into rats, blood was collected at fixed time points, the blood drug concentration was detected, and the PK of the drug in rats was calculated. The results showed that the half-lives of the antibody molecules involved in the present application and the comparative antibody (e.g., GDAb2, etc.) were similar, both within the normal range, meeting the quality requirements for drug developability, and no significant decrease in activity was observed after placement at 37°C. The results proved that the antibody molecules involved in the present application have good drug developability. Certainly, the PK experiment is not limited to rats, but can also be performed on model organisms commonly used in the art.

### Cytokine Release Verification

The antibody molecules of the present application were co-incubated with model cell lines, including but not limited to PBMC cells, etc., to detect cytokine release and predict possible cytokine storms caused by the antibodies. The results showed no significant increase in inflammatory cytokines (exemplarily, cytokines include but are not limited to IL-2, IL-4, IL-10, IL-12, TNF (TGFα, TGFβ), IFN-α, IFN-β, GM-CSF, IFNγ, VEGF, etc.).

### Biomarker Verification

The present application also relates to in vitro and in vivo biomarker verification, which can be performed by analyzing GDF15 serum concentration to verify whether there is expression of hGDF15 in the model, confirming successful modeling. Biomarker verification can be used for verification including but not limited to in vivo tumor immune models.

The solution of the present application will be explained below in conjunction with the embodiments. A person skilled in the art will understand that the following examples are only for illustrating the present application and should not be considered as limiting the scope of the present application. In the examples, where specific techniques or conditions are not indicated, the techniques or conditions described in the literature in the art or the product instructions are followed. Reagents or instruments without indicated manufacturers are conventional products that can be obtained through market purchase.

### Example 1: Preparation and Purification of Anti-GDF15 Neutralizing Antibodies

### I: Screening of Anti-GDF15 Neutralizing Antibodies and Determination of the Antibody Variable Regions thereof

### 1. Mouse Antigen Immunization

Anti-human GDF15 neutralizing antibodies were generated by immunizing mice. Experimental Balb/C, A/J, and C57bl/6 mice, female, 6 weeks old, were used. Rearing environment: SPF level. After purchase, the mice were acclimatized in the laboratory environment for 1 week under a 12/12 hour light/dark cycle regulation, temperature 20-25°C; humidity 40-60%. The acclimatized mice were immunized according to the following protocol.

Human GDF15 (Accession Q99988-1, Ala197-Ile308) or murine GDF15 (Accession Q9Z0J7, Ser189-Ala303) with an hFc or his tag was used as an antigen to immunize mice by single immunization or cross-immunization.

Immunization protocol: Routine immunization was performed with complete Freund's adjuvant (CFA) and incomplete Freund's adjuvant (IFA). For the primary immunization, the antigen and adjuvant CFA were mixed at a volume ratio of 1:1, and the injection dose was 50 µg/mouse/time; for booster immunizations, the antigen and adjuvant IFA were mixed at a volume ratio of 1:1, and the injection dose was 25 µg/mouse/time. The emulsified antigen was inoculated on days 0, 14, and 28. On day 0, 50 µg/mouse of emulsified antigen was injected intraperitoneally (IP) (primary immunization); on day 14, 25 µg/mouse of emulsified antigen was injected intraperitoneally (IP); and on day 28, 25 µg/mouse of emulsified antigen was injected intraperitoneally (IP). Blood was collected on days 21 and 35, respectively, and antibody titers in mouse serum were determined by ELISA. After the third immunization, mice with high antibody titers in serum and titers reaching a plateau were selected for splenocyte fusion. Three days before splenocyte fusion, a booster immunization was performed by intraperitoneal (IP) injection of 50 µg/mouse of antigen solution prepared in physiological saline (1 mg/mL).

### 2. Splenocyte Fusion

Splenocytes were fused with myeloma cells, Sp2/0 cells (^{ATCC®}CRL-8287TM) using an optimized PEG-mediated fusion procedure to obtain hybridoma cells. The fused hybridoma cells were resuspended in complete medium (DMEM medium containing 20% FBS, 1× HAT, 1× OPI) at a density of 0.5-1 ×10⁶/mL, seeded into 96-well plates at 100 µL/well, and incubated at 37°C, 5% CO₂ for 3-4 days. Then, 100 µL/well of HAT complete medium was added, and culture was continued for 3-4 days until pinpoint clones formed. The supernatant was removed, and 200 µL/well of HT complete medium (RPMI-1640 medium containing 20% FBS, 1× HT, and 1× OPI) was added, followed by culture at 37°C, 5% CO₂ for 3 days before ELISA detection.

### 3. Hybridoma Cell Screening

Based on hybridoma cell growth density, the hybridoma culture supernatant was detected by binding ELISA. The supernatant of positive well cells detected by binding ELISA was subjected to cell blocking experiments. Cells from wells positive for both binding and blocking were promptly expanded, cryopreserved for stock, and subjected to two to three subclonings until single-cell clones were obtained.

Each subcloned cell was tested by GDF15 binding ELISA and GFRAL blocking assay. Hybridoma clones were selected through the above experiments, and antibodies were further prepared by serum-free cell culture method, purified according to the purification examples, and used in the test examples.

### 4. Sequencing of Positive Hybridoma Clones

Positive hybridoma clones in logarithmic growth phase were collected, processed, and subjected to first-generation or second-generation sequencing to obtain antibody variable region sequences.

### 5. Beacon Screening

Spleens were collected from immunized mice, and under conditions ensuring optimal cell viability, plasma cells were enriched using the CD138b kit (stemcell, 18957). The enriched cells were loaded onto a Beacon 14K chip; signals for simultaneous binding to human GDF15, binding to murine GDF15, and blocking the interaction between GDF15 and GFRAL were analyzed to obtain target positive clones. After the positive clone cells were exported, antibody variable regions were obtained through single-cell sequencing technology.

### 6. Sequence Analysis

Through hybridoma and beacon screening, after sequencing and variable region sequence analysis, duplicates were removed, and unique sequences were obtained. Specifically, refer to the CDR sequences corresponding to G2-CH1 VH, G2-CH2 VH, G2-CH3 VH, G2-CH5 VH, G2-CH6 VH, G2-CH7 VH, G2-CH8 VH, G2-CH9 VH, G2-CH10 VH, G2-CH12 VH, G2-CH13 VH, G2-CH14 VH, G2-CH16 VH, G2-CH17 VH, G2-CH23 VH, G2-CH1 VL, G2-CH2 VL, G2-CH3 VL, G2-CH5 VL, G2-CH6 VL, G2-CH7 VL, G2-CH8 VL, G2-CH9 VL, G2-CH10 VL, G2-CH12 VL, G2-CH13 VL, G2-CH14 VL, G2-CH16 VL, G2-CH17 VL, and G2-CH23 VL in Table 1.

### II: Sequencing of Murine Antibodies and Construction of Chimeric Antibodies (Anti-GDF15 Neutralizing Antibodies)

Variable region sequences of 15 antibodies were obtained (CDR annotations are shown in Table 1, heavy chain variable regions: SEQ ID NOs: 83 to 97, light chain variable regions: SEQ ID NOs: 102 to 116), and fused with the human IgG1 constant region mutant hIgG1 (LALAGA, -K) (SEQ ID NO: 148) or hIgG1' (LALAGA, -K) (SEQ ID NO: 228). The amino acid sequences of the anti-GDF15 neutralizing antibodies are shown in Table 3.

**Table 3**

| Anti-GDF15 Neutralizing Antibody Name | Amino Acid Sequence of Heavy Chain | Amino Acid Sequence of Light Chain |
|---|---|---|
| G2-CH1 | SEQ ID NO: 150 | SEQ ID NO: 183 |
| G2-CH2 | SEQ ID NO: 151 | SEQ ID NO: 184 |
| G2-CH3 | SEQ ID NO: 152 | SEQ ID NO: 185 |
| G2-CH5 | SEQ ID NO: 153 | SEQ ID NO: 186 |
| G2-CH6 | SEQ ID NO: 154 | SEQ ID NO: 187 |
| G2-CH7 | SEQ ID NO: 155 | SEQ ID NO: 188 |
| G2-CH8 | SEQ ID NO: 156 | SEQ ID NO: 189 |
| G2-CH9 | SEQ ID NO: 157 | SEQ ID NO: 190 |
| G2-CH10 | SEQ ID NO: 158 | SEQ ID NO: 191 |
| G2-CH12 | SEQ ID NO: 159 | SEQ ID NO: 192 |
| G2-CH13 | SEQ ID NO: 160 | SEQ ID NO: 193 |
| G2-CH14 | SEQ ID NO: 161 | SEQ ID NO: 194 |
| G2-CH16 | SEQ ID NO: 162 | SEQ ID NO: 195 |
| G2-CH17 | SEQ ID NO: 163 | SEQ ID NO: 196 |
| G2-CH23 | SEQ ID NO: 164 | SEQ ID NO: 197 |

### III. Expression and Purification of Anti-GDF15 Neutralizing Antibodies

The coding sequences of the antibodies (i.e., the nucleotide sequences encoding the amino acid sequences in Table 3) were cloned into the expression vector pCDNA3.4. Plasmids were extracted from the clones confirmed correct by sequencing, and transfection expression was performed; ExpiCHO-S cells were co-transfected with the expression vectors at a heavy chain to light chain plasmid ratio of 1:2. The cells were adjusted to a density of 6×10⁶ cells/mL, and transfection was performed at a ratio of 1 µg plasmid transfecting 1 mL of cells; after transfection, cells were cultured at 37°C, 5% CO₂ for 5-7 days. The supernatant was collected by centrifugation and filtered through a 0.22 µm filter membrane; the filtered cell culture medium was loaded onto a Protein A chromatography column equilibrated with PBS. After loading, the column was washed with PBS, and impurities were eluted using 100 mM citrate buffer at pH 5.0. Antibody elution was achieved with 100 mM citrate buffer at pH 3.0, and the antibody sample was immediately neutralized with 1 M Tris-HCl buffer at pH 9.0; a portion of the antibody sample was taken for subsequent protein purity analysis, such as SDS-PAGE and SEC-HPLC. The results showed that the 15 anti-GDF15 neutralizing antibodies were successfully purified.

### Test Example 1: Activity and Function Detection of Anti-GDF15 Neutralizing Antibodies

### 1. Determination of Binding Activity and Species Selectivity of Anti-GDF15 Neutralizing Antibodies

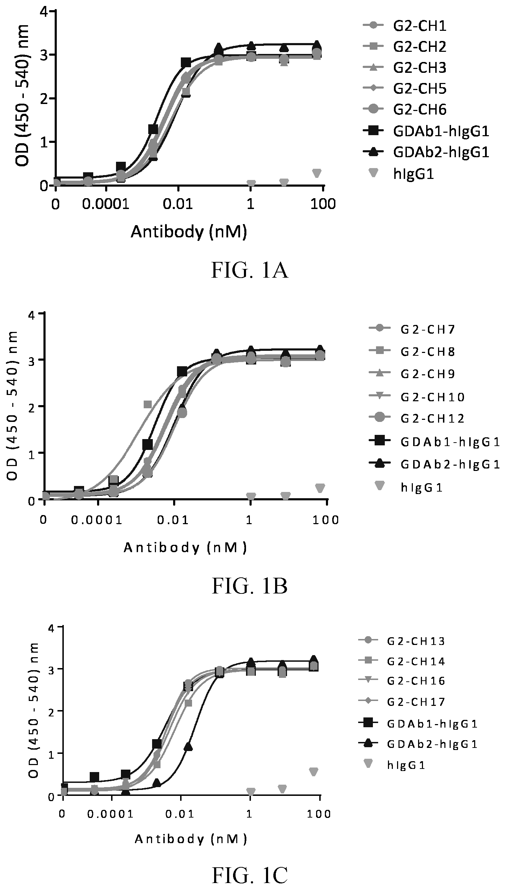

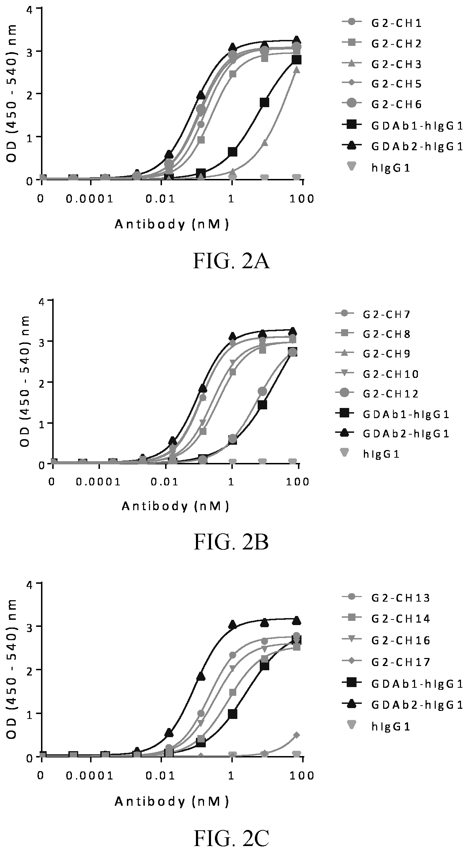

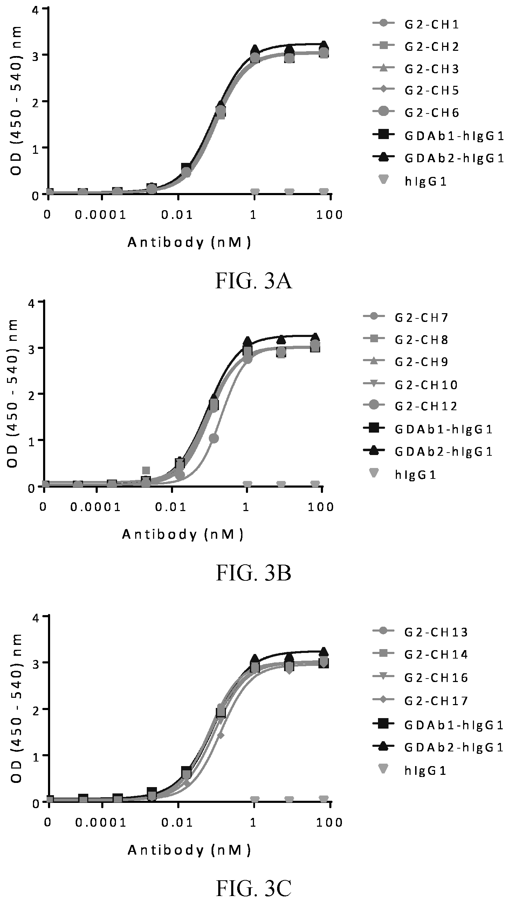

To confirm the species specificity of the candidate molecules and to select appropriate animal models for subsequent studies, the inventors conducted species binding tests on the anti-GDF15 neutralizing antibodies prepared in Example 1 (hereinafter referred to as candidate antibody molecules). The specific test steps were as follows:
The binding activity of 14 anti-GDF15 neutralizing antibodies to GDF15 proteins from three species, human, mouse, and cynomolgus monkey, was determined by ELISA. The specific detection steps were:
96-well microtiter plates were coated with the antigen molecule hGDF-15.His, mGDF-15.hFc, or cynoGDF-15.hFc at 2 µg/mL and 100 µL/well, overnight at 4°C; the next day, after washing and blocking the plates, serially diluted candidate antibody molecules were added and incubated for 1 hour at room temperature; after washing the plates, diluted anti-hFc-HRP or anti-hFab-HRP was added and incubated for 1 hour at room temperature; subsequently, the plates were washed, developed, and read using a microplate reader. The detection results are shown in Figure 1A, Figure 1B, Figure 1B, Figure 2A, Figure 2B, Figure 2C, Figure 3A, Figure 3B, Figure 3C and Table 4. The results showed that the EC₅₀ (KD) values of the 14 anti-GDF15 neutralizing antibodies for binding to human GDF15 recombinant protein (ACRO, GD5-H5149) were all <10 pM. Except for G2-CH3, G2-CH12, and G2-CH17, the EC₅₀ (KD) values for binding to mouse GDF15 recombinant protein (Kactus, GDF-MM215) were all below 0.5 nM. The EC₅₀ (KD) values for binding to cynomolgus monkey GDF15 recombinant protein (Kactus, GDF-CM215) were relatively similar, around 100 pM. The binding activities were comparable to those of the two comparative antibodies, GDAb1 (from AVEO company, WO2014100689A1) and GDAb2 (Ponsegromab from Pfizer).

**Table 4**

| Anti-GDF15 Neutralizing Antibody Name | humanGDF15 (EC₅₀, pM) | mouseGDF15 (EC₅₀, nM) | cynoGDF15 (EC₅₀, pM) |
|---|---|---|---|
| G2-CH1 | 3.87 | 0.17 | 85.98 |
| G2-CH2 | 6.27 | 0.26 | 97.98 |
| G2-CH3 | 4.53 | 56.50 | 101.80 |
| G2-CH5 | 3.94 | 0.12 | 92.92 |
| G2-CH6 | 4.34 | 0.11 | 89.21 |
| G2-CH7 | 5.52 | 0.12 | 104.50 |
| G2-CH8 | 1.22 | 0.37 | 93.35 |
| G2-CH9 | 6.34 | 0.11 | 97.01 |
| G2-CH10 | 5.92 | 0.25 | 84.11 |
| G2-CH12 | 10.58 | 5.64 | 210.80 |
| G2-CH13 | 3.82 | 0.22 | 65.36 |
| G2-CH14 | 6.63 | 0.76 | 72.12 |
| G2-CH16 | 4.76 | 0.32 | 90.93 |
| G2-CH17 | 3.93 | NA | 129.70 |
| GDAb1 | 2.68 | 6.29 | 87.45 |
| GDAb2 | 8.55 | 0.081 | 85.81 |

| | | | |
|---|---|---|---|
| Note: NA indicates not detected. | | | |

>GDAb1-VH: 
EVLLQQSGPELVKPGASVKIPCKASGYTFTDYNMDWVKQSHGKSLEWIGQINPNN
GGIFFNQKFKGKATLTVDKSSNTAFMEVRSLTSEDTAVYYCAREAITTVGAMDYWGQG
TSVTVSS (SEQ ID NO: 218);
>GDAb1-VL: 
DIQMTQSPASLSASVGETVTITCRTSENLHNYLAWYQQKQGKSPQLLVYDAKTLA
DGVPSRFSGSGSGTQYSLKINSLQPEDFGSYYCQHFWSSPYTFGGGTKLEIK (SEQ ID
NO: 219);
> GDAb2-VH: 
QVQLVQSGAEVKKPGSSVKVSCKASGYTFSSYNIDWVRQAPGQGLEWMGGINPIF
GTAFYNQKFQGRVTITADESTSTAYMELSSLRSEDTAVYYCAREAITTVGAMDHWGQG
TLVTVSS (SEQ ID NO: 220);
> GDAb2-VL:

EIVLTQSPATLSLSPGERATLSCRTSQSVHNYLAWYQQKPGQAPRLLIYDASTRADG
IPARFSGSGSGTDFTLTISSLEPEDFAVYYCQQFWSWPWTFGQGTKVEIK (SEQ ID NO:
221).

### 2. Receptor Blocking Activity of Anti-GDF15 Neutralizing Antibodies Using Reporter Gene (mGFRAL)

The receptor GFRAL blocking activity of the neutralizing antibodies was detected using a reporter gene cell strain stably transfected with the mouse GDF15 receptor mouse GFRAL and the co-receptor RET. The reporter gene cell strain used in this example was based on HEK293 cells co-transfected with the GDF15 receptor GFRAL and the co-receptor RET. The activation of the GDF15 pathway was reflected by the expression of the downstream reporter gene luciferase.

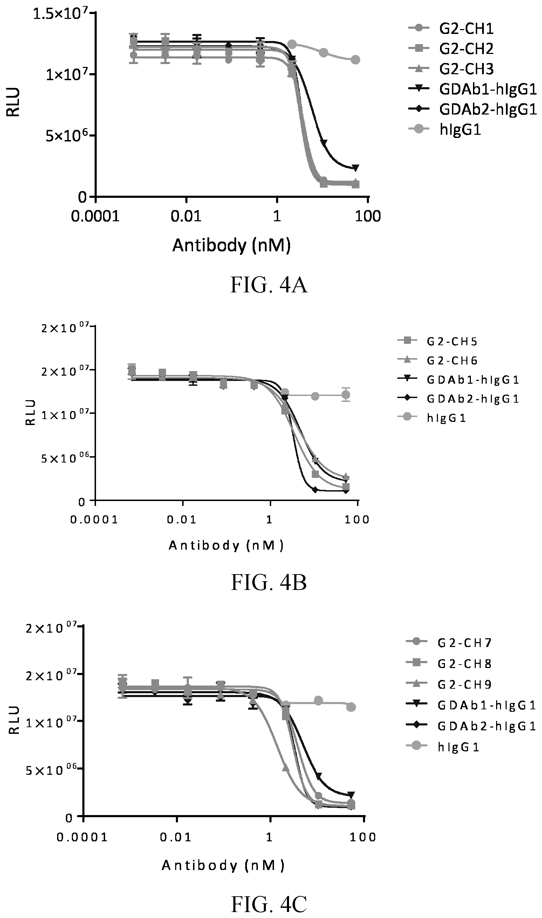

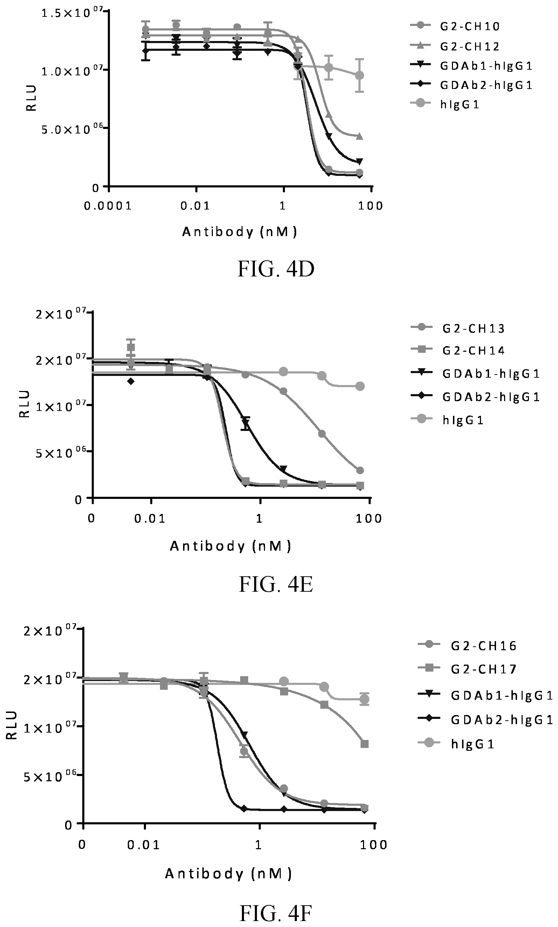

On the first day of the experiment, cells at 70%-80% confluence were collected, digested with trypsin EDTA (0.25%), and digestion was terminated using assay buffer (DMEM + 1% FBS). Cells were plated at 1.3×10⁴ cells/well and 50 µL/well, and incubated overnight at 37°C. On the second day, antibodies and hGDF15 (ACRO, GD5-H5149) were diluted with assay buffer. Different concentrations of antibodies were mixed with GDF15 at a volume ratio of 1:1, then added to the cell plate at 100 µL/well, and incubated at 37°C for 6 hours (pre-incubated at room temperature for 0.5 hour). After 6 hours, luciferase detection reagent (Promega, G7940) was added at 50 µL/well. The results are shown in Figure 4A, Figure 4B, Figure 4C, Figure 4D, Figure 4E, Figure 4F, and Table 5. The results showed that multiple antibodies, including G2-CH1, G2-CH2, G2-CH3, G2-CH5, G2-CH6, G2-CH7, G2-CH8, G2-CH9, G2-CH10, G2-CH14, and G2-CH16, exhibited neutralizing activity comparable to GDAb2 and better than GDAb1.

**Table 5**

| Anti-GDF15 Neutralizing Antibody Name | IC₅₀ (nM) | Anti-GDF15 Neutralizing Antibody Name | IC₅₀ (nM) |
|---|---|---|---|
| G2-CH1 | 3.75 | G2-CH10 | 3.40 |
| G2-CH2 | 3.24 | G2-CH12 | 6.80 |
| G2-CH3 | 3.55 | G2-CH13 | 11.68 |
| G2-CH5 | 3.45 | G2-CH14 | 0.20 |
| G2-CH6 | 4.39 | G2-CH16 | 0.45 |
| G2-CH7 | 3.83 | G2-CH17 | NA |
| G2-CH8 | 3.05 | GDAb1 | 5.75 |
| G2-CH9 | 1.42 | GDAb2 | 3.46 |

| | | | |
|---|---|---|---|
| Note: NA indicates not detected. | | | |

### 3. Detection of the Neutralization Effect of Anti-GDF15 Neutralizing Antibodies on GDF15-Induced Foxp3 Expression Levels in Jurkat Cells

Based on previous research findings, GDF15 can induce Foxp3 expression in Jurkat cells within 7 days. The neutralization effect of the screened anti-GDF15 neutralizing antibodies on GDF15-induced Foxp3 expression levels in Jurkat cells was detected by QPCR.

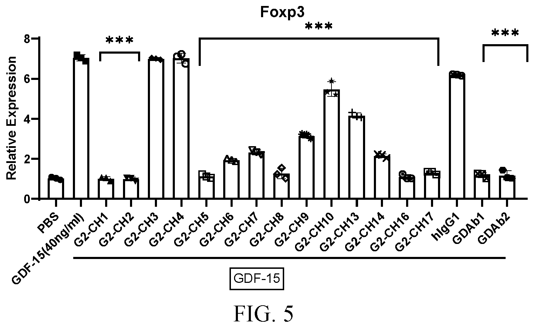

On the first day of the experiment, 12-well plates were coated with anti-CD3 and anti-CD28 antibodies at 2 µg/mL and 500 µL/well for each, overnight at 4°C. On the second day, after washing with PBS, 1×10⁶ cells were plated per well, and GDF15 (ACRO, GD5-H5149, 40 ng/mL) and the anti-GDF15 neutralizing antibodies (refer to "antibodies", 10 µg/mL) were added simultaneously. The cells were treated for 7 days, with the medium changed every 2 days. On day 7, cDNA was synthesized using a reverse transcription kit (Invitrogen 11750150), and Foxp3 expression in Jurkat cells was detected by QPCR (Vazyme Q121-03). The results are shown in Figure 5. The QPCR results showed that multiple antibodies inhibited Foxp3 expression, among which G2-CH1, G2-CH2, G2-CH5, G2-CH8, G2-CH16, and G2-CH17 molecules exhibited inhibitory activity similar to the comparative antibodies GDAb1 and GDAb2.

### Example 2: Modification of PTM Sites in Anti-GDF15 Neutralizing Antibodies

1. Research has found that during antibody production, various physicochemical factors can easily lead to the generation of variants of post-translational modifications (PTMs), such as glycosylation, oxidation, glycation, deamidation, isomerization, terminal cyclization, and others; some PTM sites typically exhibit potential modifications in the primary structure of the antibody. When PTMs occur in antibodies, they usually cause antibody heterogeneity, and when PTMs occur in the CDR regions of the antibody, they may lead to loss of antibody activity. In antibody development, to avoid the occurrence of PTMs, conservative amino acid substitutions are usually performed at potential risk sites.
Upon sequence analysis, the inventors found that the light chain CDR3 of candidate antibody G2-CH2 contained an NS deamidation risk site, the heavy chain CDR2 of G2-CH5 contained an NG deamidation risk site, and the light chain CDR1 of G2-CH8 contained an NG deamidation risk site. Therefore, the inventors modified the above CDRs of the antibodies by conservative amino acid substitution. The modified sequences of the light chain CDR3 of G2-CH2 are shown in the amino acid sequences as set forth in SEQ ID NO: 80, SEQ ID NO: 81, and SEQ ID NO: 82 in Table 1. The modified sequences of the heavy chain CDR2 of G2-CH5 are shown in the amino acid sequences as set forth in SEQ ID NO: 74, SEQ ID NO: 75, and SEQ ID NO: 76 in Table 1. The modified sequences of the light chain CDR1 of G2-CH8 are shown in the amino acid sequences as set forth in SEQ ID NO: 77, SEQ ID NO: 78, and SEQ ID NO: 79 in Table 1.
2. The anti-GDF15 neutralizing antibodies with modified PTM sites were constructed according to step 2 of Example 1, and the expression and purification of the anti-GDF15 neutralizing antibodies with modified PTM sites were performed according to step 3 of Example 1. The heavy chain variable regions and light chain variable regions, the amino acid sequences of the heavy chains, the amino acid sequences of the light chains, the nucleotide sequences of the heavy chains, and the nucleotide sequences of the light chains of the anti-GDF15 neutralizing antibodies with modified PTM sites are shown in Table 6.

**Table 6**

| Name of Anti-GDF15 Neutralizing Antibody with PTM Modification | Amino Acid Sequence of Heavy Chain Variable Region | Amino Acid Sequence of Light Chain Variable Region | Amino Acid Sequence of Heavy Chain | Amino Acid Sequence of Light Chain |
|---|---|---|---|---|
| G2-CH2-LP1-hIgG1 (LALAGA, -K) | SEQ ID NO: 84 | SEQ ID NO: 120 | SEQ ID NO: 170 | SEQ ID NO: 203 |
| G2-CH2-LP2-hIgG1 (LALAGA, -K) | SEQ ID NO: 84 | SEQ ID NO: 121 | SEQ ID NO: 170 | SEQ ID NO: 204 |
| G2-CH2-LP3-hIgG1 (LALAGA, -K) | SEQ ID NO: 84 | SEQ ID NO: 122 | SEQ ID NO: 170 | SEQ ID NO: 205 |
| G2-CH5-HP1-hIgG1 (LALAGA, -K) | SEQ ID NO: 98 | SEQ ID NO: 105 | SEQ ID NO: 166 | SEQ ID NO: 199 |
| G2-CH5-HP2-hIgG1 (LALAGA, -K) | SEQ ID NO: 99 | SEQ ID NO: 105 | SEQ ID NO: 167 | SEQ ID NO: 199 |
| G2-CH5-HP3-hIgG1 (LALAGA, -K) | SEQ ID NO: 100 | SEQ ID NO: 105 | SEQ ID NO: 168 | SEQ ID NO: 199 |
| G2-CH8-LP1-hIgG1 (LALAGA, -K) | SEQ ID NO: 89 | SEQ ID NO: 117 | SEQ ID NO: 169 | SEQ ID NO: 200 |
| G2-CH8-LP2-hIgG1 (LALAGA, -K) | SEQ ID NO: 89 | SEQ ID NO: 118 | SEQ ID NO: 169 | SEQ ID NO: 201 |
| G2-CH8-LP3-hIgG1 (LALAGA, -K) | SEQ ID NO: 89 | SEQ ID NO: 119 | SEQ ID NO: 169 | SEQ ID NO: 202 |

### Test Example 2: Activity Detection and Function Detection of Anti-GDF15 Neutralizing Antibodies after PTM Modification

### 1. Detection of Receptor Blocking Activity of Anti-GDF15 Neutralizing Antibodies by Reporter Gene Assay

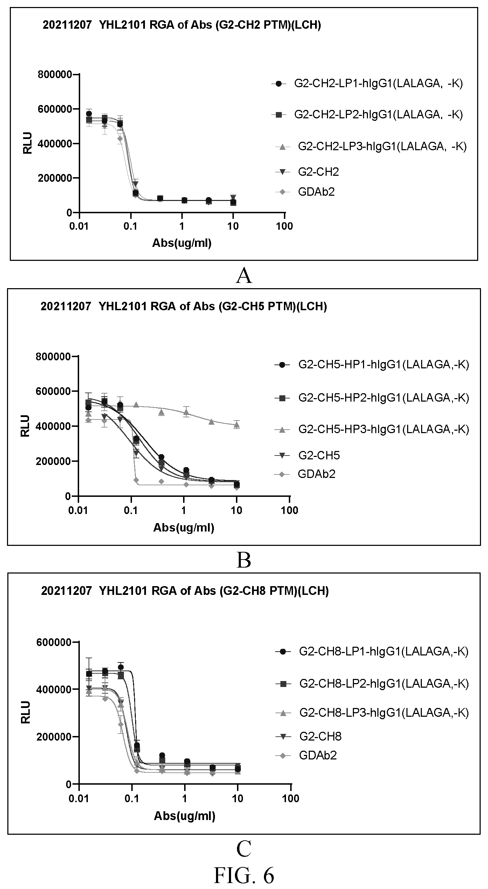

The reporter gene cell strain (mGFRAL) was used to detect the receptor blocking activity against GDF15 of the 9 anti-GDF15 neutralizing antibodies with PTM modification prepared in Example 2. The detection method referred to step 2 in Test Example 1. The detection results are shown in Figure 6 and Table 7. The results showed that after PTM modification, except for G2-CH5-HP3, the remaining 8 molecules still maintained receptor blocking activity comparable to the comparative antibody.

**Table 7**

| Name of Anti-GDF15 Neutralizing Antibody with PTM Modification | IC₅₀ (ng/mL) | Name of Anti-GDF15 Neutralizing Antibody with PTM Modification | IC₅₀ (ng/mL) |
|---|---|---|---|
| G2-CH2-LP1-hIgG1 (LALAGA, -K) | 88 | G2-CH5-HP3-hIgG1 (LALAGA, -K) | / |
| G2-CH2-LP2-hIgG1 (LALAGA, -K) | 91 | G2-CH8-LP1-hIgG1 (LALAGA, -K) | 116 |
| G2-CH2-LP3-hIgG1 (LALAGA, -K) | 93 | G2-CH8-LP2-hIgG1 (LALAGA, -K) | 100 |
| G2-CH5-HP1-hIgG1 (LALAGA, -K) | 180 | G2-CH8-LP3-hIgG1 (LALAGA, -K) | 81 |
| G2-CH5-HP2-hIgG1 (LALAGA, -K) | 142 | GDAb2 | 67 |

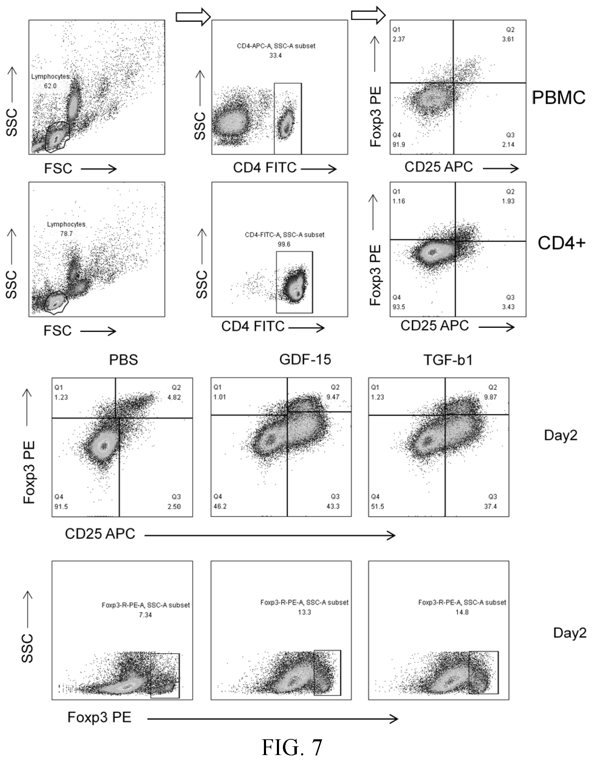

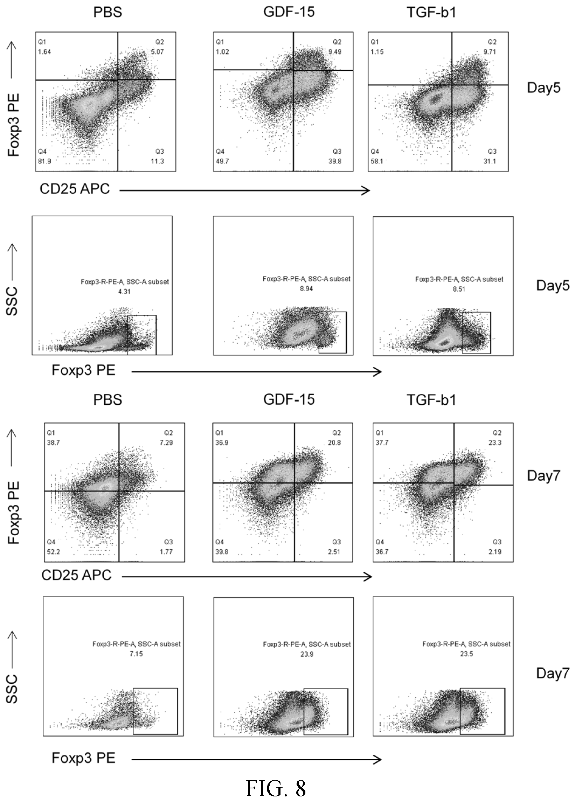

2. Based on previous research findings, GDF15 can induce CD4+ T cells to differentiate into Tregs (with high expression of Foxp3 and CD25) in vitro. As shown in Figures 7 and 8, in this example, after one week of Treg differentiation induced by GDF15, the proportion of Tregs reached a level close to that of the positive control TGFβ, exceeding 20%. After co-incubation with the candidate antibody molecules and GDF15, the proportion of Tregs induced by GDF15 could be significantly blocked, suggesting that the candidate molecules may play a role in tumor immunity. Therefore, detecting this indicator could predict that the GDF15 antibody also possesses tumor immune activity.

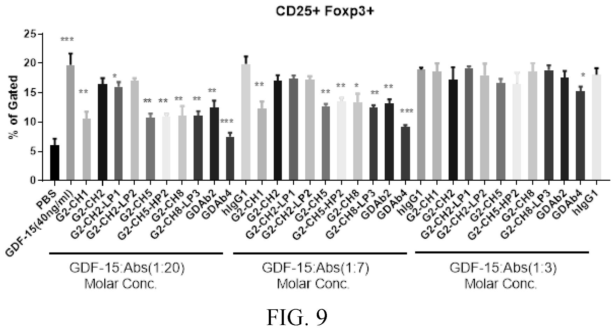

Subsequently, the inventors used flow cytometry to detect the neutralizing activity against GDF15 of G2-CH1, G2-CH2, G2-CH5, G2-CH8, and their antibodies after PTM modification. The specific experimental steps were as follows:
On the first day of the experiment, 96-well plates were coated with anti-CD3 (Invitrogen 16-0037-85) and anti-CD28 (Invitrogen 16-0289-85) antibodies at 2 µg/mL and 500 µL/well for each, overnight at 4°C. On the second day, CD4+ cells were sorted from PBMCs using CD4 beads (Miltenyi Biotec MB17-R0024), and then the sorted cells were resuspended in 1640 medium supplemented with IL-2 (Acro IL2-H4113). After washing the coated 96-well plates with PBS, cells were plated at 2×10⁵ cells/well, and simultaneously treated with each antibody and GDF15/PBS (at a final concentration of 40 ng/mL) for 7 days, with medium changed every 2 days. After 7 days, cells were collected, stained, fixed, and permeabilized. Finally, the proportions of Foxp3+ and CD25+ cells among CD4 T cells were detected by flow cytometry to determine the proportion of induced Treg differentiation, as specifically shown in Figure 9. As shown by flow cytometry, the abscissa represents each treatment group, and the ordinate represents the proportion of gated Tregs (%). Whether at a GDF15:antibody molar ratio of 1:20 or 1:7, G2-CH1 and the PTM mutants G2-CH5-HP2-hIgG1 (LALAGA, -K) and G2-CH8-LP3-hIgG1 (LALAGA, -K) could significantly downregulate the proportion of Treg differentiation induced by GDF15.

### Example 3: Humanization of Antibodies

Humanization methods are usually completed by framework grafting and humanization of amino acids on protein surfaces. The humanization experimental steps were as follows:
By comparing the IMGT human antibody heavy and light chain variable region germline gene database and using molecular modeling software, heavy and light chain variable region germline genes with high homology to the parental antibody were selected as templates. The CDRs of the murine antibody were grafted into the corresponding human templates, respectively, and corresponding back mutations were made according to the modeled structures, as specifically shown in Tables 8 and 9. Among them, G2-CH1 used IGHV4-39*01, IGHV3-23*01, and IGKV1-39*01, IGKV3-15*01 as templates; G2-CH5-HP2-hIgG1 (LALAGA, -K) used IGHV1-8*01 and IGKV1-33*04 as templates; G2-CH8-LP3-hIgG1 (LALAGA, -K) used IGHV2-70*06, IGHV2-5*01, and IGKV2-29*02, IGKV2D-29*02 as templates. Variable region sequences in the order FR1-CDR1-FR2-CDR2-FR3-CDR3-FR4 were formed, and appropriate back mutations were made to the CDRs. For example, G2-H8-H1L1-hIgG1(LALAGA,-K)(gsm) is a humanized antibody obtained after back mutation of the CDRs. Amino acid residues were determined and annotated by the Kabat numbering system.

The light and heavy chain variable region sequences and light and heavy chain sequences of the humanized antibodies are shown in Tables 10-11. Subsequently, the humanized anti-GDF15 neutralizing antibodies were constructed according to step 2 in Example 1, and the expression and purification of the humanized anti-GDF15 neutralizing antibodies were performed according to step 3 in Example 1. The purification results are shown in Tables 12-13.

**Table 8**

| G2-H1-VH | | |
|---|---|---|
| IGHV4-39*01 | G2-H1-VH_1(4-39) Not expressed | Grafted |
| | G2-H1-VH_2(4-39) | L2V, V24F, G51A, V69L, V73K, F80V (specific amino acid sequence see SEQ ID NO: 124) |
| | G2-H1-VH_3(4-39) | L2V, L3T, T23S, V24F, I50L, G51A, V69L, V73K, F80V, V94T, L113S (specific amino acid sequence see SEQ ID NO: 125) |
| IGHV3-23*01 | G2-H1-VH_1(3-23) Not expressed | Grafted |
| | G2-H1-VH_2(3-23) | A24F, V39I, S51A, F69L, R73K, N75T, L80V (specific amino acid sequence see SEQ ID NO: 126) |
| | G2-H1-VH_3(3-23) | E1Q, Q3T, A23S, A24F, V39I, V50L, S51A, F69L, R73K, N75T, L80V, L87V, K99R (specific amino acid sequence see SEQ ID NO: 127) |

| G2-H5-VH | | |
|---|---|---|
| IGHV1-8*01 | G2-H5-VH_1 | Grafted (specific amino acid sequence see SEQ ID NO: 132) |
| | G2-H5-VH_2 | T74K (specific amino acid sequence see SEQ ID NO: 133) |
| | G2-H5-VH_3 | M48I, V68A, M70L, T74K (specific amino acid sequence see SEQ ID NO: 134) |
| | G2-H5-VH_4 | F29L, M48I, V68A, M70L, D73N, T74K (specific amino acid sequence |
| | | see SEQ ID NO: 135) |

| G2-H8-VH | | |
|---|---|---|
| IGHV2-70*06 | G2-H8-VH_1 | Grafted (specific amino acid sequence see SEQ ID NO: 140) |
| | G2-H8-VH_2 | A46G (specific amino acid sequence see SEQ ID NO: 141) |
| IGHV2-5*01 | G2-H8-VH1(gsm) | Grafted (specific amino acid sequence see SEQ ID NO: 144) |
| | G2-H8-VH2(gsm) | T10A, V81F (specific amino acid sequence see SEQ ID NO: 145) |

**Table 9**

| G2-H1-VL | | |
|---|---|---|
| IGKV1-39*01 | G2-H1-VL_1 (1-39) Not expressed | Grafted |
| | G2-H1-VL_2 (1-39) | A13T, L46F, S60D, L73F, T85V (specific amino acid sequence see SEQ ID NO: 128) |
| | G2-H1-VL_3 (1-39) | Q3V, A13T, A43S, L46F, S60D, L73F, L78V, T85V, Q100G (specific amino acid sequence see SEQ ID NO: 129) |
| IGKV3-15*01 | G2-H1-VL_1(3-15) Not expressed | Grafted |
| | G2-H1-VL_2(3-15) | E1D, V13T, L46F, I57V, L73F (specific amino acid sequence see SEQ ID NO: 130) |
| | G2-H1-VL_3(3-15) | E1D, V13T, A19V, L21I, A43S, L46F, I58V, A60D, E70D, L73F, L78V (specific amino acid sequence see SEQ ID NO: 131) |

| G2-H5-VL | | |
|---|---|---|
| IGKV1-33*04 | G2-H5-VL_1 | Grafted (specific amino acid sequence see SEQ ID NO: 136) |
| | G2-H5-VL_2 | F71Y (specific amino acid sequence see SEQ ID NO: 137) |
| | G2-H5-VL_3 | Y49S, F71Y(specific amino acid sequence see SEQ ID NO: 138) |
| | G2-H5-VL_4 | Y49S, V57I, F71Y (specific amino acid sequence see SEQ ID NO: 139) |

| G2-H8-VL | | |
|---|---|---|
| IGKV2-29*02 | G2-H8-VL_1 | Grafted (specific amino acid sequence see SEQ ID NO: 142) |
| | G2-H8-VL_2 | G69S (specific amino acid sequence see SEQ ID NO: 143) |
| IGKV2D-29*02 | G2-H8-VL1(gsm) | Grafted (specific amino acid sequence see SEQ ID NO: 146) |
| | G2-H8-VL2(gsm) | T7S (specific amino acid sequence see SEQ ID NO: 147) |

**Table 10**

| Antibody Name | Heavy Chain Variable Region Name | Amino Acid Sequence of Heavy Chain Variable Region | Light Chain Variable Region Name | Amino Acid Sequence of Light Chain Variable Region |
|---|---|---|---|---|
| G2-H1-hz1-hIgG1(LALAGA, -K) | G2-H1-VH_2(4-39) | SEQ ID NO: 124 | G2-H1-VL_2 (1-39) | SEQ ID NO: 128 |
| G2-H1-hz2-hIgG1(LALAGA, -K) | G2-H1-VH_2(4-39) | SEQ ID NO: 124 | G2-H1-VL_3 (1-39) | SEQ ID NO: 129 |
| G2-H1-hz3-hIgG1(LALAGA, -K) | G2-H1-VH_2(4-39) | SEQ ID NO: 124 | G2-H1-VL_2(3-15) | SEQ ID NO: 130 |
| G2-H1-hz4-hIgG1(LALAGA, -K) | G2-H1-VH_2(4-39) | SEQ ID NO: 124 | G2-H1-VL_3(3-15) | SEQ ID NO: 131 |
| G2-H1-hz5-hIgG1(LALAGA, -K) | G2-H1-VH_3(4-39) | SEQ ID NO: 125 | G2-H1-VL_2 (1-39) | SEQ ID NO: 128 |
| G2-H1-hz6-hIgG1(LALAGA, -K) | G2-H1-VH_3(4-39) | SEQ ID NO: 125 | G2-H1-VL_3 (1-39) | SEQ ID NO: 129 |
| G2-H1-hz7-hIgG1(LALAGA, -K) | G2-H1-VH_3(4-39) | SEQ ID NO: 125 | G2-H1-VL_2(3-15) | SEQ ID NO: 130 |
| G2-H1-hz8-hIgG1(LALAGA, -K) | G2-H1-VH_3(4-39) | SEQ ID NO: 125 | G2-H1-VL_3(3-15) | SEQ ID NO: 131 |
| G2-H1-hz9-hIgG1(LALAGA, -K) | G2-H1-VH_2(3-23) | SEQ ID NO: 126 | G2-H1-VL_2 (1-39) | SEQ ID NO: 128 |
| G2-H1-hz10-hIgG1(LALAGA, -K) | G2-H1-VH_2(3-23) | SEQ ID NO: 126 | G2-H1-VL_3 (1-39) | SEQ ID NO: 129 |
| G2-H1-hz11-hIgG1(LALAGA, -K) | G2-H1-VH_2(3-23) | SEQ ID NO: 126 | G2-H1-VL_2(3-15) | SEQ ID NO: 130 |
| G2-H1-hz12-hIgG1(LALAGA, -K) | G2-H1-VH_2(3-23) | SEQ ID NO: 126 | G2-H1-VL_3(3-15) | SEQ ID NO: 131 |
| G2-H1-hz13-hIgG1(LALAGA, -K) | G2-H1-VH_3(3-23) | SEQ ID NO: 127 | G2-H1-VL_2 (1-39) | SEQ ID NO: 128 |
| G2-H1-hz14-hIgG1(LALAGA, -K) | G2-H1-VH_3(3-23) | SEQ ID NO: 127 | G2-H1-VL_3 (1-39) | SEQ ID NO: 129 |
| G2-H1-hz15-hIgG1(LALAGA, -K) | G2-H1-VH_3(3-23) | SEQ ID NO: 127 | G2-H1-VL_2(3-15) | SEQ ID NO: 130 |
| G2-H1-hz16-hIgG1(LALAGA, -K) | G2-H1-VH_3(3-23) | SEQ ID NO: 127 | G2-H1-VL_3(3-15) | SEQ ID NO: 131 |
| G2-H5-H1.2L1-hIgG1(LALAGA,-K) | G2-H5-VH_1 | SEQ ID NO: 132 | G2-H5-VL_1 | SEQ ID NO: 136 |
| G2-H5-H2.2L1-hIgG1(LALAGA, -K) | G2-H5-VH_2 | SEQ ID NO: 133 | G2-H5-VL_1 | SEQ ID NO: 136 |
| G2-H5-H3.2L1-hIgG1(LALAGA,-K) | G2-H5-VH_3 | SEQ ID NO: 134 | G2-H5-VL_1 | SEQ ID NO: 136 |
| G2-H5-H4.2L1-hIgG1(LALAGA,-K) | G2-H5-VH_4 | SEQ ID NO: 135 | G2-H5-VL_1 | SEQ ID NO: 136 |
| G2-H5-H1.2L2-hIgG1(LALAGA,-K) | G2-H5-VH_1 | SEQ ID NO: 132 | G2-H5-VL_2 | SEQ ID NO: 137 |
| G2-H5-H2.2L2-hIgG1(LALAGA,-K) | G2-H5-VH_2 | SEQ ID NO: 133 | G2-H5-VL_2 | SEQ ID NO: 137 |
| G2-H5-H3.2L2-hIgG1(LALAGA,-K) | G2-H5-VH_3 | SEQ ID NO: 134 | G2-H5-VL_2 | SEQ ID NO: 137 |
| G2-H5-H4.2L2-hIgG1(LALAGA,-K) | G2-H5-VH_4 | SEQ ID NO: 135 | G2-H5-VL_2 | SEQ ID NO: 137 |
| G2-H5-H1.2L3-hIgG1(LALAGA,-K) | G2-H5-VH_1 | SEQ ID NO: 132 | G2-H5-VL_3 | SEQ ID NO: 138 |
| G2-H5-H2.2L3-hIgG1(LALAGA,-K) | G2-H5-VH_2 | SEQ ID NO: 133 | G2-H5-VL_3 | SEQ ID NO: 138 |
| G2-H5-H3.2L3-hIgG1(LALAGA,-K) | G2-H5-VH_3 | SEQ ID NO: 134 | G2-H5-VL_3 | SEQ ID NO: 138 |
| G2-H5-H4.2L3-hIgG1(LALAGA,-K) | G2-H5-VH_4 | SEQ ID NO: 135 | G2-H5-VL_3 | SEQ ID NO: 138 |
| G2-H5-H1.2L4-hIgG1(LALAGA,-K) | G2-H5-VH_1 | SEQ ID NO: 132 | G2-H5-VL_4 | SEQ ID NO: 139 |
| G2-H5-H2.2L4-hIgG1(LALAGA,-K) | G2-H5-VH_2 | SEQ ID NO: 133 | G2-H5-VL_4 | SEQ ID NO: 139 |
| G2-H5-H3.2L4-hIgG1(LALAGA,-K) | G2-H5-VH_3 | SEQ ID NO: 134 | G2-H5-VL_4 | SEQ ID NO: 139 |
| G2-H5-H4.2L4-hIgG1(LALAGA,-K) | G2-H5-VH_4 | SEQ ID NO: 135 | G2-H5-VL_4 | SEQ ID NO: 139 |
| G2-H8-H1L1-hIgG1(LALAGA,-K) | G2-H8-VH_1 | SEQ ID NO: 140 | G2-H8-VL_1 | SEQ ID NO: 142 |
| G2-H8-H2L1-hIgG1(LALAGA,-K) | G2-H8-VH_2 | SEQ ID NO: 141 | G2-H8-VL_1 | SEQ ID NO: 142 |
| G2-H8-H1L2-hIgG1(LALAGA,-K) | G2-H8-VH_1 | SEQ ID NO: 140 | G2-H8-VL_2 | SEQ ID NO: 143 |
| G2-H8-H2L2-hIgG1(LALAGA,-K) | G2-H8-VH_2 | SEQ ID NO: 141 | G2-H8-VL_2 | SEQ ID NO: 143 |
| G2-H8-H1L1-hIgG1(LALAGA,-K)(gsm) | G2-H8-VH1(gsm) | SEQ ID NO: 144 | G2-H8-VL1(gsm) | SEQ ID NO: 146 |
| G2-H8-H1L2-hIgG1(LALAGA,-K)(gsm) | G2-H8-VH1(gsm) | SEQ ID NO: 144 | G2-H8-VL2(gsm) | SEQ ID NO: 147 |
| G2-H8-H2L1-hIgG1(LALAGA,-K)(gsm) | G2-H8-VH2(gsm) | SEQ ID NO: 145 | G2-H8-VL1(gsm) | SEQ ID NO: 146 |
| G2-H8-H2L2-hIgG1(LALAGA,-K)(gsm) | G2-H8-VH2(gsm) | SEQ ID NO: 145 | G2-H8-VL2(gsm) | SEQ ID NO: 147 |
| G2-H8-H1L1-hIgG1'(LALAGA,-K)(gsm) | G2-H8-VH1(gsm) | SEQ ID NO: 144 | G2-H8-VL1(gsm) | SEQ ID NO: 146 |

**Table 11**

| Antibody Name | Amino Acid Sequence of Heavy Chain | Amino Acid Sequence of Light Chain |
|---|---|---|
| G2-H1-hz1-hIgG1(LALAGA, -K) | SEQ ID NO: 171 | SEQ ID NO: 206 |
| G2-H1-hz2-hIgG1(LALAGA, -K) | SEQ ID NO: 171 | SEQ ID NO: 207 |
| G2-H1-hz3-hIgG1(LALAGA, -K) | SEQ ID NO: 171 | SEQ ID NO: 208 |
| G2-H1-hz4-hIgG1(LALAGA, -K) | SEQ ID NO: 171 | SEQ ID NO: 209 |
| G2-H1-hz5-hIgG1(LALAGA, -K) | SEQ ID NO: 172 | SEQ ID NO: 206 |
| G2-H1-hz6-hIgG1(LALAGA, -K) | SEQ ID NO: 172 | SEQ ID NO: 207 |
| G2-H1-hz7-hIgG1(LALAGA, -K) | SEQ ID NO: 172 | SEQ ID NO: 208 |
| G2-H1-hz8-hIgG1(LALAGA, -K) | SEQ ID NO: 172 | SEQ ID NO: 209 |
| G2-H1-hz9-hIgG1(LALAGA, -K) | SEQ ID NO: 173 | SEQ ID NO: 206 |
| G2-H1-hz10-hIgG1(LALAGA, -K) | SEQ ID NO: 173 | SEQ ID NO: 207 |
| G2-H1-hz11-hIgG1(LALAGA, -K) | SEQ ID NO: 173 | SEQ ID NO: 208 |
| G2-H1-hz12-hIgG1(LALAGA, -K) | SEQ ID NO: 173 | SEQ ID NO: 209 |
| G2-H1-hz13-hIgG1(LALAGA, -K) | SEQ ID NO: 174 | SEQ ID NO: 206 |
| G2-H1-hz14-hIgG1(LALAGA, -K) | SEQ ID NO: 174 | SEQ ID NO: 207 |
| G2-H1-hz15-hIgG1(LALAGA, -K) | SEQ ID NO: 174 | SEQ ID NO: 208 |
| G2-H1-hz16-hIgG1(LALAGA, -K) | SEQ ID NO: 174 | SEQ ID NO: 209 |
| G2-H5-H1.2L1-hIgG1(LALAGA,-K) | SEQ ID NO: 175 | SEQ ID NO: 210 |
| G2-H5-H2.2L1-hIgG1(LALAGA, -K) | SEQ ID NO: 176 | SEQ ID NO: 210 |
| G2-H5-H3.2L1-hIgG1(LALAGA,-K) | SEQ ID NO: 177 | SEQ ID NO: 210 |
| G2-H5-H4.2L1-hIgG1(LALAGA,-K) | SEQ ID NO: 178 | SEQ ID NO: 210 |
| G2-H5-H1.2L2-hIgG1(LALAGA,-K) | SEQ ID NO: 175 | SEQ ID NO: 211 |
| G2-H5-H2.2L2-hIgG1(LALAGA,-K) | SEQ ID NO: 176 | SEQ ID NO: 211 |
| G2-H5-H3.2L2-hIgG1(LALAGA,-K) | SEQ ID NO: 177 | SEQ ID NO: 211 |
| G2-H5-H4.2L2-hIgG1(LALAGA,-K) | SEQ ID NO: 178 | SEQ ID NO: 211 |
| G2-H5-H1.2L3-hIgG1(LALAGA,-K) | SEQ ID NO: 175 | SEQ ID NO: 212 |
| G2-H5-H2.2L3-hIgG1(LALAGA,-K) | SEQ ID NO: 176 | SEQ ID NO: 212 |
| G2-H5-H3.2L3-hIgG1(LALAGA,-K) | SEQ ID NO: 177 | SEQ ID NO: 212 |
| G2-H5-H4.2L3-hIgG1(LALAGA,-K) | SEQ ID NO: 178 | SEQ ID NO: 212 |
| G2-H5-H1.2L4-hIgG1(LALAGA,-K) | SEQ ID NO: 175 | SEQ ID NO: 213 |
| G2-H5-H2.2L4-hIgG1(LALAGA,-K) | SEQ ID NO: 176 | SEQ ID NO: 213 |
| G2-H5-H3.2L4-hIgG1(LALAGA,-K) | SEQ ID NO: 177 | SEQ ID NO: 213 |
| G2-H5-H4.2L4-hIgG1(LALAGA,-K) | SEQ ID NO: 178 | SEQ ID NO: 213 |
| G2-H8-H1L1-hIgG1(LALAGA,-K) | SEQ ID NO: 179 | SEQ ID NO: 214 |
| G2-H8-H2L1-hIgG1(LALAGA,-K) | SEQ ID NO: 180 | SEQ ID NO: 214 |
| G2-H8-H1L2-hIgG1(LALAGA,-K) | SEQ ID NO: 179 | SEQ ID NO: 215 |
| G2-H8-H2L2-hIgG1(LALAGA,-K) | SEQ ID NO: 180 | SEQ ID NO: 215 |
| G2-H8-H1L1-hIgG1(LALAGA,-K)(gsm) | SEQ ID NO: 181 | SEQ ID NO: 216 |
| G2-H8-H1L2-hIgG1(LALAGA,-K)(gsm) | SEQ ID NO: 181 | SEQ ID NO: 217 |
| G2-H8-H2L1-hIgG1(LALAGA,-K)(gsm) | SEQ ID NO: 182 | SEQ ID NO: 216 |
| G2-H8-H2L2-hIgG1(LALAGA,-K)(gsm) | SEQ ID NO: 182 | SEQ ID NO: 217 |
| G2-H8-H1L1-hIgG1(LALAGA,-K)(gsm) | SEQ ID NO: 229 | SEQ ID NO: 216 |

**Table 12**

| Antibody Name | Reduced Molecular Weight (Kda) | Non-Reduced Molecular Weight (Kda) | Isoelectric Point | Extinction Coefficient | Concentration (mg/ml) |
|---|---|---|---|---|---|
| G2-H1-hz1-hIgG1(LALAGA, -K) | 49/23Kda | 144Kda | 8.41 | 1.595 | 9.73 |
| G2-H1-hz2-hIgG1(LALAGA, -K) | 49/23Kda | 144Kda | 8.41 | 1.597 | 9.10 |
| G2-H1-hz4-hIgG1(LALAGA, -K) | 49/23Kda | 144Kda | 8.31 | 1.597 | 3.51 |
| G2-H1-hz6-hIgG1(LALAGA, -K) | 49/23Kda | 144Kda | 8.41 | 1.599 | 10.26 |
| G2-H1-hz8-hIgG1(LALAGA, -K) | 49/23Kda | 144Kda | 8.31 | 1.598 | 5.76 |
| G2-H1-hz13-hIgG1(LALAGA, -K) | 49/23Kda | 144Kda | 8.41 | 1.615 | 8.13 |
| G2-H1-hz14-hIgG1(LALAGA, -K) | 49/23Kda | 144Kda | 8.41 | 1.618 | 9.30 |
| G2-H1-hz15-hIgG1(LALAGA, -K) | 49/23Kda | 144Kda | 8.41 | 1.619 | 6.83 |
| G2-H5-H1.2L1-hIgG1(LALAGA,-K) | 49/23Kda | 144Kda | 7.85 | 1.521 | 6.14 |
| G2-H5-H1.2L2-hIgG1(LALAGA,-K) | 49/23Kda | 144Kda | 7.85 | 1.542 | 5.69 |
| G2-H5-H1.2L3-hIgG1(LALAGA,-K) | 49/23Kda | 144Kda | 7.85 | 1.523 | 5.76 |
| G2-H5-H1.2L4-hIgG1(LALAGA,-K) | 49/23Kda | 144Kda | 7.85 | 1.522 | 7.06 |
| G2-H5-H2.2L1-hIgG1(LALAGA, -K) | 49/23Kda | 144Kda | 8.04 | 1.521 | 7.73 |
| G2-H5-H2.2L2-hIgG1(LALAGA,-K) | 49/23Kda | 144Kda | 8.04 | 1.541 | 7.71 |
| G2-H5-H2.2L3-hIgG1(LALAGA,-K) | 49/23Kda | 144Kda | 8.04 | 1.522 | 8.53 |
| G2-H5-H2.2L4-hIgG1(LALAGA,-K) | 49/23Kda | 144Kda | 8.04 | 1.522 | 7.01 |
| G2-H5-H3.2L1-hIgG1(LALAGA,-K) | 49/23Kda | 144Kda | 8.04 | 1.522 | 9.45 |
| G2-H5-H3.2L2-hIgG1(LALAGA,-K) | 49/23Kda | 144Kda | 8.04 | 1.542 | 8.5 |
| G2-H5-H3.2L3-hIgG1(LALAGA,-K) | 49/23Kda | 144Kda | 8.04 | 1.523 | 9.07 |
| G2-H5-H3.2L4-hIgG1(LALAGA,-K) | 49/23Kda | 144Kda | 8.04 | 1.523 | 6.91 |
| G2-H5-H4.2L1-hIgG1(LALAGA,-K) | 49/23Kda | 144Kda | 8.04 | 1.523 | 7.86 |
| G2-H5-H4.2L2-hIgG1(LALAGA,-K) | 49/23Kda | 144Kda | 8.04 | 1.543 | 8.02 |
| G2-H5-H4.2L3-hIgG1(LALAGA,-K) | 49/23Kda | 144Kda | 8.04 | 1.524 | 8.5 |
| G2-H5-H4.2L4-hIgG1(LALAGA,-K) | 49/23Kda | 144Kda | 8.04 | 1.524 | 6.91 |
| G2-H8-H1L1-hIgG1(LALAGA,-K)(gsm) | 49/23Kda | 144Kda | 8.03 | 1.509 | 8.19 |
| G2-H8-H2L2-hIgG1(LALAGA,-K) | 49/23Kda | 144Kda | 8.03 | 1.509 | 8.12 |
| GDAb2-hIgG1(LALAGA) | 49/24Kda | 146Kda | 7.24 | 1.554 | 4.61 |

**Table 13**

| Antibody Name | Purity (SEC, 214 nm) | Purity (SEC, 280 nm) | Purity (SDS-PAGE) | Endotoxin EU/mg |
|---|---|---|---|---|
| G2-H1-hz1-hIgG1(LALAGA, -K) | 96.190% | 96.756% | >90% | <1 |
| G2-H1-hz2-hIgG1(LALAGA, -K) | 98.578% | 98.653% | >95% | <1 |
| G2-H1-hz4-hIgG1(LALAGA, -K) | 99.049% | 99.122% | >90% | <1 |
| G2-H1-hz6-hIgG1(LALAGA, -K) | 95.198% | 95.801% | >95% | <1 |
| G2-H1-hz8-hIgG1(LALAGA, -K) | 100.000% | 100.000% | >90% | <1 |
| G2-H1-hz13-hIgG1(LALAGA, -K) | 99.226% | 99.316% | >95% | <1 |
| G2-H1-hz14-hIgG1(LALAGA, -K) | 94.078% | 95.577% | >95% | <1 |
| G2-H1-hz15-hIgG1(LALAGA, -K) | 98.757% | 98.929% | >95% | <1 |
| G2-H5-H1.2L1-hIgG1(LALAGA,-K) | 99.554% | 99.610% | >95% | <1 |
| G2-H5-H1.2L2-hIgG1(LALAGA,-K) | 99.513% | 98.502% | >95% | <1 |
| G2-H5-H1.2L3-hIgG1(LALAGA,-K) | 99.606% | 99.671% | >95% | <1 |
| G2-H5-H1.2L4-hIgG1(LALAGA,-K) | 100.000% | 99.620% | >95% | <1 |
| G2-H5-H2.2L1-hIgG1(LALAGA, -K) | 99.543% | 99.360% | >95% | <1 |
| G2-H5-H2.2L2-hIgG1(LALAGA,-K) | 97.786% | 97.858% | >95% | <1 |
| G2-H5-H2.2L3-hIgG1(LALAGA,-K) | 99.552% | 98.619% | >95% | <1 |
| G2-H5-H2.2L4-hIgG1(LALAGA,-K) | 100.000% | 99.119% | >95% | <1 |
| G2-H5-H3.2L1-hIgG1(LALAGA,-K) | 99.009% | 99.759% | >95% | <1 |
| G2-H5-H3.2L2-hIgG1(LALAGA,-K) | 98.670% | 98.799% | >95% | <1 |
| G2-H5-H3.2L3-hIgG1(LALAGA,-K) | 98.443% | 98.775% | >95% | <1 |
| G2-H5-H3.2L4-hIgG1(LALAGA,-K) | 100.000% | 99.069% | >95% | <1 |
| G2-H5-H4.2L1-hIgG1(LALAGA,-K) | 99.022% | 99.575% | >95% | <1 |
| G2-H5-H4.2L2-hIgG1(LALAGA,-K) | 98.979% | 99.759% | >95% | <1 |
| G2-H5-H4.2L3-hIgG1(LALAGA,-K) | 98.957% | 99.002% | >95% | <1 |
| G2-H5-H4.2L4-hIgG1(LALAGA,-K) | 98.200% | 98.425% | >95% | <1 |
| G2-H8-H1L1-hIgG1(LALAGA,-K)(gsm) | 98.105% | 99.272% | >95% | <1 |
| G2-H8-H2L2-hIgG1(LALAGA,-K) | 98.810% | 99.068% | >90% | <1 |
| GDAb2-hIgG1(LALAGA) | 99.887% | 99.967% | >95% | <1 |

### Test Example 3: Activity and Function Detection of Humanized Anti-GDF15 Neutralizing Antibodies

### 1. Immunogenicity Analysis of Humanized Anti-GDF15 Neutralizing Antibodies

To verify the potential immunogenicity that may be caused by the candidate molecules, the inventors selected 3 candidate antibodies (see Table 14 for details, and see Example 3 for specific amino acid sequences) for MHCII linear epitope prediction related to immunogenicity. This study used the public website resource NETMHCIIpan-4.0 to predict MHCII epitopes for the candidate molecules. Ten HLA subtypes common in Asians, Caucasians, and people of color, i.e., HLA-DRB1*01, 1*03, 1*04, 1*07, 1*08, 1*11, 1*13, 1*15, 1*09, and 1*12 alleles, were selected for analysis. Strong binding peptides appearing in three or more alleles with a rank within the top 1% were identified as epitope peptides. The prediction results are shown in Table 14. The results indicated that the immunogenicity of the three molecules was weak, and the risk of immunogenicity was low.

**Table 14**

| Peptide Chain of the Three Candidate Molecules | Number of Epitopes | Location |
|---|---|---|
| Heavy chain of G2-H8-H1L1-hIgG1(LALAGA,-K) | 1 | Fc |
| Light chain of G2-H8-H1L1-hIgG1(LALAGA,-K) | 0 | - |
| Heavy chain of G2-H5-H4.2L4-hIgG1(LALAGA,-K) | 2 | CDR2, Fc |
| Light chain of G2-H5-H4.2L4-hIgG1(LALAGA,-K) | 1 | VL non-CDR region |
| Heavy chain of G2-H1-hz2-hIgG1(LALAGA,-K) | 1 | Fc |
| Light chain of G2-H1-hz2-hIgG1(LALAGA,-K) | 1 | VL non-CDR region |

### 2. Detection of Affinity Kinetics of Humanized Antibodies to GDF 15 by SPR Method

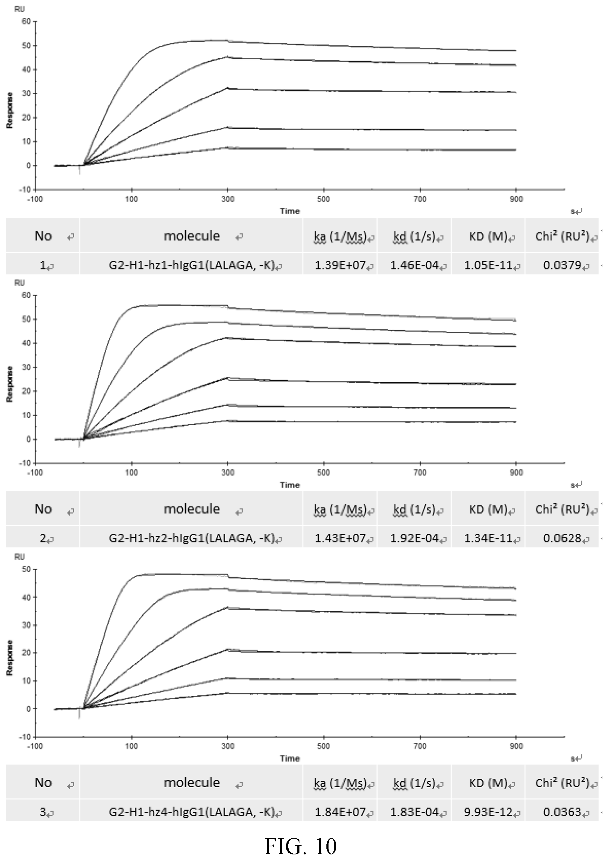

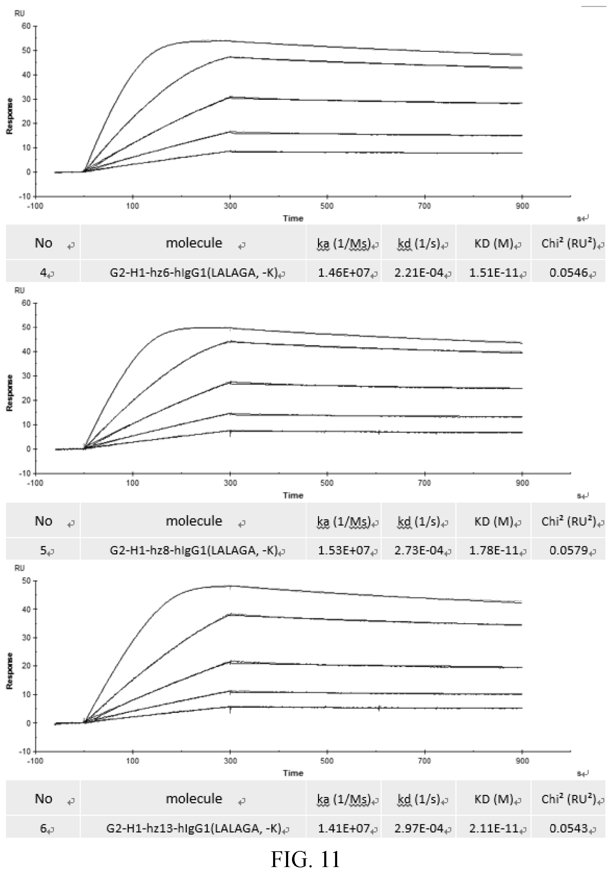

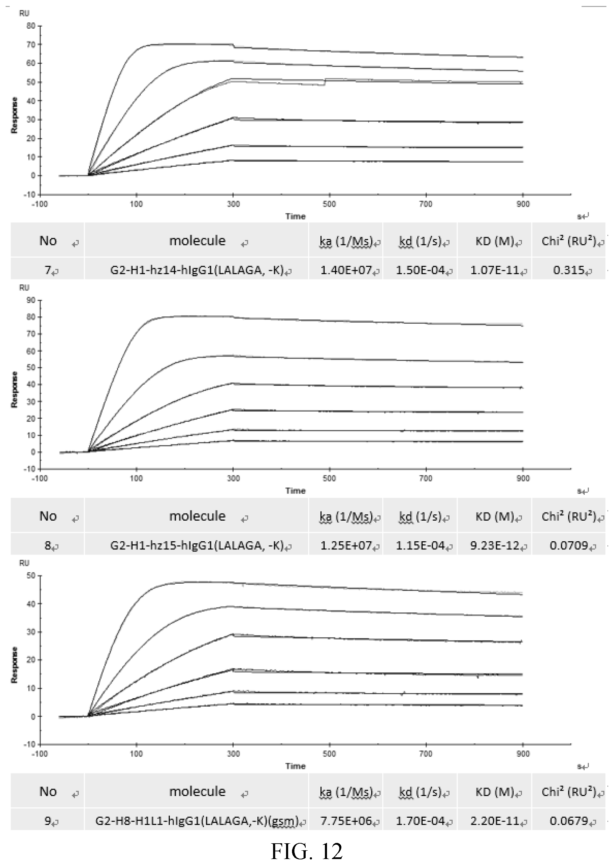

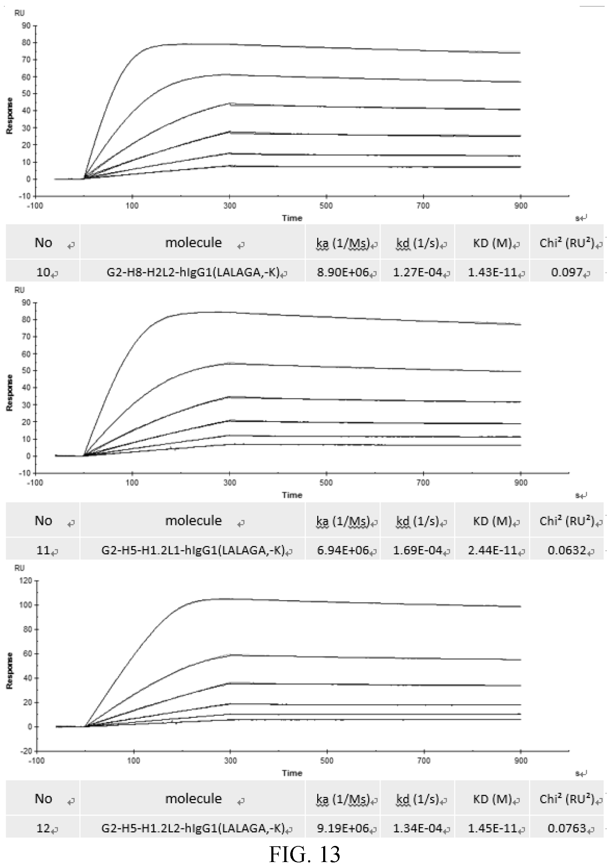

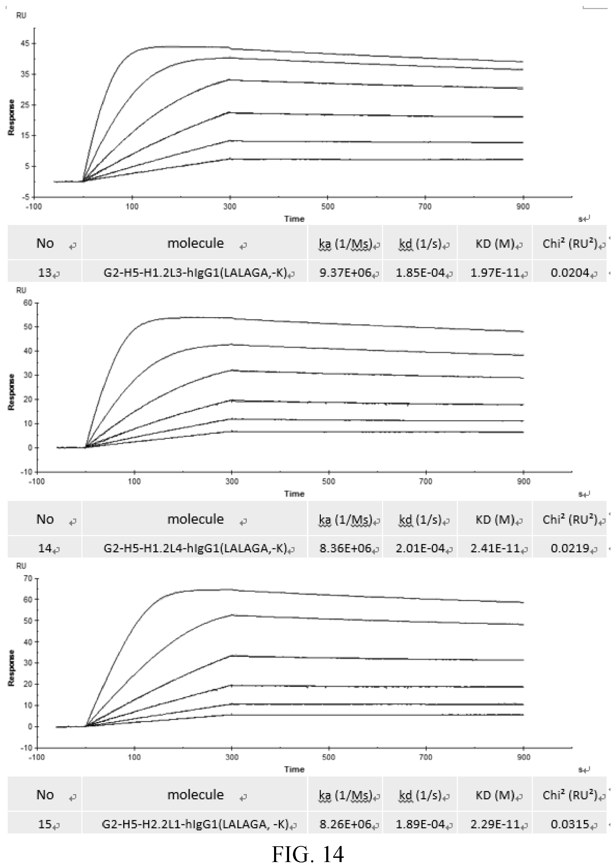

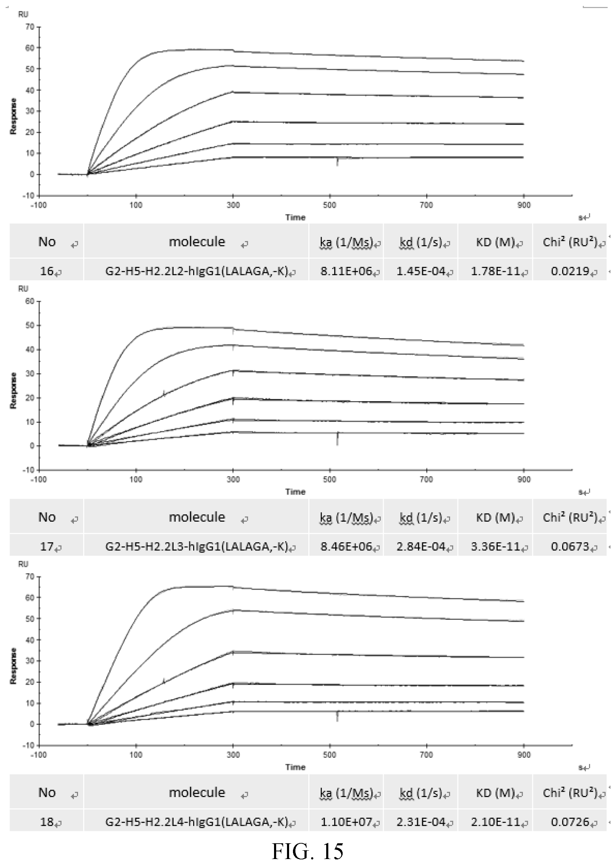

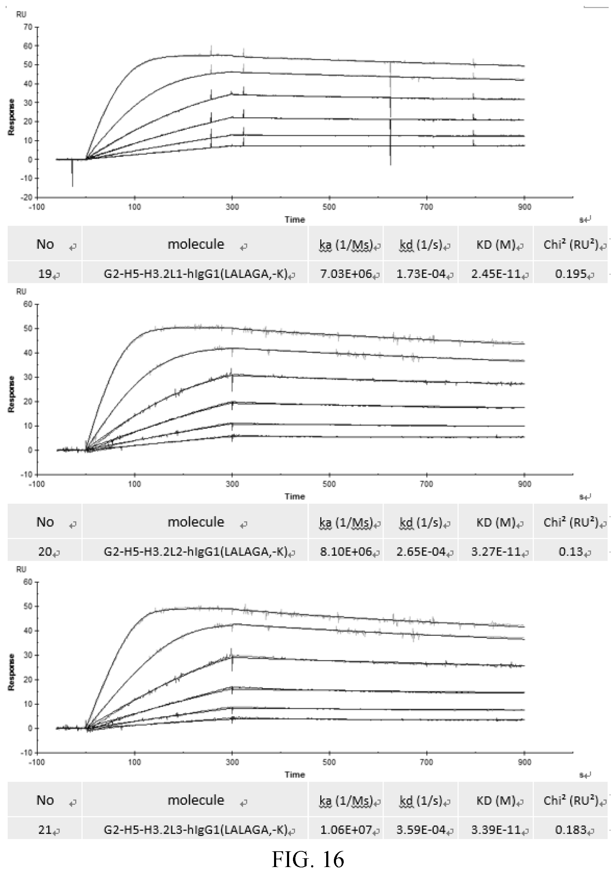

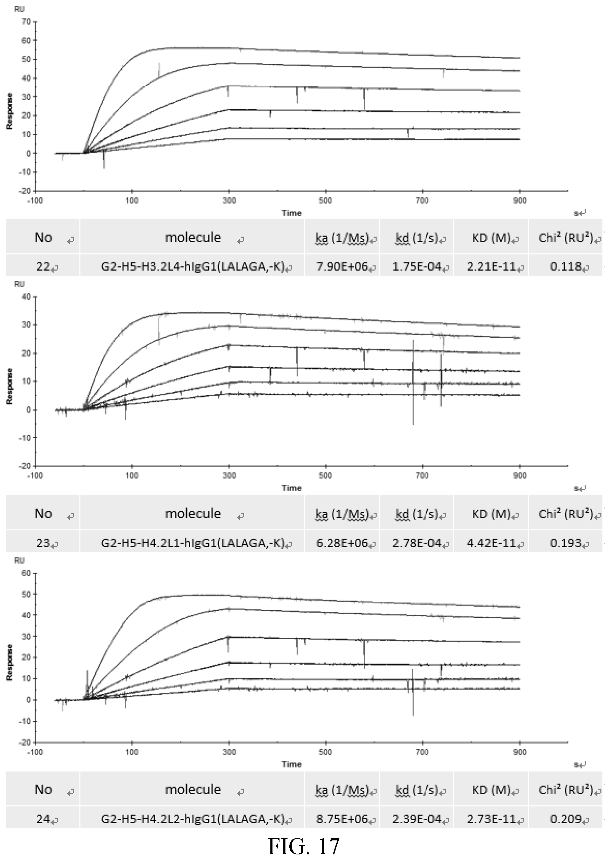

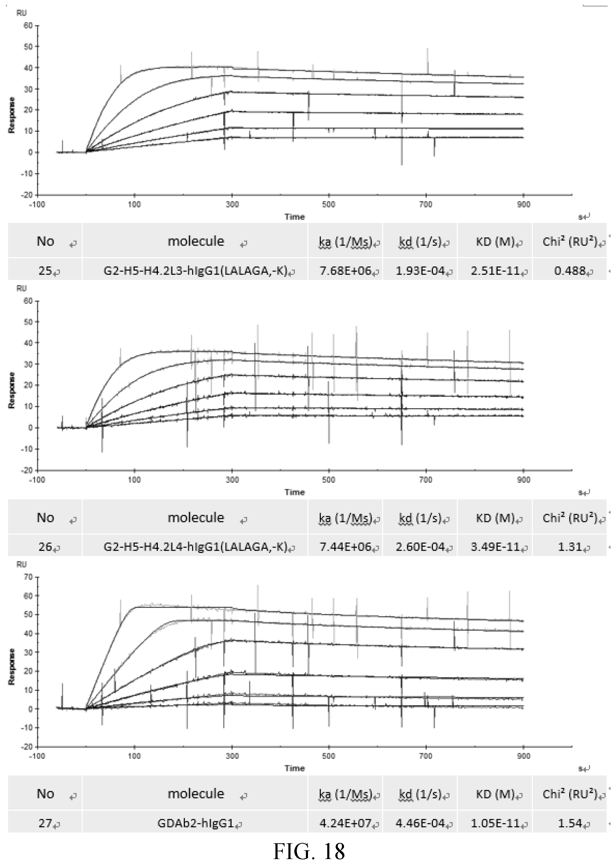

The affinity of the humanized anti-GDF15 neutralizing antibody samples prepared in Example 3 to GDF15 protein was determined by SPR using a Biacore T200 (Cytiva). The specific steps were as follows:
A CM5 chip (Cytiva, 29104988) was pre-coupled with an anti-hFc antibody (Cytiva, 29234600) as the detection chip. HBS-EP+ (Cytiva, BR100669) was used as the running buffer. Each cycle included capture of the ligand, injection of different concentrations of the analyte, and regeneration. Antibody protein at 1 µg/mL was injected into channels 1 and 2 of the chip at a flow rate of 10 µL/min for 60 sec to be captured on the channel surface via the anti-hFc antibody, with an immobilization level of about 300 RU. Channel 1 of the chip served as the reference channel. Using the High Performance model, at a flow rate of 30 µL/min, diluted GDF15 protein (R&D, 8146-GD) was injected sequentially into channels 1 and 2 at concentrations of 10 nM, 5 nM, 2.5 nM, 1.25 nM, 0.625 nM, 0.3125 nM, and 0 nM, with an association time of 300 sec and a dissociation time of 600 sec. The chip was regenerated by injecting 3 M MgCl₂ at a flow rate of 30 µL/mL for 60 sec. Data were analyzed using Biacore T200 analysis software with the 1:1 Binding model, and KD was automatically calculated and provided by the software. The data are shown in detail in Table 15 and Figures 10-18. The detection results indicated that the affinities of the 26 antibodies were comparable to GDAb2, all in the order of 10⁻¹¹ to 10⁻¹². It should be noted that in this test example, G2-H1-hz1-hIgG1(LALAGA, -K), G2-H1-hz1-hIgG1, and G2-H1-hz1 are synonymous, and the same applies to other groups. The comparative antibody GDAb2-hIgG1(LALAGA) refers to hlgG1(LALAGA, -K) without the deletion of the terminal K; GDAb2 appearing subsequently is synonymous with GDAb2-hIgG1(LALAGA).

**Table 15**

| Antibody Name | ka (1/Ms) | kd (1/s) | KD (M) | Chi² (RU²) |
|---|---|---|---|---|
| G2-H1-hz1-hIgG1(LALAGA, -K) | 1.39E+07 | 1.46E-04 | 1.05E-11 | 0.0379 |
| G2-H1-hz2-hIgG1(LALAGA, -K) | 1.43E+07 | 1.92E-04 | 1.34E-11 | 0.0628 |
| G2-H1-hz4-hIgG1(LALAGA, -K) | 1.84E+07 | 1.83E-04 | 9.93E-12 | 0.0363 |
| G2-H1-hz6-hIgG1(LALAGA, -K) | 1.46E+07 | 2.21E-04 | 1.51E-11 | 0.0546 |
| G2-H1-hz8-hIgG1(LALAGA, -K) | 1.53E+07 | 2.73E-04 | 1.78E-11 | 0.0579 |
| G2-H1-hz13-hIgG1(LALAGA, -K) | 1.41E+07 | 2.97E-04 | 2.11E-11 | 0.0543 |
| G2-H1-hz14-hIgG1(LALAGA, -K) | 1.40E+07 | 1.50E-04 | 1.07E-11 | 0.315 |
| G2-H1-hz15-hIgG1(LALAGA, -K) | 1.25E+07 | 1.15E-04 | 9.23E-12 | 0.0709 |
| G2-H8-H1L1-hIgG1(LALAGA,-K)(gsm) | 7.75E+06 | 1.70E-04 | 2.20E-11 | 0.0679 |
| G2-H8-H2L2-hIgG1(LALAGA,-K) | 8.90E+06 | 1.27E-04 | 1.43E-11 | 0.097 |
| G2-H5-H1.2L1-hIgG1(LALAGA,-K) | 6.94E+06 | 1.69E-04 | 2.44E-11 | 0.0632 |
| G2-H5-H1.2L2-hIgG1(LALAGA,-K) | 9.19E+06 | 1.34E-04 | 1.45E-11 | 0.0763 |
| G2-H5-H1.2L3-hIgG1(LALAGA,-K) | 9.37E+06 | 1.85E-04 | 1.97E-11 | 0.0204 |
| G2-H5-H1.2L4-hIgG1(LALAGA,-K) | 8.36E+06 | 2.01E-04 | 2.41E-11 | 0.0219 |
| G2-H5-H2.2L1-hIgG1(LALAGA, -K) | 8.26E+06 | 1.89E-04 | 2.29E-11 | 0.0315 |
| G2-H5-H2.2L2-hIgG1(LALAGA,-K) | 8.11E+06 | 1.45E-04 | 1.78E-11 | 0.0219 |
| G2-H5-H2.2L3-hIgG1(LALAGA,-K) | 8.46E+06 | 2.84E-04 | 3.36E-11 | 0.0673 |
| G2-H5-H2.2L4-hIgG1(LALAGA,-K) | 1.10E+07 | 2.31E-04 | 2.10E-11 | 0.0726 |
| G2-H5-H3.2L1-hIgG1(LALAGA,-K) | 7.03E+06 | 1.73E-04 | 2.45E-11 | 0.195 |
| G2-H5-H3.2L2-hIgG1(LALAGA,-K) | 8.10E+06 | 2.65E-04 | 3.27E-11 | 0.13 |
| G2-H5-H3.2L3-hIgG1(LALAGA,-K) | 1.06E+07 | 3.59E-04 | 3.39E-11 | 0.183 |
| G2-H5-H3.2L4-hIgG1(LALAGA,-K) | 7.90E+06 | 1.75E-04 | 2.21E-11 | 0.118 |
| G2-H5-H4.2L1-hIgG1(LALAGA,-K) | 6.28E+06 | 2.78E-04 | 4.42E-11 | 0.193 |
| G2-H5-H4.2L2-hIgG1(LALAGA,-K) | 8.75E+06 | 2.39E-04 | 2.73E-11 | 0.209 |
| G2-H5-H4.2L3-hIgG1(LALAGA,-K) | 7.68E+06 | 1.93E-04 | 2.51E-11 | 0.488 |
| G2-H5-H4.2L4-hIgG1(LALAGA,-K) | 7.44E+06 | 2.60E-04 | 3.49E-11 | 1.31 |
| GDAb2-hIgG1(LALAGA) | 4.24E+07 | 4.46E-04 | 1.05E-11 | 1.54 |

### 3. Detection of Receptor Blocking Activity of Humanized Antibodies by Reporter Gene Assay

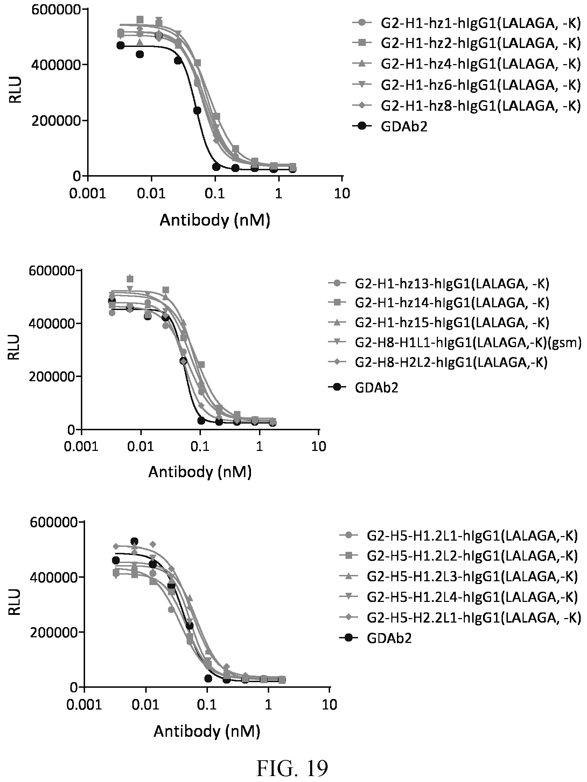

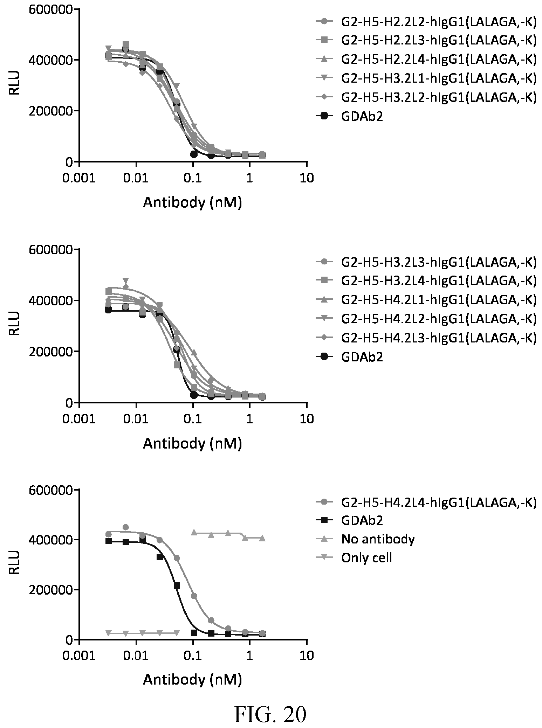

The neutralizing activity of the humanized antibodies prepared in Example 3 was detected using a reporter gene cell strain stably transfected with the hGDF15 receptor GFRAL and the co-receptor RET. For the specific detection method, refer to step 2 in Test Example 1. The detection results are shown in Figures 19-20 and Table 16. The results indicated that all antibodies can significantly inhibit the activation of the GFRAL-RET pathway by GDF15, and the blocking activity of all antibodies was comparable to that of GDAb2.

**Table 16**

| Antibody Name | IC₅₀ (pM) | Antibody Name | IC₅₀ (pM) |
|---|---|---|---|
| G2-H1-hz1-hIgG1(LALAGA,-K) | 63 | G2-H5-H2.2L2-hIgG1(LALAGA,-K) | 49 |
| G2-H1-hz2-hIgG1(LALAGA,-K) | 80 | G2-H5-H2.2L3-hIgG1(LALAGA,-K) | 48 |
| G2-H1-hz4-hIgG1(LALAGA,-K) | 71 | G2-H5-H2.2L4-hIgG1(LALAGA,-K) | 48 |
| G2-H1-hz6-hIgG1(LALAGA,-K) | 73 | G2-H5-H3.2L1-hIgG1(LALAGA,-K) | 68 |
| G2-H1-hz8-hIgG1(LALAGA,-K) | 62 | G2-H5-H3.2L2-hIgG1(LALAGA,-K) | 41 |
| G2-H1-hz13-hIgG1(LALAGA,-K) | 62 | G2-H5-H3.2L3-hIgG1(LALAGA,-K) | 64 |
| G2-H1-hz14-hIgG1(LALAGA,-K) | 85 | G2-H5-H3.2L4-hIgG1(LALAGA,-K) | 39 |
| G2-H1-hz15-hIgG1(LALAGA,-K) | 77 | G2-H5-H4.2L1-hIgG1(LALAGA,-K) | 87 |
| G2-H8-H1L1-hIgG1 (gsm)(LALAGA,-K) | 69 | G2-H5-H4.2L2-hIgG1(LALAGA,-K) | 59 |
| G2-H8-H2L2-hIgG1(LALAGA,-K) | 53 | G2-H5-H4.2L3-hIgG1(LALAGA,-K) | 49 |
| G2-H5-H1.2L1-hIgG1(LALAGA,-K) | 36 | G2-H5-H4.2L4-hIgG1(LALAGA,-K) | 81 |
| G2-H5-H1.2L2-hIgG1(LALAGA,-K) | 46 | GDAb2 | 50 |
| G2-H5-H1.2L3-hIgG1(LALAGA,-K) | 69 | | |
| G2-H5-H1.2L4-hIgG1(LALAGA,-K) | 53 | | |
| G2-H5-H2.2L1-hIgG1(LALAGA,-K) | 55 | | |

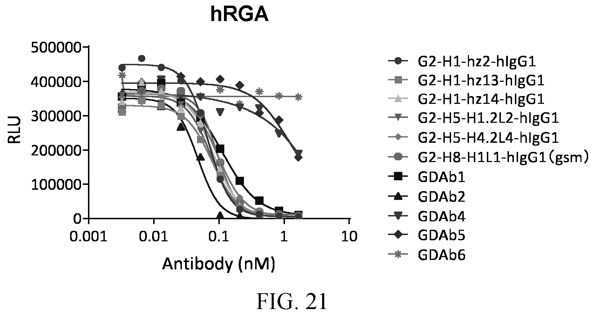

4. Detection of the function of neutralizing GDF15 and blocking the GFRAL pathway for 6 humanized antibodies prepared in Examples 3 and 4 reported comparative antibodies by reporter gene assay. The specific detection steps were as follows:
To compare the GDF15 mediated GFRAL pathway blocking activity of the GDF15 antibodies prepared in Example 3 and the comparative antibodies, a reporter gene cell strain (hGFRAL/RET reporter gene cells) was used to compare the blocking activity on the GDF15/GFRAL/RET signaling pathway of 6 humanized antibodies and 5 currently reported comparative antibodies. The reporter gene cell strain was based on HEK293 cells co-transfected with the human GDF15 receptor GFRAL and the co-receptor RET. The activation of the GDF15 pathway was reflected by the expression of the downstream reporter gene luciferase. The results are shown in Figure 21, where the abscissa represents the logarithmically transformed antibody concentration, and the ordinate represents the fluorescence intensity detected in the reporter gene cells, indicating the degree of pathway activation. Among them, the 6 humanized antibodies prepared in Example 3 were G2-H1-hz2-hIgG1, G2-H1-hz14-hIgG1, G2-H1-hz13-hIgG1, G2-H5-H1.2L2-hIgG1, G2-H5-H4.2L4-hIgG1, and G2-H8-H1L1-hIgG1(gsm) (wherein "IgG1" is "IgG1(LALAGA,-K)"); the selected positive controls (i.e., comparative antibodies) were GDAb1, GDAb2, GDAb4 (from the Fourth Military Medical University of the Chinese People's Liberation Army, CN111393526B), GDAb5 (Visugromab from CaltalYM), and GDAb6 (from Industry Academic Cooperation Foundation of Kangwon National University, WO2019004550A1). The results indicated that the activity of the 6 humanized antibodies was close to that of GDAb1 and GDAb2, and stronger than that of GDAb4, GDAb5, and GDAb6.

Among them,
>GDAb4-VH: 
DVQLQESGPGLVKPSQSLSLTCTVTGYSITSAYAWNWIRQFPGNKLEWMGYISYSG
STSYNPSLKSRISITRDTSKNQFFLQFNSVTTEDTATYYCARGGDAEDYWGQGTTLTVSS
(SEQ ID NO: 222);
>GDAb4-VL: 
DIQMTQSPASLSVSVGETVTITCRASENIYSNLAWFQQKQGKSPQLLVYVATNLVD
GVPSRFSGSGSGTQYSLKINSLQSEDFGTYYCQHFWGTPWTFGGGTKLEIK(SEQ ID NO:
223);
>GDAb5-VH: 
QITLKESGPTLVKPTQTLTLTCTFSGFSLSTSGMGVSWIRQPPGKGLEWLAHIYWDD
DKRYNPTLKSRLTITKDPSKNQVVLTMTNMDPVDTATYYCARSSYGAMDYWGQGTLV
TVSS(SEQ ID NO: 224);
>GDAb5-VL: 
DIVLTQSPSFLSASVGDRVTITCKASQNVGTNVAWFQQKPGKSPKALIYSASYRYSG
VPDRFTGSGSGTEFTLTISSLQPEDFAAYFCQQYNNFPYTFGGGTKLEIK(SEQ ID NO:
225);
>GDAb6-VH: 
EVQLQQSGAELVRPGALVKLSCKASGFNIKDYYMHWVRQRPEQGLEWIGWIDPEN
GNTIYDPKFQGKASITTDTSSNTAYMQLSSLTCEDTAVYYCAVPDSWGQGTTLTVSS(SE
 Q ID NO: 226);
>GDAb6-VL:

DIVMTQAAPSVPVTPGESVSISCRSSKSLLHSNGNTYLYWFLQRPDQSPQLLIYRMS
NLASGVPDRFSGSGSGTAFTLRISRVEAEDVGVYYCMQHLEYPFTFGSGTKLEIK(SEQ
ID NO: 227).

### 5. Detection of Neutralization of GDF15-Induced Treg Differentiation by Candidate Antibodies Using Flow Cytometry

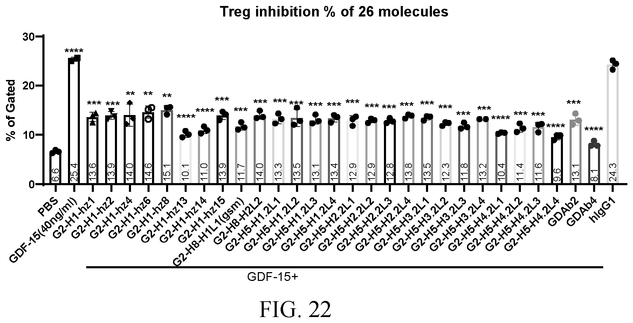

The GDF15 blocking activity of the humanized antibodies prepared in Example 3 was detected according to step 2 in Test Example 2. A bar graph was plotted with the abscissa representing each treatment group and the ordinate representing the proportion of gated Tregs (%), as shown in Figure 22. Compared with the GDF15-alone treatment group, the treatment groups co-incubated with humanized molecules and the comparative antibodies GDAb2 and GDAb4 (see step 4 of this test example for details) could all significantly neutralize the Treg differentiation induced by GDF15. Compared with GDAb2, G2-H1-hz13 (G2-H1-hz13-hIgG1), G2-H5-H4.2L1 (G2-H5-H4.2L1-hIgG1), and G2-H5-H4.2L4 (G2-H5-H4.2L4-hIgG1) showed stronger neutralization ability. The difference in Treg proportion among groups was statistically analyzed using one-way ANOVA.

In this test example, for instance, G2-H1-hz13-hIgG1 refers to G2-H1-hz13-hIgG1 (LALAGA, -K), and the same applies to other groups. Since there are too many groups to display their full names in Figure 22 and subsequent figures, the abbreviation hIgG1 is used in the figures, and (LALAGA, -K) is no longer indicated.

### 6. Efficacy Study of Anti-GDF15 Antibodies in Reversing Cachexia in the HT1080 Cachexia Model

The classic HT1080 cachexia model was selected for in vivo efficacy verification. The specific experimental steps were as follows:
6-8 week old female NCG mice (from GemPharmatech) were used to construct the HT1080 human fibrosarcoma cachexia model. When the mouse body weights decreased by about 10% (day 16 after tumor inoculation), the mice were grouped based on body weight. The grouping day was defined as D0, and group administration began on the grouping day according to the experimental protocol design:
G1: hIgG1 blank control;
G2: Positive control GDAb2 (see step 4 of this test example for details);
G3: G2-H1-hz2-hIgG1;
G4: G2-H5-H2.2L4-hIgG1;
G5: G2-H8-H1L1-hIgG1(gsm)
(In this test example, G2-H1-hz2-hIgG1 refers to G2-H1-hz2-hIgG1(LALAGA,-K), G2-H5-H2.2L4-hIgG1 refers to G2-H5-H2.2L4-hIgG1(LALAGA,-K), and G2-H8-H1L1-hIgG1(gsm) refers to G2-H8-H1L1-hIgG1(LALAGA,-K)(gsm)).

Administration volume: Adjusted according to mouse body weight (mouse administration volume = 10 µL/g × mouse body weight (g)); dosage 10 mg/kg, Q3D*6 times (i.e., administered once every 3 days, for a total of 6 doses). After grouping, body weight was measured daily, and tumor volume was measured twice a week. Tumor volume was calculated as: Tumor volume (mm³) = 0.5 × tumor long diameter × tumor short diameter². Serum was collected before grouping and on D18. Grip strength was measured on D12, and fat and muscle mass were measured on D13. The experiment ended on D18, and the mice were euthanized. For result analysis, since animals were euthanized sequentially after D13, data from D0 to D13 were analyzed.

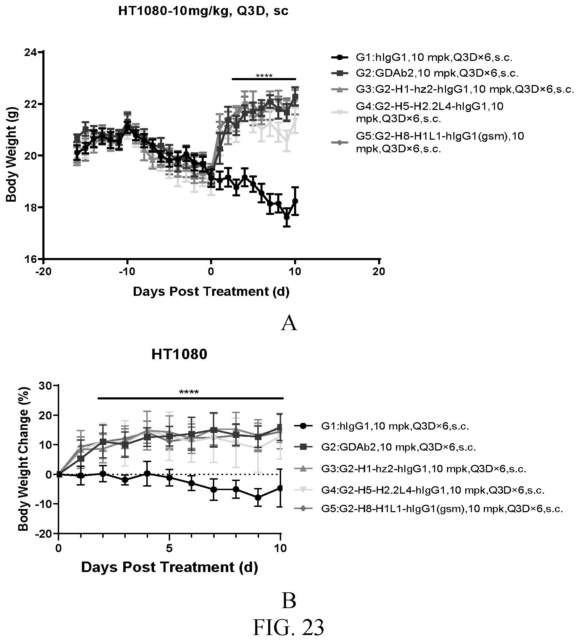

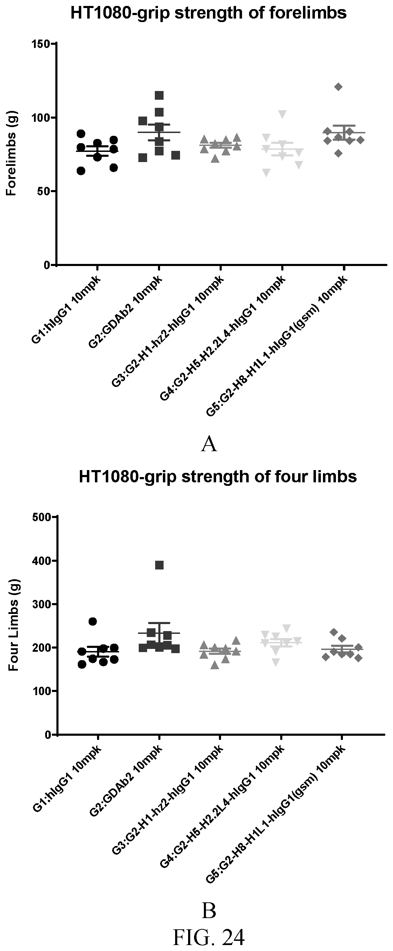

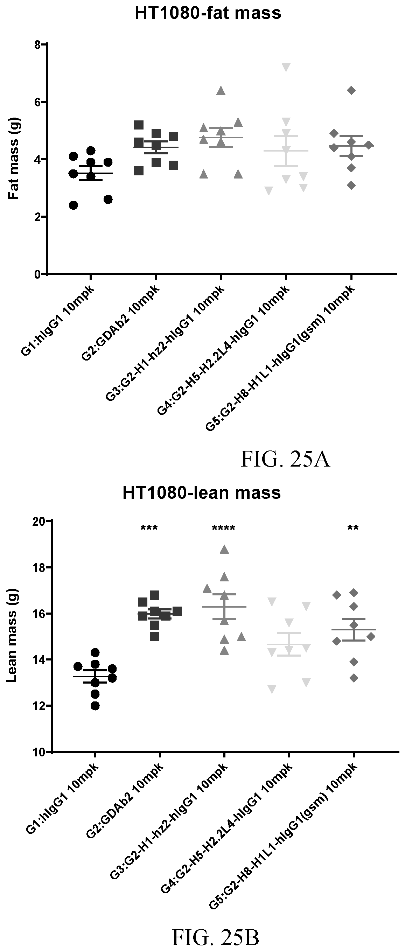

The experimental results are as set forth in Figures 23-25. The results showed that compared with the blank control group, after applying drug treatment in the comparative antibody control group and the candidate drug groups, the body weight of the mice gradually recovered from the first day (as shown in Figure 23A). From D3 to D13, the body weight of all administration groups was significantly higher than that of the blank control group (Figure 23A shows body weight change, and Figure 23B shows body weight change rate). Statistical analysis was performed using two-way ANOVA, and P < 0.05 was considered statistically significant. On day 12 after grouping, the forelimb (Figure 24A) and four-limb (Figure 24B) grip strength of each group was measured. The data are shown in Table 17 and Figure 24. There was no significant difference between all administration groups and the blank control group. On day 13 after grouping, fat and muscle mass were measured using DEXO. The results, as shown in Table 18, Figure 25A, and Figure 25B, indicated that the drug groups could significantly reverse muscle mass, but the effect on fat mass showed no statistical difference. Therefore, the in vivo efficacy experiment results in HT1080 demonstrated that the three tested drugs, G2-H1-hz2-hIgG1, G2-H5-H2.2L4-hIgG1, and G2-H8-H1L1-hIgG1(gsm), had a good effect on improving cachexia symptoms in the HT1080 cachexia model.

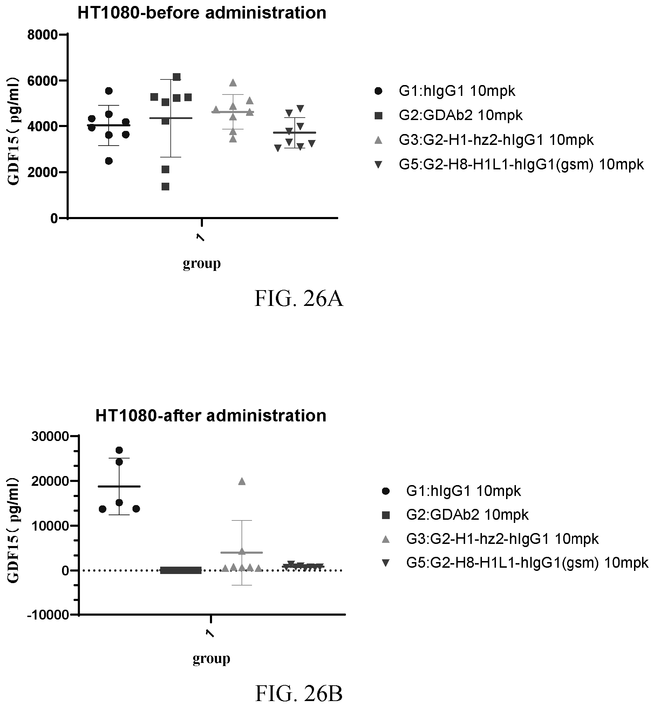

Simultaneously, to compare the neutralization of the target GDF15 by the drugs, the levels of serum free GDF15 in animals of each group before and after administration were measured, as shown in Figure 26A and Figure 26B. Before administration, GDF15 in animals of all groups was nearly 4 ng/mL. After administration, GDF15 in the blank control group increased to about 20 ng/mL. Compared with the blank control, serum free GDF15 was significantly decreased after treatment with the drugs GDAb2, G2-H1-hz2-hIgG1, and G2-H8-H1L1-hIgG1(gsm) (statistical analysis was performed using one-way ANOVA, and P < 0.05 was considered statistically significant), supporting the pharmacological effect of the drugs in reversing weight loss and improving cachexia symptoms after neutralizing GDF15.

**Table 17 Changes in Forelimb Grip Strength and Four-Limb Grip Strength of Mice in Different Groups**

| Group | Forelimb Grip Strength (g) | Four-Limb Grip Strength (g) |
|---|---|---|
| G1 | 77.1 | 190.2 |
| G2 | 89.9 | 232.9 |
| G3 | 81.1 | 191.5 |
| G4 | 78.5 | 211.1 |
| G5 | 89.7 | 196.3 |

### Statistical Analysis of P-Values for Forelimb Grip Strength and Four-Limb Grip Strength of Mice in Different Groups

| Comparison Group | Forelimb Grip Strength (g) | Four-Limb Grip Strength (g) |
|---|---|---|
| G2 vs·G1 | 0.1064 | 0.0794 |
| G3·vs·G1 | 0.9001 | 0.9999 |
| G4 vs·G1 | 0.9977 | 0.6120 |
| G5·vs·G1 | 0.1137 | 0.9918 |

**Table 18 Changes in Fat Mass, Muscle Mass and Body Fat Percentage of Mice in Different Groups**

| Group | Fat Mass (g) | Muscle Mass (g) | Body Fat Percentage (%) |
|---|---|---|---|
| G1 | 3.5 | 13.3 | 20.9 |
| G2 | 4.4 | 16.0 | 21.6 |
| G3 | 4.8 | 16.3 | 22.7 |
| G4 | 4.3 | 14.7 | 22.4 |
| G5 | 4.5 | 15.3 | 22.4 |

### Statistical Analysis of P-Values for Fat Mass, Muscle Mass, and Body Fat Percentage of Mice in Different Groups

| Comparison Group | Fat Mass (g) | Muscle Mass (g) | Body Fat Percentage (%) |
|---|---|---|---|
| G2 vs·G1 | 0.2214 | <0.0002*** | 0.9900 |
| G3·vs·G1 | 0.0517 | <0.0001**** | 0.8026 |
| G4 vs·G1 | 0.3399 | 0.0761 | 0.8830 |
| G5·vs·G1 | 0.1838 | 0.0054** | 0.8739 |

| | | | |
|---|---|---|---|
| Note: Statistical analysis here was performed using one-way ANOVA, and P < 0.05 was considered statistically significant. | | | |

### 7. Efficacy Study of Anti-GDF15 Antibodies in Reversing Cachexia in the LNCaP Cachexia Model

The LNCaP cachexia model was selected for in vivo efficacy verification. The specific experimental steps were as follows:
6-8 week old male CB17 SCID mice (from WuXi AppTec) were used to construct the LNCaP human prostate cancer cachexia model. When the mouse body weights decreased by about 9%, the mice were grouped based on body weight. The grouping day was defined as Day 0 (i.e., D0), and group administration began on the grouping day according to the experimental protocol design:
G1: IgG1 isotype control;
G2: Control antibody GDAb2;
G3: Control antibody GDAb5;
G4: G2-H1-hz2-hIgG1;
G5: G2-H1-hz14-hIgG1;
G6: G2-H8-H1L1-hIgG1 (gsm);
G7: G2-H5-H4.2L4-hIgG1;
G8: G2-H5-H1.2L2-hIgG1;
G9: G2-H1-hz13-hIgG1;
G10: NTA (non-tumor group of animals).
(In this test example: G2-H5-H4.2L4-hIgG1 refers to G2-H5-H4.2L4-hIgG1(LALAGA,-K), and the same applies to other groups.)

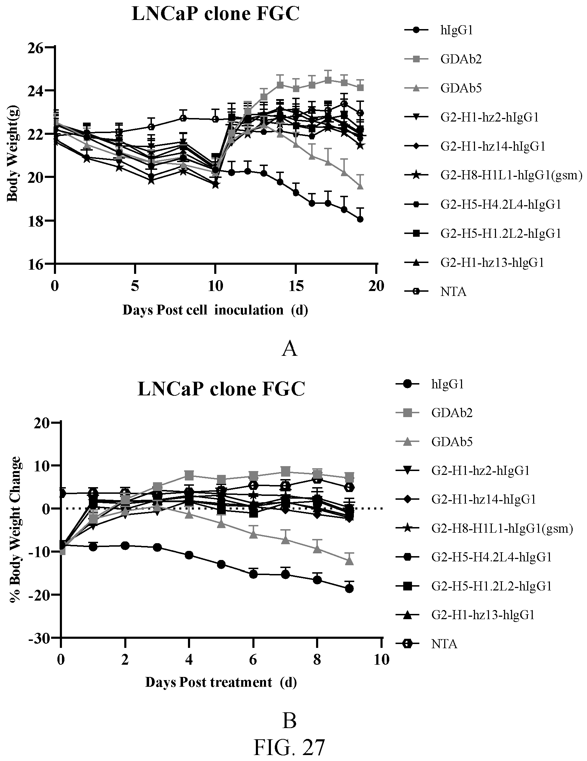

Administration volume: Adjusted according to mouse body weight (mouse administration volume = 10 µL/g × mouse body weight (g)); dosage 20 mg/kg, Q6D*2 times (i.e., administered once every 6 days, for a total of 2 doses). After grouping, body weight was measured daily, and tumor volume was measured twice a week. Tumor volume was calculated as: Tumor volume (mm³) = 0.5 × tumor long diameter × tumor short diameter². At the end of the experiment, the body fat percentage, fat mass, and muscle mass of the mice were measured. The experimental results are shown in Figures 27-28 and Table 19. The results in Figure 27 showed that compared with the isotype control group, after drug treatment in the control antibody GDAb2 and the test drug groups, the body weight of the mice gradually recovered from the first day until the end of the experiment; in the control antibody GDAb5 group, the body weight of the mice recovered to a certain extent at the beginning of administration, but as the administration time extended, the body weight gradually decreased and failed to maintain an upward trend, and subsequently, compared with the vehicle group, the decrease in mouse body weight was not significantly alleviated. The statistical analysis of body weight at different time points between each administration group and the isotype control group is shown in Table 1 (statistical analysis was performed using two-way ANOVA, P < 0.05 was considered statistically significant, and P values were obtained by comparison with the hIgG1 group).

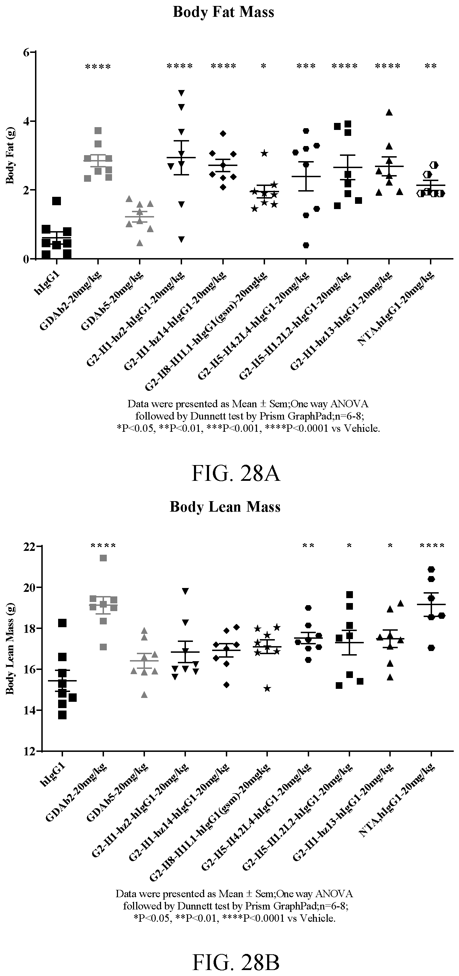

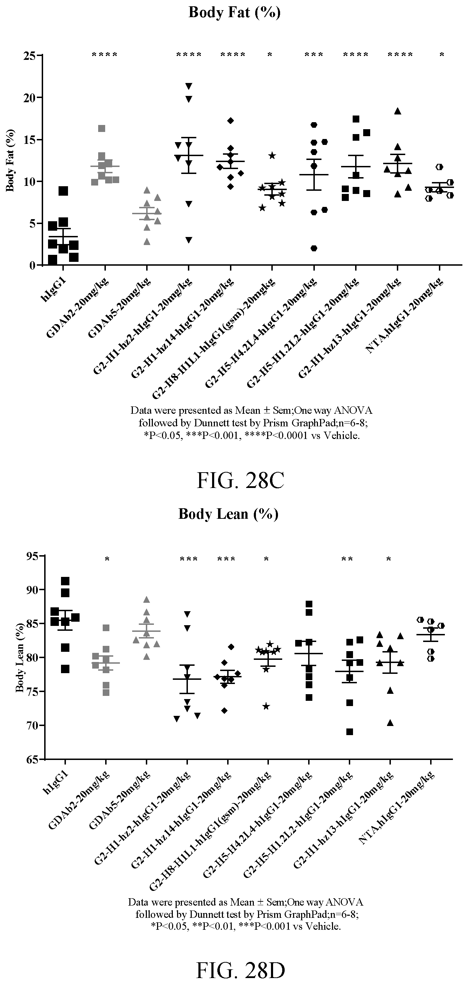

The results in Figure 28 showed that compared with the isotype control group, the fat mass in the test groups (except the GDAb5 antibody group) was statistically different from the control group; for muscle mass in the test groups, GDAb2, G2-H5-H4.2L4-hIgG1, G2-H5-H1.2L2-hIgG1, and G2-H1-hz13-hIgG1 were statistically different from the control group; the fat percentage results were similar to the fat mass results, with no difference in the GDAb5 antibody group compared to the control, while the other groups all showed statistical differences; the muscle percentage in each test group was lower than that in the control group.

In summary, the in vivo efficacy experiment results in the LNCaP model for the test drugs indicated that each test drug had good efficacy in reversing cachexia-induced weight loss.

**Table 19**

| Day | **Group** | | | | | | | | | |
|---|---|---|---|---|---|---|---|---|---|---|
| | **G1** | **G2** | **G3** | **G4** | **G5** | **G6** | **G7** | **G8** | **G9** | **G10** |
| 0 | - | *p*>0.05 | *p*>0.05 | *p*>0.05 | *p*>0.05 | *p*>0.05 | *p*>0.05 | *p*>0.05 | *p*>0.05 | *P < 0.05* |
| 1 | - | *p*>0.05 | *p*>0.05 | *p*>0.05 | *p*<0.01 | *p*>0.05 | *P < 0.05* | *p*<0.01 | *p*>0.05 | *p*<0.01 |
| 2 | - | *p*<0.001 | *p*>0.05 | *P < 0.05* | *P < 0.05* | *p*>0.05 | *p*>0.05 | *p*<0.01 | *p*<0.01 | *p*<0.01 |
| 3 | - | *p*<0.0001 | *P < 0.05* | *P < 0.05* | *p*<0.001 | *p*<0.01 | *P < 0.05* | *p*<0.01 | *p*<0.001 | *p*<0.01 |
| 4 | - | *p*<0.0001 | *P < 0.05* | *p*<0.001 | *p*<0.0001 | *p*<0.01 | *p*<0.01 | *p*<0.001 | *p*<0.0001 | *p*<0.001 |
| 5 | - | *p*<0.0001 | *P < 0.05* | *p*<0.0001 | *p*<0.0001 | *p*<0.0001 | *p*<0.01 | *p*<0.001 | *p*<0.0001 | *p*<0.0001 |
| 6 | - | *p*<0.0001 | *P < 0.05* | *p*<0.0001 | *p*<0.0001 | *p*<0.0001 | *p*<0.001 | *p*<0.0001 | *p*<0.0001 | *p*<0.0001 |
| 7 | - | *p*<0.0001 | *P < 0.05* | *p*<0.0001 | *p*<0.0001 | *p*<0.0001 | *p*<0.0001 | *p*<0.0001 | *p*<0.0001 | *p*<0.0001 |
| 8 | - | *p*<0.0001 | *p*>0.05 | *p*<0.0001 | *p*<0.0001 | *p*<0.0001 | *p*<0.0001 | *p*<0.0001 | *p*<0.0001 | *p*<0.0001 |
| 9 | - | *p*<0.0001 | *p*>0.05 | *p*<0.0001 | *p*<0.0001 | *p*<0.0001 | *p*<0.0001 | *p*<0.0001 | *p*<0.0001 | *p*<0.0001 |

### 8. Efficacy Study of Anti-GDF15 Antibodies in Reversing Cachexia in the MKN45 Cachexia Model

The MKN45 cachexia model was selected for in vivo efficacy verification. The specific experimental steps were as follows:
6-8 week old female BALB/c nude mice (from WuXi AppTec) were used to construct the MKN45 human gastric cancer cachexia model. When the average body weight of the mice decreased by about 6.6% (day 15 after tumor inoculation), the mice were grouped based on body weight. The grouping day was defined as D0, and group administration began on the grouping day according to the experimental protocol design:
G1: hIgG1 isotype control, dose of 10 mg/kg;
G2: Positive control GDAb2, dose of 1 mg/kg;
G3: Positive control GDAb2, dose of 3 mg/kg;
G4: G2-H8-H1L1-hIgG1(gsm), dose of 1 mg/kg;
G5: G2-H8-H1L1-hIgG1(gsm), dose of 3 mg/kg;
G6: G2-H8-H1L1-hIgG1(gsm), dose of 10 mg/kg;
G7: NTA (non-tumor group of animals).
(In this test example, G2-H8-H1L1-hIgG1(gsm) refers to G2-H8-H1L1-hIgG1(LALAGA, -K)(gsm).)

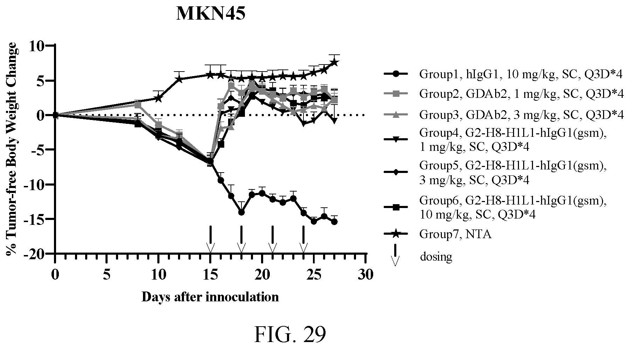

Administration volume: Adjusted according to mouse body weight (mouse administration volume = 10 µL/g × mouse body weight (g)); doses as above, Q3D*4 times (i.e., administered once every 3 days, for a total of 4 doses). After grouping, body weight was measured daily, and tumor volume was measured. Tumor volume was calculated as: Tumor volume (mm³) = 0.5 × tumor long diameter × tumor short diameter². Tumor weight was estimated as 1g/1000 mm³. Body fat was measured at the end of the experiment, and serum was collected and frozen at the endpoint. The body weight change and body fat percentage of mice in each group were measured at the end of the experiment. The experimental results are shown in Figures 29-30 and Table 20.

The body weight change results in Figure 29 showed that compared with the isotype control group, after drug treatment in the positive antibody control group and the candidate antibody group (G2-CH8-H1L1), the body weight of the mice gradually recovered from the first day until the end of the experiment. At the end of the experiment, the body weight of mice in all experimental groups returned to the baseline level (day of tumor inoculation). In the isotype control group, the body weight of the mice gradually decreased. Figure 29 shows the body weight change rate after tumor removal (statistical analysis of the experimental results was performed using one-way ANOVA, and P < 0.05 was considered statistically significant).

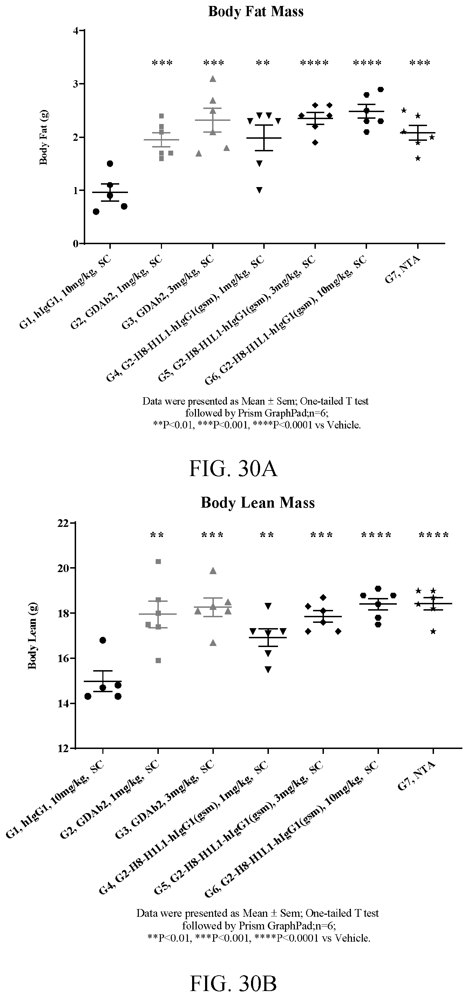

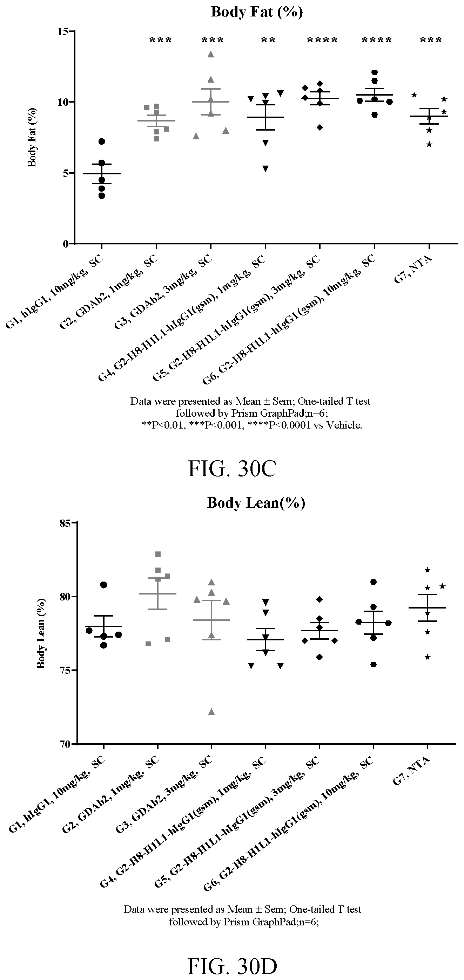

The body fat percentage results in Figure 30 and Table 1 showed that compared with the isotype control group, the fat mass, muscle mass, and fat percentage of mice in all experimental groups had significant differences, while there was no significant difference in muscle percentage.

Therefore, the comprehensive results as set forth in Figures 29 and 30 indicate that the candidate antibody of the present application demonstrated a good reversal of body weight loss in the MKN45 cachexia model mice; analysis of body fat results showed that compared with the isotype control group, the candidate antibody simultaneously reversed the loss of fat and muscle mass.

**Table 20-1: Mass and Proportion of Fat and Muscle in Different Groups of Mice**

| Group | Fat Mass (g) | Muscle Mass (g) | Fat Percentage (%) | Muscle Percentage (%) |
|---|---|---|---|---|
| G1 | 1.0 | 15.0 | 4.9 | 78.0 |
| G2 | 1.9 | 18.0 | 8.7 | 80.2 |
| G3 | 2.3 | 18.2 | 10.0 | 78.4 |
| G4 | 2.0 | 16.9 | 8.9 | 77.1 |
| G5 | 2.4 | 17.9 | 10.3 | 77.7 |
| G6 | 2.5 | 18.4 | 10.5 | 78.2 |
| G7 | 2.1 | 18.4 | 9.0 | 79.2 |

**Table 20-2: Statistical Analysis of P-Values for Mass and Proportion of Fat and Muscle in Different Groups of Mice**

| Group | Fat Mass (g) | Muscle Mass (g) | Fat Percentage (%) | Muscle Percentage (%) |
|---|---|---|---|---|
| G2 vs G1 | 0.0005*** | 0.0021** | 0.0004*** | 0.0657 |
| G3 vs G1 | 0.0005*** | 0.0003*** | 0.0010*** | 0.4003 |
| G4 vs G1 | 0.0041** | 0.0054** | 0.0039** | 0.2084 |
| G5 vs G1 | <0.0001**** | 0.0001*** | <0.0001 **** | 0.3743 |
| G6 vs G1 | <0.0001**** | <0.0001**** | <0.0001**** | 0.4095 |
| G7 vs G1 | 0.0002*** | <0.0001**** | 0.0006*** | 0.1575 |

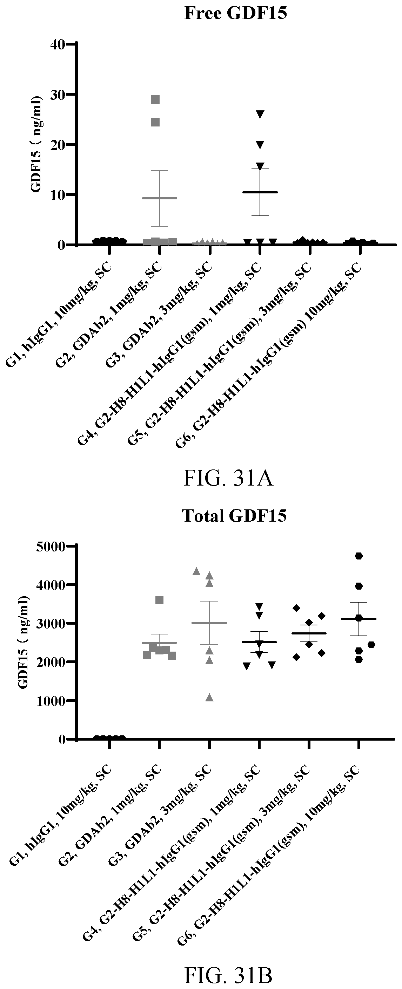

Furthermore, to analyze the neutralization of the target GDF15 by the test drugs, the levels of free and total (free + bound) GDF15 in the serum of animals in all tumor-bearing groups (excluding the NTA group) at the end of the experiment were measured. The detection results are shown in Figure 31A and Figure 31B. The serum free GDF15 results showed that doses of 3 mg/kg and 10 mg/kg were sufficient to neutralize all free GDF15 in the corresponding experimental groups of mice, i.e., the free GDF15 concentration decreased to the level of the isotype control group (<1 ng/mL). The total GDF15 concentration results showed that the total GDF15 concentration in the administration groups (positive control group, candidate antibody groups) was around 2-3 µg/mL, with no statistical difference between the administration groups (statistical analysis was performed using one-way ANOVA, and P < 0.05 was considered statistically significant).

### 9. Verification of the Activity of Candidate Antibodies in Tumor Immunity

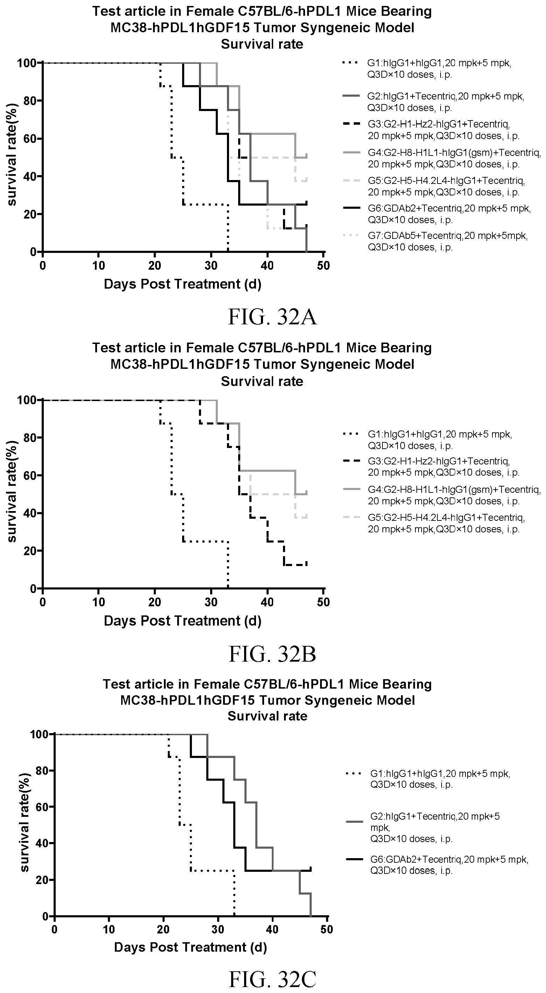

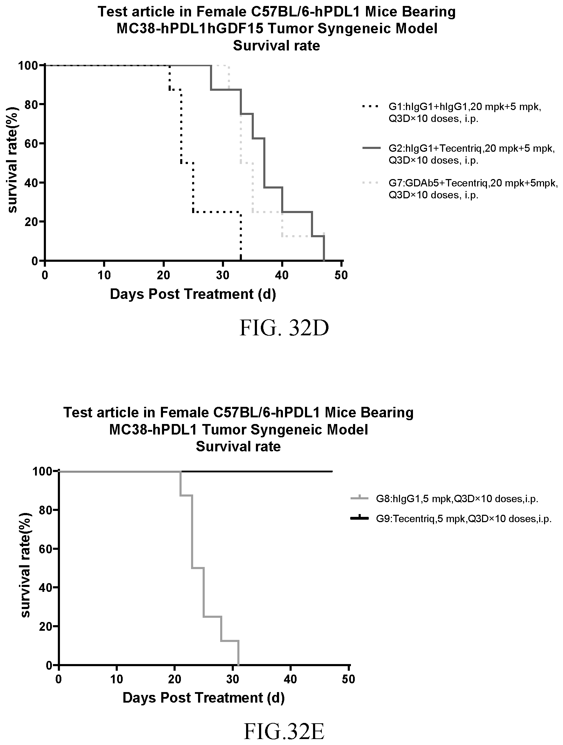

To verify the activity of the candidate antibodies in tumor immunity, this test example used C57BL/6-hPDL1 humanized mice inoculated with tumor cell of MC38-hPDL1-hGDF15 cells or its parental cell strain MC38-hPDL1 cells to establish models, with 8 mice per group. Administration was performed using anti-PDL1 agent (Tecentriq, referred to as T drug) alone or in combination with anti-PDL1 agent and GDF15 antibody. Changes in tumor volume and survival rate were observed (T drug was 5 mg/kg, GDF15 antibody prepared in Example 3 was 20 mg/kg, 8 animals per group). In the positive control group, the GDF15 antibody was substituted with a positive control antibody, where the selected positive controls (i.e., comparative antibodies) were GDAb2 (see step 1 of Test Example 1 for specific amino acid sequences) and GDAb5 (Visugromab, see step 4 of Test Example 3 for specific amino acid sequences). The results are shown in Tables 21 and 22, and Figures 32A, 32B, 32C, 32D, and 32E. The results indicated that the candidate antibody G2-H8-H1L1-hIgG1(gsm) combined with T drug group (G4) and the G2-H5-H4.2L4-hIgG1 combined with T drug group (G5) exhibited a better anti-tumor trend compared to the T drug alone group (G2), GDAb2 combined with T drug group (G6), and GDAb5 combined with T drug group (G7). Using 1000 mm³ as the humane endpoint, the survival rate of animals in each group was observed. Among the groups, the combination group of the G2-H8-H1L1-hIgG1(gsm) molecule showed a better anti-tumor trend compared to T drug alone.
G1: hIgG1 (20 mg/kg) + hIgG1 (5 mg/kg);
G2: hIgG1 (20 mg/kg) + Tecentriq (5 mg/kg);
G3: G2-H1-hz2-hIgG1 (20 mg/kg) + Tecentriq (5 mg/kg);
G4: G2-H8-H1L1-hIgG1(gsm) (20 mg/kg) + Tecentriq (5 mg/kg);
G5: G2-H5-H4.2L4-hIgG1 (20 mg/kg) + Tecentriq (5 mg/kg);
G6: GDAb2 (20 mg/kg) + Tecentriq (5 mg/kg);
G7: GDAb5 (20 mg/kg) + Tecentriq (5 mg/kg);
G8: hIgG1 (5 mg/kg);
G9: Tecentriq (5 mg/kg).
(In this test example, the heavy chain constant regions of the antibodies in groups G3-G5 all used hIgG1. G2-H1-hz2-hIgG1 is the same as G2-H1-hz2-hIgG1(LALAKA,-K), and the same applies to groups G4 and G5.)

**Table 21: Statistical Analysis of Survival Curves for Groups G1-G7**

| Group | Mean Survival Time (days) | Median Survival Time (days) |
|---|---|---|
| G1 | 25.8±4.7 | 24.0 |
| G2 | 37.8±6.2 | 37.0 |
| G3 | 37.3±6.0 | 36.0 |
| G4 | 41.8±6.8 | 46.0 |
| G5 | 40.5±6.7 | 41.0 |
| G6 | 34.9±8.1 | 33.0 |
| G7 | 35.9±5.2 | 34.0 |

**Table 22: Statistical Analysis of Survival Curves for Groups G8-G9**

| Group | Mean Survival Time (days) | Median Survival Time (days) |
|---|---|---|
| G8 | 24.9±3.2 | 24.0 |
| G9 | 47.0±0.0 | >47.0 |

The experiment found that 33 days after administration, tumors in animals inoculated with MC38-hPDL1 treated with T drug were all controlled, with tumor volumes all below 100 mm³. However, tumors in animals inoculated with MC38-hPDL1-hGDF15 treated with T drug all progressed, with a mean tumor volume of 800 mm³. There was a statistical difference between the two groups (P < 0.05), thereby proving that overexpression of hGDF15 indeed causes tumor resistance to PD-1.

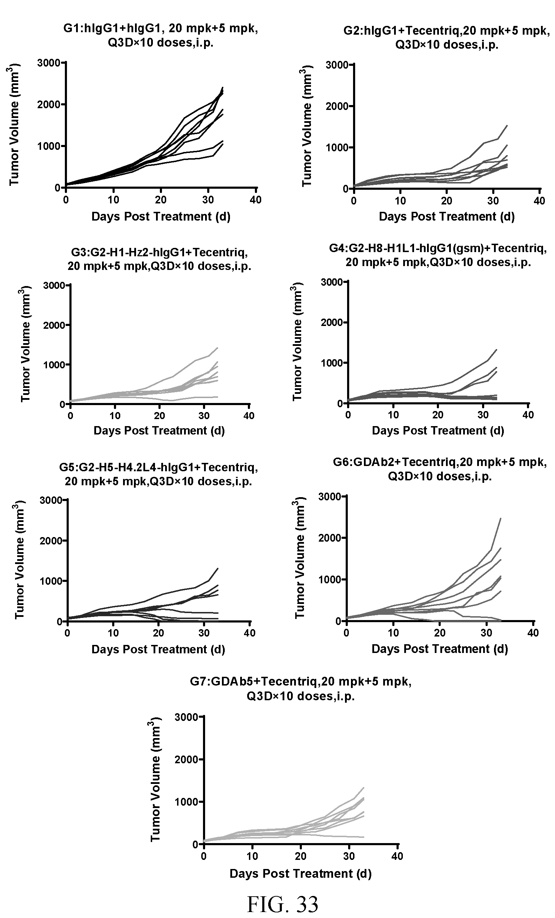

Using the anti-GDF15 antibody prepared in Example 3 of the present application in combination with T drug resulted in varying degrees of tumor volume reduction and regression in the animals. By day 33 after administration (the last time point when all animals in all groups were still alive), the TGI (tumor growth inhibition) rate for the T drug alone group (G2 group) was 57%. In the GDAb2 and T drug combination group (G6 group), 2 out of 8 animals showed complete regression, while the rest still progressed, with a TGI of 45%. In the G2-H5-H4.2L4-hIgG1 and T drug combination group (G5 group), 4 animals showed regression and reduction, with tumor volumes controlled below 200 mm³, and a TGI of 74%. In the G2-H8-H1L1-hIgG1(gsm) and T drug combination group (G4 group), 5 animals showed reduction and regression, with tumor volumes all below 200 mm³, and a TGI of 77%. In the G2-H1-hz2-hIgG1 and T drug combination group (G3 group), animal progression was similar to the trend in the T drug alone group, with a TGI of 57%. In the GDAb5 and T drug combination group (G7 group), 1 animal showed tumor reduction, with a TGI of 53%. The tumor volume and TGI results are shown in Figures 32-33. Therefore, the results indicated that the candidate antibodies G2-H8-H1L1-hIgG1(gsm) and G2-H5-H4.2L4-hIgG1 showed a better anti-tumor trend than GDAb2.

In the description of this specification, descriptions referring to the terms "one embodiment", "some embodiments", "example", "specific example", or "some examples" mean that specific features, structures, materials, or characteristics described in connection with the embodiment or example are included in at least one embodiment or example of the present application. In this specification, the schematic representations of the above terms do not necessarily refer to the same embodiment or example. Furthermore, the specific features, structures, materials, or characteristics described may be combined in any suitable manner in any one or more embodiments or examples. Moreover, a person skilled in the art may combine and assemble the different embodiments or examples and the features of the different embodiments or examples described in this specification, provided they are not mutually contradictory.

Although the embodiments of the present application have been shown and described above, it should be understood that the above embodiments are exemplary and are not to be construed as limitations of the present application. A person of ordinary skill in the art may make changes, modifications, substitutions, and alterations to the above embodiments within the scope of the present application.

## Claims

1. An antibody or antigen-binding fragment thereof, comprising:
a CDR sequence selected from the group consisting of at least one of the following or an amino acid sequence having at least 66% identity thereto:
heavy chain variable region CDR sequences: SEQ ID NOs: 1 to 34 and SEQ ID NOs: 74 to 76;
light chain variable region CDR sequences: SEQ ID NOs: 36 to 73 and SEQ ID NOs: 77 to 82.

2. The antibody or antigen-binding fragment thereof according to claim 1, wherein the antibody comprises:
a heavy chain variable region CDR1 selected from the group consisting of the amino acid as set forth in any one of SEQ ID NO: 1, SEQ ID NO: 4, SEQ ID NO: 7, SEQ ID NO: 9, SEQ ID NO: 12, SEQ ID NO: 15, SEQ ID NO: 23, and SEQ ID NO: 32;
a heavy chain variable region CDR2 selected from the group consisting of the amino acid as set forth in any one of SEQ ID NO: 2, SEQ ID NO: 5, SEQ ID NO: 10, SEQ ID NO: 13, SEQ ID NO: 16, SEQ ID NO: 17, SEQ ID NO: 19, SEQ ID NO: 21, SEQ ID NO: 24, SEQ ID NO: 26, SEQ ID NO: 28, SEQ ID NO: 33, SEQ ID NO: 74, SEQ ID NO: 75, and SEQ ID NO: 76;
a heavy chain variable region CDR3 selected from the group consisting of the amino acid as set forth in any one of SEQ ID NO: 3, SEQ ID NO: 6, SEQ ID NO: 8, SEQ ID NO: 11, SEQ ID NO: 14, SEQ ID NO: 18, SEQ ID NO: 20, SEQ ID NO: 22, SEQ ID NO: 25, SEQ ID NO: 27, SEQ ID NO: 29, SEQ ID NO: 30, SEQ ID NO: 31, and SEQ ID NO: 34;
a light chain variable region CDR1 selected from the group consisting of the amino acid as set forth in any one of SEQ ID NO: 36, SEQ ID NO: 39, SEQ ID NO: 43, SEQ ID NO: 46, SEQ ID NO: 49, SEQ ID NO: 52, SEQ ID NO: 55, SEQ ID NO: 58, SEQ ID NO: 63, SEQ ID NO: 66, SEQ ID NO: 68, SEQ ID NO: 70, SEQ ID NO: 73, SEQ ID NO: 77, SEQ ID NO: 78, and SEQ ID NO: 79;
a light chain variable region CDR2 selected from the group consisting of the amino acid as set forth in any one of SEQ ID NO: 37, SEQ ID NO: 40, SEQ ID NO: 44, SEQ ID NO: 47, SEQ ID NO: 50, SEQ ID NO: 53, SEQ ID NO: 56, SEQ ID NO: 59, SEQ ID NO: 61, SEQ ID NO: 64, and SEQ ID NO: 71; or
a light chain variable region CDR3 selected from the group consisting of the amino acid as set forth in any one of SEQ ID NO: 38, SEQ ID NO: 41, SEQ ID NO: 42, SEQ ID NO: 45, SEQ ID NO: 48, SEQ ID NO: 51, SEQ ID NO: 54, SEQ ID NO: 57, SEQ ID NO: 60, SEQ ID NO: 62, SEQ ID NO: 65, SEQ ID NO: 67, SEQ ID NO: 69, SEQ ID NO: 72, SEQ ID NO: 80, SEQ ID NO: 81, and SEQ ID NO: 82.

3. The antibody or antigen-binding fragment thereof according to claim 1 or 2, wherein the antibody comprises:
heavy chain variable region CDR1, CDR2, and CDR3 sequences as set forth in the amino acid sequences of SEQ ID NOs: 1, 17, and 18, respectively; or
heavy chain variable region CDR1, CDR2, and CDR3 sequences as set forth in the amino acid sequences of SEQ ID NOs: 1, 2, and 3, respectively; or
heavy chain variable region CDR1, CDR2, and CDR3 sequences as set forth in the amino acid sequences of SEQ ID NOs: 9, 75, and 11, respectively; or
heavy chain variable region CDR1, CDR2, and CDR3 sequences as set forth in the amino acid sequences of SEQ ID NOs: 4, 5, and 6, respectively; or
heavy chain variable region CDR1, CDR2, and CDR3 sequences as set forth in the amino acid sequences of SEQ ID NOs: 7, 5, and 8, respectively; or
heavy chain variable region CDR1, CDR2, and CDR3 sequences as set forth in the amino acid sequences of SEQ ID NOs: 9, 10, and 11, respectively; or
heavy chain variable region CDR1, CDR2, and CDR3 sequences as set forth in the amino acid sequences of SEQ ID NOs: 12, 13, and 14, respectively; or
heavy chain variable region CDR1, CDR2, and CDR3 sequences as set forth in the amino acid sequences of SEQ ID NOs: 15, 16, and 14, respectively; or
heavy chain variable region CDR1, CDR2, and CDR3 sequences as set forth in the amino acid sequences of SEQ ID NOs: 15, 19, and 20, respectively; or
heavy chain variable region CDR1, CDR2, and CDR3 sequences as set forth in the amino acid sequences of SEQ ID NOs: 4, 21, and 22, respectively; or
heavy chain variable region CDR1, CDR2, and CDR3 sequences as set forth in the amino acid sequences of SEQ ID NOs: 23, 24, and 25, respectively; or
heavy chain variable region CDR1, CDR2, and CDR3 sequences as set forth in the amino acid sequences of SEQ ID NOs: 12, 26, and 27, respectively; or
heavy chain variable region CDR1, CDR2, and CDR3 sequences as set forth in the amino acid sequences of SEQ ID NOs: 7, 28, and 29, respectively; or
heavy chain variable region CDR1, CDR2, and CDR3 sequences as set forth in the amino acid sequences of SEQ ID NOs: 7, 5, and 30, respectively; or
heavy chain variable region CDR1, CDR2, and CDR3 sequences as set forth in the amino acid sequences of SEQ ID NOs: 7, 5, and 31, respectively; or
heavy chain variable region CDR1, CDR2, and CDR3 sequences as set forth in the amino acid sequences of SEQ ID NOs: 32, 33, and 34, respectively; or
heavy chain variable region CDR1, CDR2, and CDR3 sequences as set forth in the amino acid sequences of SEQ ID NOs: 9, 74, and 11, respectively; or
heavy chain variable region CDR1, CDR2, and CDR3 sequences as set forth in the amino acid sequences of SEQ ID NOs: 9, 76, and 11, respectively;
optionally, the antibody comprises:
light chain variable region CDR1, CDR2, and CDR3 sequences as set forth in the amino acid sequences of SEQ ID NOs: 73, 53, and 54, respectively; or
light chain variable region CDR1, CDR2, and CDR3 sequences as set forth in the amino acid sequences of SEQ ID NOs: 36, 37, and 38, respectively; or
light chain variable region CDR1, CDR2, and CDR3 sequences as set forth in the amino acid sequences of SEQ ID NOs: 43, 44, and 45, respectively; or
light chain variable region CDR1, CDR2, and CDR3 sequences as set forth in the amino acid sequences of SEQ ID NOs: 39, 40, and 41, respectively; or
light chain variable region CDR1, CDR2, and CDR3 sequences as set forth in the amino acid sequences of SEQ ID NOs: 39, 40, and 42, respectively; or
light chain variable region CDR1, CDR2, and CDR3 sequences as set forth in the amino acid sequences of SEQ ID NOs: 46, 47, and 48, respectively; or
light chain variable region CDR1, CDR2, and CDR3 sequences as set forth in the amino acid sequences of SEQ ID NOs: 49, 50, and 51, respectively; or
light chain variable region CDR1, CDR2, and CDR3 sequences as set forth in the amino acid sequences of SEQ ID NOs: 52, 53, and 54, respectively; or
light chain variable region CDR1, CDR2, and CDR3 sequences as set forth in the amino acid sequences of SEQ ID NOs: 49, 47, and 51, respectively; or
light chain variable region CDR1, CDR2, and CDR3 sequences as set forth in the amino acid sequences of SEQ ID NOs: 55, 56, and 57, respectively; or
light chain variable region CDR1, CDR2, and CDR3 sequences as set forth in the amino acid sequences of SEQ ID NOs: 58, 59, and 60, respectively; or
light chain variable region CDR1, CDR2, and CDR3 sequences as set forth in the amino acid sequences of SEQ ID NOs: 49, 61, and 62, respectively; or
light chain variable region CDR1, CDR2, and CDR3 sequences as set forth in the amino acid sequences of SEQ ID NOs: 63, 64, and 65, respectively; or
light chain variable region CDR1, CDR2, and CDR3 sequences as set forth in the amino acid sequences of SEQ ID NOs: 66, 40, and 67, respectively; or
light chain variable region CDR1, CDR2, and CDR3 sequences as set forth in the amino acid sequences of SEQ ID NOs: 68, 40, and 69, respectively; or
light chain variable region CDR1, CDR2, and CDR3 sequences as set forth in the amino acid sequences of SEQ ID NOs: 70, 71, and 72, respectively; or
light chain variable region CDR1, CDR2, and CDR3 sequences as set forth in the amino acid sequences of SEQ ID NOs: 77, 53, and 54, respectively; or
light chain variable region CDR1, CDR2, and CDR3 sequences as set forth in the amino acid sequences of SEQ ID NOs: 78, 53, and 54, respectively; or
light chain variable region CDR1, CDR2, and CDR3 sequences as set forth in the amino acid sequences of SEQ ID NOs: 79, 53, and 54, respectively; or
light chain variable region CDR1, CDR2, and CDR3 sequences as set forth in the amino acid sequences of SEQ ID NOs: 39, 40, and 80, respectively; or
light chain variable region CDR1, CDR2, and CDR3 sequences as set forth in the amino acid sequences of SEQ ID NOs: 39, 40, and 81, respectively; or
light chain variable region CDR1, CDR2, and CDR3 sequences as set forth in the amino acid sequences of SEQ ID NOs: 39, 40, and 82, respectively.

4. The antibody or antigen-binding fragment thereof according to any one of claims 1 to 3, wherein the antibody comprises:
heavy chain variable region CDR1, CDR2, and CDR3 sequences as set forth in the amino acid sequences of SEQ ID NOs: 1, 17, and 18, respectively, and light chain variable region CDR1, CDR2, and CDR3 sequences as set forth in the amino acid sequences of SEQ ID NOs: 73, 53, and 54, respectively; or
heavy chain variable region CDR1, CDR2, and CDR3 sequences as set forth in the amino acid sequences of SEQ ID NOs: 1, 2, and 3, respectively, and light chain variable region CDR1, CDR2, and CDR3 sequences as set forth in the amino acid sequences of SEQ ID NOs: 36, 37, and 38, respectively; or
heavy chain variable region CDR1, CDR2, and CDR3 sequences as set forth in the amino acid sequences of SEQ ID NOs: 9, 75, and 11, respectively, and light chain variable region CDR1, CDR2, and CDR3 sequences as set forth in the amino acid sequences of SEQ ID NOs: 43, 44, and 45, respectively; or
heavy chain variable region CDR1, CDR2, and CDR3 sequences as set forth in the amino acid sequences of SEQ ID NOs: 4, 5, and 6, respectively, and light chain variable region CDR1, CDR2, and CDR3 sequences as set forth in the amino acid sequences of SEQ ID NOs: 39, 40, and 41, respectively; or
heavy chain variable region CDR1, CDR2, and CDR3 sequences as set forth in the amino acid sequences of SEQ ID NOs: 7, 5, and 8, respectively, and light chain variable region CDR1, CDR2, and CDR3 sequences as set forth in the amino acid sequences of SEQ ID NOs: 39, 40, and 42, respectively; or
heavy chain variable region CDR1, CDR2, and CDR3 sequences as set forth in the amino acid sequences of SEQ ID NOs: 9, 10, and 11, respectively, and light chain variable region CDR1, CDR2, and CDR3 sequences as set forth in the amino acid sequences of SEQ ID NOs: 43, 44, and 45, respectively; or
heavy chain variable region CDR1, CDR2, and CDR3 sequences as set forth in the amino acid sequences of SEQ ID NOs: 12, 13, and 14, respectively, and light chain variable region CDR1, CDR2, and CDR3 sequences as set forth in the amino acid sequences of SEQ ID NOs: 46, 47, and 48, respectively; or
heavy chain variable region CDR1, CDR2, and CDR3 sequences as set forth in the amino acid sequences of SEQ ID NOs: 15, 16, and 14, respectively, and light chain variable region CDR1, CDR2, and CDR3 sequences as set forth in the amino acid sequences of SEQ ID NOs: 49, 50, and 51, respectively; or
heavy chain variable region CDR1, CDR2, and CDR3 sequences as set forth in the amino acid sequences of SEQ ID NOs: 1, 17, and 18, respectively, and light chain variable region CDR1, CDR2, and CDR3 sequences as set forth in the amino acid sequences of SEQ ID NOs: 52, 53, and 54, respectively; or
heavy chain variable region CDR1, CDR2, and CDR3 sequences as set forth in the amino acid sequences of SEQ ID NOs: 15, 19, and 20, respectively, and light chain variable region CDR1, CDR2, and CDR3 sequences as set forth in the amino acid sequences of SEQ ID NOs: 49, 47, and 51, respectively; or
heavy chain variable region CDR1, CDR2, and CDR3 sequences as set forth in the amino acid sequences of SEQ ID NOs: 4, 21, and 22, respectively, and light chain variable region CDR1, CDR2, and CDR3 sequences as set forth in the amino acid sequences of SEQ ID NOs: 55, 56, and 57, respectively; or
heavy chain variable region CDR1, CDR2, and CDR3 sequences as set forth in the amino acid sequences of SEQ ID NOs: 23, 24, and 25, respectively, and light chain variable region CDR1, CDR2, and CDR3 sequences as set forth in the amino acid sequences of SEQ ID NOs: 58, 59, and 60, respectively; or
heavy chain variable region CDR1, CDR2, and CDR3 sequences as set forth in the amino acid sequences of SEQ ID NOs: 12, 26, and 27, respectively, and light chain variable region CDR1, CDR2, and CDR3 sequences as set forth in the amino acid sequences of SEQ ID NOs: 49, 61, and 62, respectively; or
heavy chain variable region CDR1, CDR2, and CDR3 sequences as set forth in the amino acid sequences of SEQ ID NOs: 7, 28, and 29, respectively, and light chain variable region CDR1, CDR2, and CDR3 sequences as set forth in the amino acid sequences of SEQ ID NOs: 63, 64, and 65, respectively; or
heavy chain variable region CDR1, CDR2, and CDR3 sequences as set forth in the amino acid sequences of SEQ ID NOs: 7, 5, and 30, respectively, and light chain variable region CDR1, CDR2, and CDR3 sequences as set forth in the amino acid sequences of SEQ ID NOs: 66, 40, and 67, respectively; or
heavy chain variable region CDR1, CDR2, and CDR3 sequences as set forth in the amino acid sequences of SEQ ID NOs: 7, 5, and 31, respectively, and light chain variable region CDR1, CDR2, and CDR3 sequences as set forth in the amino acid sequences of SEQ ID NOs: 68, 40, and 69, respectively; or
heavy chain variable region CDR1, CDR2, and CDR3 sequences as set forth in the amino acid sequences of SEQ ID NOs: 32, 33, and 34, respectively, and light chain variable region CDR1, CDR2, and CDR3 sequences as set forth in the amino acid sequences of SEQ ID NOs: 70, 71, and 72, respectively; or
heavy chain variable region CDR1, CDR2, and CDR3 sequences as set forth in the amino acid sequences of SEQ ID NOs: 9, 74, and 11, respectively, and light chain variable region CDR1, CDR2, and CDR3 sequences as set forth in the amino acid sequences of SEQ ID NOs: 43, 44, and 45, respectively; or
heavy chain variable region CDR1, CDR2, and CDR3 sequences as set forth in the amino acid sequences of SEQ ID NOs: 9, 76, and 11, respectively, and light chain variable region CDR1, CDR2, and CDR3 sequences as set forth in the amino acid sequences of SEQ ID NOs: 43, 44, and 45, respectively; or
heavy chain variable region CDR1, CDR2, and CDR3 sequences as set forth in the amino acid sequences of SEQ ID NOs: 1, 17, and 18, respectively, and light chain variable region CDR1, CDR2, and CDR3 sequences as set forth in the amino acid sequences of SEQ ID NOs: 77, 53, and 54, respectively; or
heavy chain variable region CDR1, CDR2, and CDR3 sequences as set forth in the amino acid sequences of SEQ ID NOs: 1, 17, and 18, respectively, and light chain variable region CDR1, CDR2, and CDR3 sequences as set forth in the amino acid sequences of SEQ ID NOs: 78, 53, and 54, respectively; or
heavy chain variable region CDR1, CDR2, and CDR3 sequences as set forth in the amino acid sequences of SEQ ID NOs: 1, 17, and 18, respectively, and light chain variable region CDR1, CDR2, and CDR3 sequences as set forth in the amino acid sequences of SEQ ID NOs: 79, 53, and 54, respectively; or
heavy chain variable region CDR1, CDR2, and CDR3 sequences as set forth in the amino acid sequences of SEQ ID NOs: 4, 5, and 6, respectively, and light chain variable region CDR1, CDR2, and CDR3 sequences as set forth in the amino acid sequences of SEQ ID NOs: 39, 40, and 80, respectively; or
heavy chain variable region CDR1, CDR2, and CDR3 sequences as set forth in the amino acid sequences of SEQ ID NOs: 4, 5, and 6, respectively, and light chain variable region CDR1, CDR2, and CDR3 sequences as set forth in the amino acid sequences of SEQ ID NOs: 39, 40, and 81, respectively; or
heavy chain variable region CDR1, CDR2, and CDR3 sequences as set forth in the amino acid sequences of SEQ ID NOs: 4, 5, and 6, respectively, and light chain variable region CDR1, CDR2, and CDR3 sequences as set forth in the amino acid sequences of SEQ ID NOs: 39, 40, and 82, respectively; or
optionally, the antibody or antigen-binding fragment thereof specifically recognizes GDF15.

5. The antibody or antigen-binding fragment thereof according to any one of claims 1 to 4, wherein the antibody further comprises:
at least one of a heavy chain framework region sequence and a light chain framework region sequence;
wherein at least a portion of at least one of the heavy chain framework region sequence and the light chain framework region sequence is derived from at least one of a murine antibody, a human antibody, a primate antibody, or a mutant thereof;
optionally, at least a portion of the heavy chain framework region sequence and the light chain framework region sequence is derived from a human antibody or a mutant thereof.

6. The antibody or antigen-binding fragment thereof according to any one of claims 1 to 5, wherein the antibody has a heavy chain variable region of the amino acid sequence as set forth in any one of SEQ ID NOs: 83 to 100, SEQ ID NOs: 124 to 127, SEQ ID NOs: 132 to 135, SEQ ID NOs: 140 to 141, and SEQ ID NOs: 144 to 145;
optionally, the antibody has a light chain variable region of the amino acid sequence as set forth in any one of SEQ ID NOs: 102 to 122, SEQ ID NOs: 128 to 131, SEQ ID NOs: 136 to 139, SEQ ID NOs: 142 to 143, and SEQ ID NOs: 146 to 147.

7. The antibody or antigen-binding fragment thereof according to claim 6, wherein the antibody has
a heavy chain variable region of the amino acid sequence as set forth in SEQ ID NO: 144 and a light chain variable region of the amino acid sequence as set forth in SEQ ID NO: 146;
optionally, the antibody has a heavy chain variable region of the amino acid sequence as set forth in SEQ ID NO: 124 and a light chain variable region of the amino acid sequence as set forth in SEQ ID NO: 129;
optionally, the antibody has a heavy chain variable region of the amino acid sequence as set forth in SEQ ID NO: 135 and a light chain variable region of the amino acid sequence as set forth in SEQ ID NO: 139;
optionally, the antibody has a heavy chain variable region of the amino acid sequence as set forth in SEQ ID NO: 83 and a light chain variable region of the amino acid sequence as set forth in SEQ ID NO: 102;
optionally, the antibody has a heavy chain variable region of the amino acid sequence as set forth in SEQ ID NO: 84 and a light chain variable region of the amino acid sequence as set forth in SEQ ID NO: 103;
optionally, the antibody has a heavy chain variable region of the amino acid sequence as set forth in SEQ ID NO: 85 and a light chain variable region of the amino acid sequence as set forth in SEQ ID NO: 104;
optionally, the antibody has a heavy chain variable region of the amino acid sequence as set forth in SEQ ID NO: 86 and a light chain variable region of the amino acid sequence as set forth in SEQ ID NO: 105;
optionally, the antibody has a heavy chain variable region of the amino acid sequence as set forth in SEQ ID NO: 87 and a light chain variable region of the amino acid sequence as set forth in SEQ ID NO: 106;
optionally, the antibody has a heavy chain variable region of the amino acid sequence as set forth in SEQ ID NO: 88 and a light chain variable region of the amino acid sequence as set forth in SEQ ID NO: 107;
optionally, the antibody has a heavy chain variable region of the amino acid sequence as set forth in SEQ ID NO: 89 and a light chain variable region of the amino acid sequence as set forth in SEQ ID NO: 108;
optionally, the antibody has a heavy chain variable region of the amino acid sequence as set forth in SEQ ID NO: 90 and a light chain variable region of the amino acid sequence as set forth in SEQ ID NO: 109;
optionally, the antibody has a heavy chain variable region of the amino acid sequence as set forth in SEQ ID NO: 91 and a light chain variable region of the amino acid sequence as set forth in SEQ ID NO: 110;
optionally, the antibody has a heavy chain variable region of the amino acid sequence as set forth in SEQ ID NO: 92 and a light chain variable region of the amino acid sequence as set forth in SEQ ID NO: 111;
optionally, the antibody has a heavy chain variable region of the amino acid sequence as set forth in SEQ ID NO: 93 and a light chain variable region of the amino acid sequence as set forth in SEQ ID NO: 112;
optionally, the antibody has a heavy chain variable region of the amino acid sequence as set forth in SEQ ID NO: 94 and a light chain variable region of the amino acid sequence as set forth in SEQ ID NO: 113;
optionally, the antibody has a heavy chain variable region of the amino acid sequence as set forth in SEQ ID NO: 95 and a light chain variable region of the amino acid sequence as set forth in SEQ ID NO: 114;
optionally, the antibody has a heavy chain variable region of the amino acid sequence as set forth in SEQ ID NO: 96 and a light chain variable region of the amino acid sequence as set forth in SEQ ID NO: 115;
optionally, the antibody has a heavy chain variable region of the amino acid sequence as set forth in SEQ ID NO: 97 and a light chain variable region of the amino acid sequence as set forth in SEQ ID NO: 116;
optionally, the antibody has a heavy chain variable region of the amino acid sequence as set forth in SEQ ID NO: 98 and a light chain variable region of the amino acid sequence as set forth in SEQ ID NO: 105;
optionally, the antibody has a heavy chain variable region of the amino acid sequence as set forth in SEQ ID NO: 99 and a light chain variable region of the amino acid sequence as set forth in SEQ ID NO: 105;
optionally, the antibody has a heavy chain variable region of the amino acid sequence as set forth in SEQ ID NO: 100 and a light chain variable region of the amino acid sequence as set forth in SEQ ID NO: 105;
optionally, the antibody has a heavy chain variable region of the amino acid sequence as set forth in SEQ ID NO: 89 and a light chain variable region of the amino acid sequence as set forth in SEQ ID NO: 117;
optionally, the antibody has a heavy chain variable region of the amino acid sequence as set forth in SEQ ID NO: 89 and a light chain variable region of the amino acid sequence as set forth in SEQ ID NO: 118;
optionally, the antibody has a heavy chain variable region of the amino acid sequence as set forth in SEQ ID NO: 89 and a light chain variable region of the amino acid sequence as set forth in SEQ ID NO: 119;
optionally, the antibody has a heavy chain variable region of the amino acid sequence as set forth in SEQ ID NO: 84 and a light chain variable region of the amino acid sequence as set forth in SEQ ID NO: 120;
optionally, the antibody has a heavy chain variable region of the amino acid sequence as set forth in SEQ ID NO: 84 and a light chain variable region of the amino acid sequence as set forth in SEQ ID NO: 121;
optionally, the antibody has a heavy chain variable region of the amino acid sequence as set forth in SEQ ID NO: 84 and a light chain variable region of the amino acid sequence as set forth in SEQ ID NO: 122;
optionally, the antibody has a heavy chain variable region of the amino acid sequence as set forth in SEQ ID NO: 124 and a light chain variable region of the amino acid sequence as set forth in SEQ ID NO: 128;
optionally, the antibody has a heavy chain variable region of the amino acid sequence as set forth in SEQ ID NO: 124 and a light chain variable region of the amino acid sequence as set forth in SEQ ID NO: 130;
optionally, the antibody has a heavy chain variable region of the amino acid sequence as set forth in SEQ ID NO: 124 and a light chain variable region of the amino acid sequence as set forth in SEQ ID NO: 131;
optionally, the antibody has a heavy chain variable region of the amino acid sequence as set forth in SEQ ID NO: 125 and a light chain variable region of the amino acid sequence as set forth in SEQ ID NO: 128;
optionally, the antibody has a heavy chain variable region of the amino acid sequence as set forth in SEQ ID NO: 125 and a light chain variable region of the amino acid sequence as set forth in SEQ ID NO: 129;
optionally, the antibody has a heavy chain variable region of the amino acid sequence as set forth in SEQ ID NO: 125 and a light chain variable region of the amino acid sequence as set forth in SEQ ID NO: 130;
optionally, the antibody has a heavy chain variable region of the amino acid sequence as set forth in SEQ ID NO: 125 and a light chain variable region of the amino acid sequence as set forth in SEQ ID NO: 131;
optionally, the antibody has a heavy chain variable region of the amino acid sequence as set forth in SEQ ID NO: 126 and a light chain variable region of the amino acid sequence as set forth in SEQ ID NO: 128;
optionally, the antibody has a heavy chain variable region of the amino acid sequence as set forth in SEQ ID NO: 126 and a light chain variable region of the amino acid sequence as set forth in SEQ ID NO: 129;
optionally, the antibody has a heavy chain variable region of the amino acid sequence as set forth in SEQ ID NO: 126 and a light chain variable region of the amino acid sequence as set forth in SEQ ID NO: 130;
optionally, the antibody has a heavy chain variable region of the amino acid sequence as set forth in SEQ ID NO: 126 and a light chain variable region of the amino acid sequence as set forth in SEQ ID NO: 131;
optionally, the antibody has a heavy chain variable region of the amino acid sequence as set forth in SEQ ID NO: 127 and a light chain variable region of the amino acid sequence as set forth in SEQ ID NO: 128;
optionally, the antibody has a heavy chain variable region of the amino acid sequence as set forth in SEQ ID NO: 127 and a light chain variable region of the amino acid sequence as set forth in SEQ ID NO: 129;
optionally, the antibody has a heavy chain variable region of the amino acid sequence as set forth in SEQ ID NO: 127 and a light chain variable region of the amino acid sequence as set forth in SEQ ID NO: 130;
optionally, the antibody has a heavy chain variable region of the amino acid sequence as set forth in SEQ ID NO: 127 and a light chain variable region of the amino acid sequence as set forth in SEQ ID NO: 131;
optionally, the antibody has a heavy chain variable region of the amino acid sequence as set forth in SEQ ID NO: 132 and a light chain variable region of the amino acid sequence as set forth in SEQ ID NO: 136;
optionally, the antibody has a heavy chain variable region of the amino acid sequence as set forth in SEQ ID NO: 133 and a light chain variable region of the amino acid sequence as set forth in SEQ ID NO: 136;
optionally, the antibody has a heavy chain variable region of the amino acid sequence as set forth in SEQ ID NO: 134 and a light chain variable region of the amino acid sequence as set forth in SEQ ID NO: 136;
optionally, the antibody has a heavy chain variable region of the amino acid sequence as set forth in SEQ ID NO: 135 and a light chain variable region of the amino acid sequence as set forth in SEQ ID NO: 136;
optionally, the antibody has a heavy chain variable region of the amino acid sequence as set forth in SEQ ID NO: 132 and a light chain variable region of the amino acid sequence as set forth in SEQ ID NO: 137;
optionally, the antibody has a heavy chain variable region of the amino acid sequence as set forth in SEQ ID NO: 133 and a light chain variable region of the amino acid sequence as set forth in SEQ ID NO: 137;
optionally, the antibody has a heavy chain variable region of the amino acid sequence as set forth in SEQ ID NO: 134 and a light chain variable region of the amino acid sequence as set forth in SEQ ID NO: 137;
optionally, the antibody has a heavy chain variable region of the amino acid sequence as set forth in SEQ ID NO: 135 and a light chain variable region of the amino acid sequence as set forth in SEQ ID NO: 137;
optionally, the antibody has a heavy chain variable region of the amino acid sequence as set forth in SEQ ID NO: 132 and a light chain variable region of the amino acid sequence as set forth in SEQ ID NO: 138;
optionally, the antibody has a heavy chain variable region of the amino acid sequence as set forth in SEQ ID NO: 133 and a light chain variable region of the amino acid sequence as set forth in SEQ ID NO: 138;
optionally, the antibody has a heavy chain variable region of the amino acid sequence as set forth in SEQ ID NO: 134 and a light chain variable region of the amino acid sequence as set forth in SEQ ID NO: 138;
optionally, the antibody has a heavy chain variable region of the amino acid sequence as set forth in SEQ ID NO: 135 and a light chain variable region of the amino acid sequence as set forth in SEQ ID NO: 138;
optionally, the antibody has a heavy chain variable region of the amino acid sequence as set forth in SEQ ID NO: 132 and a light chain variable region of the amino acid sequence as set forth in SEQ ID NO: 139;
optionally, the antibody has a heavy chain variable region of the amino acid sequence as set forth in SEQ ID NO: 133 and a light chain variable region of the amino acid sequence as set forth in SEQ ID NO: 139;
optionally, the antibody has a heavy chain variable region of the amino acid sequence as set forth in SEQ ID NO: 134 and a light chain variable region of the amino acid sequence as set forth in SEQ ID NO: 139;
optionally, the antibody has a heavy chain variable region of the amino acid sequence as set forth in SEQ ID NO: 140 and a light chain variable region of the amino acid sequence as set forth in SEQ ID NO: 142;
optionally, the antibody has a heavy chain variable region of the amino acid sequence as set forth in SEQ ID NO: 141 and a light chain variable region of the amino acid sequence as set forth in SEQ ID NO: 142;
optionally, the antibody has a heavy chain variable region of the amino acid sequence as set forth in SEQ ID NO: 140 and a light chain variable region of the amino acid sequence as set forth in SEQ ID NO: 143;
optionally, the antibody has a heavy chain variable region of the amino acid sequence as set forth in SEQ ID NO: 141 and a light chain variable region of the amino acid sequence as set forth in SEQ ID NO: 143;
optionally, the antibody has a heavy chain variable region of the amino acid sequence as set forth in SEQ ID NO: 144 and a light chain variable region of the amino acid sequence as set forth in SEQ ID NO: 147;
optionally, the antibody has a heavy chain variable region of the amino acid sequence as set forth in SEQ ID NO: 145 and a light chain variable region of the amino acid sequence as set forth in SEQ ID NO: 146;
optionally, the antibody has a heavy chain variable region of the amino acid sequence as set forth in SEQ ID NO: 145 and a light chain variable region of the amino acid sequence as set forth in SEQ ID NO: 147.

8. An antibody or antigen-binding fragment thereof, comprising a heavy chain variable region and a light chain variable region, wherein the antibody comprises three heavy chain variable region CDRs from a heavy chain variable region of the amino acid sequence as set forth in SEQ ID NO: 144, and three light chain variable region CDRs from a light chain variable region of the amino acid sequence as set forth in SEQ ID NO: 146;
optionally, the antibody comprises three heavy chain variable region CDRs from a heavy chain variable region of the amino acid sequence as set forth in SEQ ID NO: 124, and three light chain variable region CDRs from a light chain variable region of the amino acid sequence as set forth in SEQ ID NO: 129;
optionally, the antibody comprises three heavy chain variable region CDRs from a heavy chain variable region of the amino acid sequence as set forth in SEQ ID NO: 135, and three light chain variable region CDRs from a light chain variable region of the amino acid sequence as set forth in SEQ ID NO: 139;
optionally, the antibody comprises three heavy chain variable region CDRs from a heavy chain variable region of the amino acid sequence as set forth in SEQ ID NO: 83, and three light chain variable region CDRs from a light chain variable region of the amino acid sequence as set forth in SEQ ID NO: 102;
optionally, the antibody comprises three heavy chain variable region CDRs from a heavy chain variable region of the amino acid sequence as set forth in SEQ ID NO: 84, and three light chain variable region CDRs from a light chain variable region of the amino acid sequence as set forth in SEQ ID NO: 103;
optionally, the antibody comprises three heavy chain variable region CDRs from a heavy chain variable region of the amino acid sequence as set forth in SEQ ID NO: 85, and three light chain variable region CDRs from a light chain variable region of the amino acid sequence as set forth in SEQ ID NO: 104;
optionally, the antibody comprises three heavy chain variable region CDRs from a heavy chain variable region of the amino acid sequence as set forth in SEQ ID NO: 86, and three light chain variable region CDRs from a light chain variable region of the amino acid sequence as set forth in SEQ ID NO: 105;
optionally, the antibody comprises three heavy chain variable region CDRs from a heavy chain variable region of the amino acid sequence as set forth in SEQ ID NO: 87, and three light chain variable region CDRs from a light chain variable region of the amino acid sequence as set forth in SEQ ID NO: 106;
optionally, the antibody comprises three heavy chain variable region CDRs from a heavy chain variable region of the amino acid sequence as set forth in SEQ ID NO: 88, and three light chain variable region CDRs from a light chain variable region of the amino acid sequence as set forth in SEQ ID NO: 107;
optionally, the antibody comprises three heavy chain variable region CDRs from a heavy chain variable region of the amino acid sequence as set forth in SEQ ID NO: 89, and three light chain variable region CDRs from a light chain variable region of the amino acid sequence as set forth in SEQ ID NO: 108;
optionally, the antibody comprises three heavy chain variable region CDRs from a heavy chain variable region of the amino acid sequence as set forth in SEQ ID NO: 90, and three light chain variable region CDRs from a light chain variable region of the amino acid sequence as set forth in SEQ ID NO: 109;
optionally, the antibody comprises three heavy chain variable region CDRs from a heavy chain variable region of the amino acid sequence as set forth in SEQ ID NO: 91, and three light chain variable region CDRs from a light chain variable region of the amino acid sequence as set forth in SEQ ID NO: 110;
optionally, the antibody comprises three heavy chain variable region CDRs from a heavy chain variable region of the amino acid sequence as set forth in SEQ ID NO: 92, and three light chain variable region CDRs from a light chain variable region of the amino acid sequence as set forth in SEQ ID NO: 111;
optionally, the antibody comprises three heavy chain variable region CDRs from a heavy chain variable region of the amino acid sequence as set forth in SEQ ID NO: 93, and three light chain variable region CDRs from a light chain variable region of the amino acid sequence as set forth in SEQ ID NO: 112;
optionally, the antibody comprises three heavy chain variable region CDRs from a heavy chain variable region of the amino acid sequence as set forth in SEQ ID NO: 94, and three light chain variable region CDRs from a light chain variable region of the amino acid sequence as set forth in SEQ ID NO: 113;
optionally, the antibody comprises three heavy chain variable region CDRs from a heavy chain variable region of the amino acid sequence as set forth in SEQ ID NO: 95, and three light chain variable region CDRs from a light chain variable region of the amino acid sequence as set forth in SEQ ID NO: 114;
optionally, the antibody comprises three heavy chain variable region CDRs from a heavy chain variable region of the amino acid sequence as set forth in SEQ ID NO: 96, and three light chain variable region CDRs from a light chain variable region of the amino acid sequence as set forth in SEQ ID NO: 115;
optionally, the antibody comprises three heavy chain variable region CDRs from a heavy chain variable region of the amino acid sequence as set forth in SEQ ID NO: 97, and three light chain variable region CDRs from a light chain variable region of the amino acid sequence as set forth in SEQ ID NO: 116;
optionally, the antibody comprises three heavy chain variable region CDRs from a heavy chain variable region of the amino acid sequence as set forth in SEQ ID NO: 98, and three light chain variable region CDRs from a light chain variable region of the amino acid sequence as set forth in SEQ ID NO: 105;
optionally, the antibody comprises three heavy chain variable region CDRs from a heavy chain variable region of the amino acid sequence as set forth in SEQ ID NO: 99, and three light chain variable region CDRs from a light chain variable region of the amino acid sequence as set forth in SEQ ID NO: 105;
optionally, the antibody comprises three heavy chain variable region CDRs from a heavy chain variable region of the amino acid sequence as set forth in SEQ ID NO: 100, and three light chain variable region CDRs from a light chain variable region of the amino acid sequence as set forth in SEQ ID NO: 105;
optionally, the antibody comprises three heavy chain variable region CDRs from a heavy chain variable region of the amino acid sequence as set forth in SEQ ID NO: 89, and three light chain variable region CDRs from a light chain variable region of the amino acid sequence as set forth in SEQ ID NO: 117;
optionally, the antibody comprises three heavy chain variable region CDRs from a heavy chain variable region of the amino acid sequence as set forth in SEQ ID NO: 89, and three light chain variable region CDRs from a light chain variable region of the amino acid sequence as set forth in SEQ ID NO: 118;
optionally, the antibody comprises three heavy chain variable region CDRs from a heavy chain variable region of the amino acid sequence as set forth in SEQ ID NO: 89, and three light chain variable region CDRs from a light chain variable region of the amino acid sequence as set forth in SEQ ID NO: 119;
optionally, the antibody comprises three heavy chain variable region CDRs from a heavy chain variable region of the amino acid sequence as set forth in SEQ ID NO: 84, and three light chain variable region CDRs from a light chain variable region of the amino acid sequence as set forth in SEQ ID NO: 120;
optionally, the antibody comprises three heavy chain variable region CDRs from a heavy chain variable region of the amino acid sequence as set forth in SEQ ID NO: 84, and three light chain variable region CDRs from a light chain variable region of the amino acid sequence as set forth in SEQ ID NO: 121;
optionally, the antibody comprises three heavy chain variable region CDRs from a heavy chain variable region of the amino acid sequence as set forth in SEQ ID NO: 84, and three light chain variable region CDRs from a light chain variable region of the amino acid sequence as set forth in SEQ ID NO: 122;
optionally, the antibody comprises three heavy chain variable region CDRs from a heavy chain variable region of the amino acid sequence as set forth in SEQ ID NO: 124, and three light chain variable region CDRs from a light chain variable region of the amino acid sequence as set forth in SEQ ID NO: 128;
optionally, the antibody comprises three heavy chain variable region CDRs from a heavy chain variable region of the amino acid sequence as set forth in SEQ ID NO: 124, and three light chain variable region CDRs from a light chain variable region of the amino acid sequence as set forth in SEQ ID NO: 130;
optionally, the antibody comprises three heavy chain variable region CDRs from a heavy chain variable region of the amino acid sequence as set forth in SEQ ID NO: 124, and three light chain variable region CDRs from a light chain variable region of the amino acid sequence as set forth in SEQ ID NO: 131;
optionally, the antibody comprises three heavy chain variable region CDRs from a heavy chain variable region of the amino acid sequence as set forth in SEQ ID NO: 125, and three light chain variable region CDRs from a light chain variable region of the amino acid sequence as set forth in SEQ ID NO: 128;
optionally, the antibody comprises three heavy chain variable region CDRs from a heavy chain variable region of the amino acid sequence as set forth in SEQ ID NO: 125, and three light chain variable region CDRs from a light chain variable region of the amino acid sequence as set forth in SEQ ID NO: 129;
optionally, the antibody comprises three heavy chain variable region CDRs from a heavy chain variable region of the amino acid sequence as set forth in SEQ ID NO: 125, and three light chain variable region CDRs from a light chain variable region of the amino acid sequence as set forth in SEQ ID NO: 130;
optionally, the antibody comprises three heavy chain variable region CDRs from a heavy chain variable region of the amino acid sequence as set forth in SEQ ID NO: 125, and three light chain variable region CDRs from a light chain variable region of the amino acid sequence as set forth in SEQ ID NO: 131;
optionally, the antibody comprises three heavy chain variable region CDRs from a heavy chain variable region of the amino acid sequence as set forth in SEQ ID NO: 126, and three light chain variable region CDRs from a light chain variable region of the amino acid sequence as set forth in SEQ ID NO: 128;
optionally, the antibody comprises three heavy chain variable region CDRs from a heavy chain variable region of the amino acid sequence as set forth in SEQ ID NO: 126, and three light chain variable region CDRs from a light chain variable region of the amino acid sequence as set forth in SEQ ID NO: 129;
optionally, the antibody comprises three heavy chain variable region CDRs from a heavy chain variable region of the amino acid sequence as set forth in SEQ ID NO: 126, and three light chain variable region CDRs from a light chain variable region of the amino acid sequence as set forth in SEQ ID NO: 130;
optionally, the antibody comprises three heavy chain variable region CDRs from a heavy chain variable region of the amino acid sequence as set forth in SEQ ID NO: 126, and three light chain variable region CDRs from a light chain variable region of the amino acid sequence as set forth in SEQ ID NO: 131;
optionally, the antibody comprises three heavy chain variable region CDRs from a heavy chain variable region of the amino acid sequence as set forth in SEQ ID NO: 127, and three light chain variable region CDRs from a light chain variable region of the amino acid sequence as set forth in SEQ ID NO: 128;
optionally, the antibody comprises three heavy chain variable region CDRs from a heavy chain variable region of the amino acid sequence as set forth in SEQ ID NO: 127, and three light chain variable region CDRs from a light chain variable region of the amino acid sequence as set forth in SEQ ID NO: 129;
optionally, the antibody comprises three heavy chain variable region CDRs from a heavy chain variable region of the amino acid sequence as set forth in SEQ ID NO: 127, and three light chain variable region CDRs from a light chain variable region of the amino acid sequence as set forth in SEQ ID NO: 130;
optionally, the antibody comprises three heavy chain variable region CDRs from a heavy chain variable region of the amino acid sequence as set forth in SEQ ID NO: 127, and three light chain variable region CDRs from a light chain variable region of the amino acid sequence as set forth in SEQ ID NO: 131;
optionally, the antibody comprises three heavy chain variable region CDRs from a heavy chain variable region of the amino acid sequence as set forth in SEQ ID NO: 132, and three light chain variable region CDRs from a light chain variable region of the amino acid sequence as set forth in SEQ ID NO: 136;
optionally, the antibody comprises three heavy chain variable region CDRs from a heavy chain variable region of the amino acid sequence as set forth in SEQ ID NO: 133, and three light chain variable region CDRs from a light chain variable region of the amino acid sequence as set forth in SEQ ID NO: 136;
optionally, the antibody comprises three heavy chain variable region CDRs from a heavy chain variable region of the amino acid sequence as set forth in SEQ ID NO: 134, and three light chain variable region CDRs from a light chain variable region of the amino acid sequence as set forth in SEQ ID NO: 136;
optionally, the antibody comprises three heavy chain variable region CDRs from a heavy chain variable region of the amino acid sequence as set forth in SEQ ID NO: 135, and three light chain variable region CDRs from a light chain variable region of the amino acid sequence as set forth in SEQ ID NO: 136;
optionally, the antibody comprises three heavy chain variable region CDRs from a heavy chain variable region of the amino acid sequence as set forth in SEQ ID NO: 132, and three light chain variable region CDRs from a light chain variable region of the amino acid sequence as set forth in SEQ ID NO: 137;
optionally, the antibody comprises three heavy chain variable region CDRs from a heavy chain variable region of the amino acid sequence as set forth in SEQ ID NO: 133, and three light chain variable region CDRs from a light chain variable region of the amino acid sequence as set forth in SEQ ID NO: 137;
optionally, the antibody comprises three heavy chain variable region CDRs from a heavy chain variable region of the amino acid sequence as set forth in SEQ ID NO: 134, and three light chain variable region CDRs from a light chain variable region of the amino acid sequence as set forth in SEQ ID NO: 137;
optionally, the antibody comprises three heavy chain variable region CDRs from a heavy chain variable region of the amino acid sequence as set forth in SEQ ID NO: 135, and three light chain variable region CDRs from a light chain variable region of the amino acid sequence as set forth in SEQ ID NO: 137;
optionally, the antibody comprises three heavy chain variable region CDRs from a heavy chain variable region of the amino acid sequence as set forth in SEQ ID NO: 132, and three light chain variable region CDRs from a light chain variable region of the amino acid sequence as set forth in SEQ ID NO: 138;
optionally, the antibody comprises three heavy chain variable region CDRs from a heavy chain variable region of the amino acid sequence as set forth in SEQ ID NO: 133, and three light chain variable region CDRs from a light chain variable region of the amino acid sequence as set forth in SEQ ID NO: 138;
optionally, the antibody comprises three heavy chain variable region CDRs from a heavy chain variable region of the amino acid sequence as set forth in SEQ ID NO: 134, and three light chain variable region CDRs from a light chain variable region of the amino acid sequence as set forth in SEQ ID NO: 138;
optionally, the antibody comprises three heavy chain variable region CDRs from a heavy chain variable region of the amino acid sequence as set forth in SEQ ID NO: 135, and three light chain variable region CDRs from a light chain variable region of the amino acid sequence as set forth in SEQ ID NO: 138;
optionally, the antibody comprises three heavy chain variable region CDRs from a heavy chain variable region of the amino acid sequence as set forth in SEQ ID NO: 132, and three light chain variable region CDRs from a light chain variable region of the amino acid sequence as set forth in SEQ ID NO: 139;
optionally, the antibody comprises three heavy chain variable region CDRs from a heavy chain variable region of the amino acid sequence as set forth in SEQ ID NO: 133, and three light chain variable region CDRs from a light chain variable region of the amino acid sequence as set forth in SEQ ID NO: 139;
optionally, the antibody comprises three heavy chain variable region CDRs from a heavy chain variable region of the amino acid sequence as set forth in SEQ ID NO: 134, and three light chain variable region CDRs from a light chain variable region of the amino acid sequence as set forth in SEQ ID NO: 139;
optionally, the antibody comprises three heavy chain variable region CDRs from a heavy chain variable region of the amino acid sequence as set forth in SEQ ID NO: 140, and three light chain variable region CDRs from a light chain variable region of the amino acid sequence as set forth in SEQ ID NO: 142;
optionally, the antibody comprises three heavy chain variable region CDRs from a heavy chain variable region of the amino acid sequence as set forth in SEQ ID NO: 141, and three light chain variable region CDRs from a light chain variable region of the amino acid sequence as set forth in SEQ ID NO: 142;
optionally, the antibody comprises three heavy chain variable region CDRs from a heavy chain variable region of the amino acid sequence as set forth in SEQ ID NO: 140, and three light chain variable region CDRs from a light chain variable region of the amino acid sequence as set forth in SEQ ID NO: 143;
optionally, the antibody comprises three heavy chain variable region CDRs from a heavy chain variable region of the amino acid sequence as set forth in SEQ ID NO: 141, and three light chain variable region CDRs from a light chain variable region of the amino acid sequence as set forth in SEQ ID NO: 143;
optionally, the antibody comprises three heavy chain variable region CDRs from a heavy chain variable region of the amino acid sequence as set forth in SEQ ID NO: 144, and three light chain variable region CDRs from a light chain variable region of the amino acid sequence as set forth in SEQ ID NO: 147;
optionally, the antibody comprises three heavy chain variable region CDRs from a heavy chain variable region of the amino acid sequence as set forth in SEQ ID NO: 145, and three light chain variable region CDRs from a light chain variable region of the amino acid sequence as set forth in SEQ ID NO: 146;
optionally, the antibody comprises three heavy chain variable region CDRs from a heavy chain variable region of the amino acid sequence as set forth in SEQ ID NO: 145, and three light chain variable region CDRs from a light chain variable region of the amino acid sequence as set forth in SEQ ID NO: 147;
the above CDR sequences further comprise an amino acid sequence having at least 66% identity thereto, and the above CDRs are determined according to the Kabat, IMGT, Chothia, or North numbering system.

9. The antibody or antigen-binding fragment thereof according to any one of claims 1 to 8, wherein the antibody contains at least one of a heavy chain constant region and a light chain constant region, and at least a portion of at least one of the heavy chain constant region and the light chain constant region is derived from at least one of a murine antibody, a human antibody, a primate antibody, or a mutant thereof;
optionally, both the light chain constant region and the heavy chain constant region are derived from a human IgG antibody or a mutant thereof;
optionally, the heavy chain constant region is a human IgG mutant;
optionally, the heavy chain constant region has at least one of the following site mutations compared to a heavy chain constant region of a human wild-type IgG1 antibody:
L234A, L235A, G237A, and K447 deletion;
optionally, the heavy chain constant region has the amino acid sequence as set forth in SEQ ID NO: 148 or SEQ ID NO: 228;
optionally, the light chain constant region has the amino acid sequence as set forth in SEQ ID NO: 149.

10. The antibody or antigen-binding fragment thereof according to any one of claims 1 to 9, wherein the antibody has a heavy chain as set forth in the amino acid sequence of any one of SEQ ID NOs: 150 to 164, 166 to 182, and 229 or a light chain as set forth in the amino acid sequence of any one of SEQ ID NOs: 183 to 197 and 199 to 217;
optionally, the antibody comprises:
a heavy chain as set forth in the amino acid sequence of SEQ ID NO: 181 and a light chain as set forth in the amino acid sequence of SEQ ID NO: 216; or
a heavy chain as set forth in the amino acid sequence of SEQ ID NO: 229 and a light chain as set forth in the amino acid sequence of SEQ ID NO: 216; or
a heavy chain as set forth in the amino acid sequence of SEQ ID NO: 171 and a light chain as set forth in the amino acid sequence of SEQ ID NO: 207; or
a heavy chain as set forth in the amino acid sequence of SEQ ID NO: 178 and a light chain as set forth in the amino acid sequence of SEQ ID NO: 213; or
a heavy chain as set forth in the amino acid sequence of SEQ ID NO: 150 and a light chain as set forth in the amino acid sequence of SEQ ID NO: 183; or
a heavy chain as set forth in the amino acid sequence of SEQ ID NO: 151 and a light chain as set forth in the amino acid sequence of SEQ ID NO: 184; or
a heavy chain as set forth in the amino acid sequence of SEQ ID NO: 152 and a light chain as set forth in the amino acid sequence of SEQ ID NO: 185; or
a heavy chain as set forth in the amino acid sequence of SEQ ID NO: 153 and a light chain as set forth in the amino acid sequence of SEQ ID NO: 186; or
a heavy chain as set forth in the amino acid sequence of SEQ ID NO: 154 and a light chain as set forth in the amino acid sequence of SEQ ID NO: 187; or
a heavy chain as set forth in the amino acid sequence of SEQ ID NO: 155 and a light chain as set forth in the amino acid sequence of SEQ ID NO: 188; or
a heavy chain as set forth in the amino acid sequence of SEQ ID NO: 156 and a light chain as set forth in the amino acid sequence of SEQ ID NO: 189; or
a heavy chain as set forth in the amino acid sequence of SEQ ID NO: 157 and a light chain as set forth in the amino acid sequence of SEQ ID NO: 190; or
a heavy chain as set forth in the amino acid sequence of SEQ ID NO: 158 and a light chain as set forth in the amino acid sequence of SEQ ID NO: 191; or
a heavy chain as set forth in the amino acid sequence of SEQ ID NO: 159 and a light chain as set forth in the amino acid sequence of SEQ ID NO: 192; or
a heavy chain as set forth in the amino acid sequence of SEQ ID NO: 160 and a light chain as set forth in the amino acid sequence of SEQ ID NO: 193; or
a heavy chain as set forth in the amino acid sequence of SEQ ID NO: 161 and a light chain as set forth in the amino acid sequence of SEQ ID NO: 194; or
a heavy chain as set forth in the amino acid sequence of SEQ ID NO: 162 and a light chain as set forth in the amino acid sequence of SEQ ID NO: 195; or
a heavy chain as set forth in the amino acid sequence of SEQ ID NO: 163 and a light chain as set forth in the amino acid sequence of SEQ ID NO: 196; or
a heavy chain as set forth in the amino acid sequence of SEQ ID NO: 166 and a light chain as set forth in the amino acid sequence of SEQ ID NO: 199; or
a heavy chain as set forth in the amino acid sequence of SEQ ID NO: 167 and a light chain as set forth in the amino acid sequence of SEQ ID NO: 199; or
a heavy chain as set forth in the amino acid sequence of SEQ ID NO: 168 and a light chain as set forth in the amino acid sequence of SEQ ID NO: 199; or
a heavy chain as set forth in the amino acid sequence of SEQ ID NO: 169 and a light chain as set forth in the amino acid sequence of SEQ ID NO: 200; or
a heavy chain as set forth in the amino acid sequence of SEQ ID NO: 169 and a light chain as set forth in the amino acid sequence of SEQ ID NO: 201; or
a heavy chain as set forth in the amino acid sequence of SEQ ID NO: 169 and a light chain as set forth in the amino acid sequence of SEQ ID NO: 202; or
a heavy chain as set forth in the amino acid sequence of SEQ ID NO: 170 and a light chain as set forth in the amino acid sequence of SEQ ID NO: 203; or
a heavy chain as set forth in the amino acid sequence of SEQ ID NO: 170 and a light chain as set forth in the amino acid sequence of SEQ ID NO: 204; or
a heavy chain as set forth in the amino acid sequence of SEQ ID NO: 170 and a light chain as set forth in the amino acid sequence of SEQ ID NO: 205; or
a heavy chain as set forth in the amino acid sequence of SEQ ID NO: 171 and a light chain as set forth in the amino acid sequence of SEQ ID NO: 206; or
a heavy chain as set forth in the amino acid sequence of SEQ ID NO: 171 and a light chain as set forth in the amino acid sequence of SEQ ID NO: 208; or
a heavy chain as set forth in the amino acid sequence of SEQ ID NO: 171 and a light chain as set forth in the amino acid sequence of SEQ ID NO: 209; or
a heavy chain as set forth in the amino acid sequence of SEQ ID NO: 172 and a light chain as set forth in the amino acid sequence of SEQ ID NO: 206; or
a heavy chain as set forth in the amino acid sequence of SEQ ID NO: 172 and a light chain as set forth in the amino acid sequence of SEQ ID NO: 207; or
a heavy chain as set forth in the amino acid sequence of SEQ ID NO: 172 and a light chain as set forth in the amino acid sequence of SEQ ID NO: 208; or
a heavy chain as set forth in the amino acid sequence of SEQ ID NO: 172 and a light chain as set forth in the amino acid sequence of SEQ ID NO: 209; or
a heavy chain as set forth in the amino acid sequence of SEQ ID NO: 173 and a light chain as set forth in the amino acid sequence of SEQ ID NO: 206; or
a heavy chain as set forth in the amino acid sequence of SEQ ID NO: 173 and a light chain as set forth in the amino acid sequence of SEQ ID NO: 207; or
a heavy chain as set forth in the amino acid sequence of SEQ ID NO: 173 and a light chain as set forth in the amino acid sequence of SEQ ID NO: 208; or
a heavy chain as set forth in the amino acid sequence of SEQ ID NO: 173 and a light chain as set forth in the amino acid sequence of SEQ ID NO: 209; or
a heavy chain as set forth in the amino acid sequence of SEQ ID NO: 174 and a light chain as set forth in the amino acid sequence of SEQ ID NO: 206; or
a heavy chain as set forth in the amino acid sequence of SEQ ID NO: 174 and a light chain as set forth in the amino acid sequence of SEQ ID NO: 207; or
a heavy chain as set forth in the amino acid sequence of SEQ ID NO: 174 and a light chain as set forth in the amino acid sequence of SEQ ID NO: 208; or
a heavy chain as set forth in the amino acid sequence of SEQ ID NO: 174 and a light chain as set forth in the amino acid sequence of SEQ ID NO: 209; or
a heavy chain as set forth in the amino acid sequence of SEQ ID NO: 175 and a light chain as set forth in the amino acid sequence of SEQ ID NO: 210; or
a heavy chain as set forth in the amino acid sequence of SEQ ID NO: 175 and a light chain as set forth in the amino acid sequence of SEQ ID NO: 211; or
a heavy chain as set forth in the amino acid sequence of SEQ ID NO: 175 and a light chain as set forth in the amino acid sequence of SEQ ID NO: 212; or
a heavy chain as set forth in the amino acid sequence of SEQ ID NO: 175 and a light chain as set forth in the amino acid sequence of SEQ ID NO: 213; or
a heavy chain as set forth in the amino acid sequence of SEQ ID NO: 176 and a light chain as set forth in the amino acid sequence of SEQ ID NO: 210; or
a heavy chain as set forth in the amino acid sequence of SEQ ID NO: 176 and a light chain as set forth in the amino acid sequence of SEQ ID NO: 211; or
a heavy chain as set forth in the amino acid sequence of SEQ ID NO: 176 and a light chain as set forth in the amino acid sequence of SEQ ID NO: 212; or
a heavy chain as set forth in the amino acid sequence of SEQ ID NO: 176 and a light chain as set forth in the amino acid sequence of SEQ ID NO: 213; or
a heavy chain as set forth in the amino acid sequence of SEQ ID NO: 177 and a light chain as set forth in the amino acid sequence of SEQ ID NO: 210; or
a heavy chain as set forth in the amino acid sequence of SEQ ID NO: 177 and a light chain as set forth in the amino acid sequence of SEQ ID NO: 211; or
a heavy chain as set forth in the amino acid sequence of SEQ ID NO: 177 and a light chain as set forth in the amino acid sequence of SEQ ID NO: 212; or
a heavy chain as set forth in the amino acid sequence of SEQ ID NO: 177 and a light chain as set forth in the amino acid sequence of SEQ ID NO: 213; or
a heavy chain as set forth in the amino acid sequence of SEQ ID NO: 178 and a light chain as set forth in the amino acid sequence of SEQ ID NO: 210; or
a heavy chain as set forth in the amino acid sequence of SEQ ID NO: 178 and a light chain as set forth in the amino acid sequence of SEQ ID NO: 211; or
a heavy chain as set forth in the amino acid sequence of SEQ ID NO: 178 and a light chain as set forth in the amino acid sequence of SEQ ID NO: 212; or
a heavy chain as set forth in the amino acid sequence of SEQ ID NO: 179 and a light chain as set forth in the amino acid sequence of SEQ ID NO: 214; or
a heavy chain as set forth in the amino acid sequence of SEQ ID NO: 179 and a light chain as set forth in the amino acid sequence of SEQ ID NO: 215; or
a heavy chain as set forth in the amino acid sequence of SEQ ID NO: 180 and a light chain as set forth in the amino acid sequence of SEQ ID NO: 214; or
a heavy chain as set forth in the amino acid sequence of SEQ ID NO: 180 and a light chain as set forth in the amino acid sequence of SEQ ID NO: 215; or
a heavy chain as set forth in the amino acid sequence of SEQ ID NO: 181 and a light chain as set forth in the amino acid sequence of SEQ ID NO: 217; or
a heavy chain as set forth in the amino acid sequence of SEQ ID NO: 182 and a light chain as set forth in the amino acid sequence of SEQ ID NO: 216; or
a heavy chain as set forth in the amino acid sequence of SEQ ID NO: 182 and a light chain as set forth in the amino acid sequence of SEQ ID NO: 217.

11. The antibody or antigen-binding fragment thereof according to any one of claims 1 to 10, wherein the antibody comprises at least one selected from the group consisting of a monoclonal antibody, a polyclonal antibody, a multimeric antibody, and a CDR grafted antibody;
optionally, the antibody comprises at least one selected from the group consisting of a single-chain antibody, a Fab antibody, an Fv antibody, a single-chain antibody, a single-domain antibody, and a minimal recognition unit;
optionally, the antigen-binding fragment comprises at least one of a Fab fragment, a (Fab)₂ fragment, an scFv-Fc fusion protein, an scFv-Fv fusion protein, an Fv fragment, and a minimal recognition unit.

12. A nucleic acid molecule, wherein the nucleic acid molecule encodes the antibody or antigen-binding fragment thereof according to any one of claims 1 to 11.

13. The nucleic acid molecule according to claim 12, wherein the nucleic acid molecule is DNA.

14. An expression vector, carrying the nucleic acid molecule according to claim 12 or 13.

15. The expression vector according to claim 14, wherein the expression vector is a eukaryotic expression vector or a prokaryotic expression vector; preferably, the expression vector is a plasmid expression vector.

16. A recombinant cell, wherein the recombinant cell:
carries the nucleic acid molecule according to claim 12 or 13; or
expresses the antibody or antigen-binding fragment thereof according to any one of claims 1 to 11.

17. The recombinant cell according to claim 16, wherein the recombinant cell is obtained by introducing the expression vector according to claim 14 or 15 into a host cell;
optionally, the recombinant cell is a eukaryotic cell;
preferably, the recombinant cell is a mammalian cell.

18. A pharmaceutical composition, comprising:
the antibody or antigen-binding fragment thereof according to any one of claims 1 to 11;
the nucleic acid molecule according to claim 12 or 13;
the expression vector according to claim 14 or 15; or
the recombinant cell according to claim 16 or 17;
optionally, the pharmaceutical composition further comprises a pharmaceutically acceptable excipient.

19. A combination medicament or pharmaceutical kit, comprising:
the antibody or antigen-binding fragment thereof according to any one of claims 1 to 11 or the pharmaceutical composition according to claim 18 as a first active ingredient; and
an anti-PDL1 agent as a second active ingredient;
optionally, the anti-PDL1 agent is an anti-PDL1 antibody.

20. Use of the antibody or antigen-binding fragment thereof according to any one of claims 1 to 11, the nucleic acid molecule according to claim 12 or 13, the expression vector according to claim 14 or 15, the recombinant cell according to claim 16 or 17, the pharmaceutical composition according to claim 18, or the combination medicament or pharmaceutical kit according to claim 19, wherein the use comprises:
use for preventing and/or treating cancer and cachexia;
use in the manufacture of a medicament for preventing and/or treating cancer and cachexia.

21. A kit, comprising: the antibody according to any one of claims 1 to 11; the nucleic acid molecule according to claim 12 or 13; the expression vector according to claim 14 or 15; or the recombinant cell according to claim 16 or 17.

22. Use of the antibody or antigen-binding fragment thereof according to any one of claims 1 to 11, the nucleic acid molecule according to claim 12 or 13, the expression vector according to claim 14 or 15, or the recombinant cell according to claim 16 or 17, wherein the use comprises:
use for detecting GDF15;
use in the manufacture of a kit for detecting GDF15.

23. A method for preventing and/or treating cancer and cachexia, comprising:
administering to a subject a pharmaceutically acceptable amount of the antibody or antigen-binding fragment thereof according to any one of claims 1 to 11, the nucleic acid molecule according to claim 12 or 13, the expression vector according to claim 14 or 15, the recombinant cell according to claim 16 or 17, the pharmaceutical composition according to claim 18, or the combination medicament or pharmaceutical kit according to claim 19.

24. A method for detecting GDF15, comprising:
detecting a sample to be tested using the antibody or antigen-binding fragment thereof according to any one of claims 1 to 11, or the kit according to claim 21;
optionally, forming an immune complex between the antibody or antigen-binding fragment thereof and GDF15 in the sample to be tested;
detecting the presence of the immune complex, and determining whether the sample to be tested contains GDF15 based on the presence of the immune complex.
